# EUROPEAN PATENT SPECIFICATION

(11) **EP 2 861 555 B1**
(45) Date of publication and mention of the grant of the patent: **29.08.2018**
(21) Application number: 13729385.8
(22) Date of filing: 18.06.2013
(51) Int. Cl.: C07C 69/732, C07C 235/34, A61Q 19/02, C07D 209/40, C07D 211/68, C07D 265/30, A61K 8/42, A61K 8/44, C07D 313/08, C07C 323/59, C07D 307/33, A61K 8/49

(54) **PROCESS FOR DEPIGMENTING KERATIN MATERIALS USING NOVEL RESORCINOL-BASED COMPOUNDS**
VERFAHREN ZUR DEPIGMENTIERUNG EINES KERATINMATERIALS MITHILFE NEUARTIGER VERBINDUNGEN AUF RESORCINOLBASIS
PROCÉDÉ DE DÉPIGMENTATION DE MATIÈRES KÉRATINIQUES AU MOYEN DE NOUVEAUX COMPOSÉS À BASE DE RÉSORCINOL

(30) Priority: 19.06.2012 FR 1255752
(43) Date of publication of application: 22.04.2015
(73) Proprietor: L'Oréal, 75008 Paris (FR)
(72) Inventor: MARAT, Xavier, F-75010 Paris (FR)
(74) Representative: Le Blainvaux Bellegarde, Françoise
(86) International application number: PCT/EP2013/062623
(87) International publication number: WO 2013/189930

(56) References cited:
- WO-A1-2012/079938
- JP-A- 11 255 638
- JP-A- 11 255 639

## Description

The present invention relates to novel resorcinol-based compounds and to a cosmetic treatment process, in particular for depigmenting and/or bleaching the skin, employing such a compound.

At different periods in their lives, some people witness the appearance on the skin and more especially on the hands and face of darker and/or more highly coloured blemishes which give the skin a heterogeneous appearance. These blemishes are due in particular to a high concentration of melanin in the keratinocytes situated at the surface of the skin.

The use of inoffensive topical depigmenting substances which are highly effective is very particularly sought after with a view to treating pigment blemishes.

The mechanism of formation of the pigmentation of the skin, that is to say of the formation of melanin, is particularly complex and involves, schematically, the following main stages:
Tyrosine ---> Dopa ---> Dopaquinone ---> Dopachrome ---> Melanin Tyrosinase (monophenol dihydroxyl phenylalanine: oxygen oxidoreductase EC 1.14.18.1) is the essential enzyme involved in this sequence of reactions. In particular, it catalyses the conversion reaction of tyrosine to give Dopa (dihydroxyphenylalanine), by virtue of its hydroxylase activity, and the conversion reaction of Dopa to give dopaquinone, by virtue of its oxidase activity. This tyrosinase only acts when it is in the maturation state under the effect of certain biological factors.

A substance is recognized as depigmenting if it acts directly on the vitality of the epidermal melanocytes where melanogenesis takes place and/or if it interferes with one of the steps of melanin biosynthesis, either by inhibiting one of the enzymes involved in melanogenesis or by being inserted as a structural analogue of one of the chemical compounds in the sequence for the synthesis of melanin, which sequence can then be blocked and thus ensure depigmentation.

Arbutin and kojic acid are known as agents for depigmenting the skin. 3-Alkyl substituted resorcinol derivatives are also known to be useful as bleaching cosmetics (see JP11255639 and JP11255638). Substances have been sought which have efficient depigmenting action, in particular superior to that of arbutin and kojic acid.

In this regard, the Applicant Company has discovered, surprisingly and unexpectedly, that some resorcinol-based compounds show good depigmenting activity, even at low concentration.

A subject of the invention is thus novel compounds of formula (I) as defined below.

Another subject of the invention is a composition comprising, in a physiologically acceptable medium, at least one compound of formula (I) as defined below.

Another subject of the invention is a non-therapeutic cosmetic process for depigmenting, lightening and/or bleaching keratin materials, in particular the skin, comprising the application of the composition described above.

The invention also relates to the non-therapeutic cosmetic use of a compound of formula (I) as an agent for bleaching, lightening and/or depigmenting keratin materials, in particular the skin.

The compounds according to the invention make it possible to effectively depigment and/or lighten, indeed even to bleach, human skin. They are in particular intended to be applied to the skin of individuals exhibiting brownish pigmentation blemishes or blemishes due to ageing or to the skin of individuals desiring to combat the appearance of a brownish colour originating from melanogenesis.

They can also make it possible to depigment and/or lighten bodily hair, the eyelashes or head hair, and also the lips and/or the nails.

The novel compounds according to the invention thus correspond to formula (I) below: 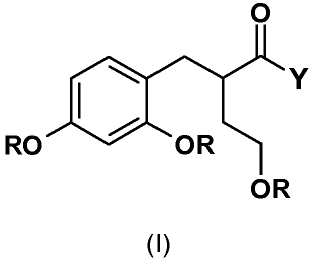 in which:
- **R** denotes a hydrogen atom;
- **Y** denotes a radical chosen from OR' and NAR";
**R'** denotes a radical chosen from:
a) -H;
b) a linear saturated C1-C20 or unsaturated C2-C20 or branched C3-C20 or cyclic C3-C8 alkyl group, optionally interrupted with one or more heteroatoms
   or groups chosen from N, O, -CO- or a combination thereof such as -NHCO- or -NHCONH-, and/or optionally substituted with one or more identical or
   different groups chosen from:
   i) -OR5
   ii) -SR5
   iii) -NR6R7
   iv) -CONHR6
   v) -CONR6R7
   vi) -COOR6
   vii) -NHCONHR6
   viii) -C(O)-(C1-C4)alkyl
   ix) a C5-C12 (hetero)aryl group, optionally containing one or more heteroatoms chosen from O, N and S, optionally substituted with one or more hydroxyls and/or with one or more C1-C8 alkoxy radicals;
   x) a saturated or unsaturated non-aromatic 5- to 8-membered heterocycle, comprising one or more heteroatoms chosen from O, N and S, optionally substituted with one or more hydroxyls and/or with one or more C1-C8 alkoxy or C1-C4 alkyl radicals; one of the ring members possibly being a carbonyl group;
c) a C5-C12 (hetero)aryl group, optionally containing one or more heteroatoms chosen from O, N and S, optionally substituted with one or more hydroxyls and/or with one or more radicals chosen from C1-C8 alkoxy and C1-C8 alkyl groups;
**R5** being chosen from H, a saturated linear C1-C10 or branched C3-C10 or unsaturated C2-C10 or cyclic C3-C8 alkyl hydrocarbon-based group;
**R6** and **R7,** which may be identical or different, being chosen from H, a saturated linear C1-C10 or branched C3-C10 or unsaturated C2-C10 or cyclic C3-C8 alkyl hydrocarbon-based group; a (C1-C4)alkyl(hetero)(C6)aryl group optionally containing a nitrogen atom, especially a benzyl group;
**R6** and **R7** possibly forming, with the nitrogen atom that bears them, a 5-to 8-membered heterocycle which may contain one or more heteroatoms or groups chosen from
N, O and-CO- and/or optionally substituted with a C1-C10 hydrocarbon-based chain;
**A** denotes a radical chosen from:
a) -H;
b) a linear saturated C1-C20 or unsaturated C2-C20 or branched C3-C20 or cyclic C3-C8 alkyl group, optionally interrupted with one or more heteroatoms
   or groups chosen from N, O, -CO- or a combination thereof such as -NHCO- or -NHCONH-, and/or optionally substituted with one or more identical or
   different groups chosen from:
   i) -OR15
   ii) -SR15
   iii) -NR16R17
   iv) -CONHR16
   v) -CONR16R17
   vi) -COOR16
   vii) -NHCONHR16
   viii) -C(O)-(C1-C4)alkyl
   ix) a C5-C12 (hetero)aryl group, optionally containing one or more heteroatoms chosen from O, N and S, optionally substituted with one or more hydroxyls and/or with one or more C1-C8 alkoxy radicals;
   x) a saturated or unsaturated non-aromatic 5- to 8-membered heterocycle, comprising one or more heteroatoms chosen from O, N and S, optionally substituted with one or more hydroxyls and/or with one or more C1-C8 alkoxy or C1-C4 alkyl radicals; one of the ring members possibly being a carbonyl group;
c) a C5-C12 (hetero)aryl group, optionally containing one or more heteroatoms chosen from O, N and S, optionally substituted with one or more hydroxyls and/or with one or more radicals chosen from C1-C8 alkoxy
   and C1-C8 alkyl groups;
   xi) -NH-C=NH(NH₂) (guanidine group)
d) -NR12R13;
e) -OR14;
f) -C(O)NHR14;
g) C(O)-(C1-C10)alkyl
**R12** and **R13,** which may be identical or different, denoting a radical chosen from:
a) -H;
b) a saturated linear C1-C10 or unsaturated C2-C10 or branched C3-C10 or cyclic C3-C8 alkyl group, optionally interrupted with one or more heteroatoms or groups chosen from N, O, -CO- or a combination thereof such as -NHCO- or -NHCONH-, and/or optionally substituted with one or more groups, which may be identical or different, chosen from -OR5;
c) a C5-C12 (hetero)aryl group, optionally containing one or more heteroatoms chosen from O, N and S, optionally substituted with one or more hydroxyls and/or with one or more C1-C8 alkoxy radicals; R12 and R13 possibly forming, with the nitrogen that bears them, a 5- to 8-membered heterocycle, which may contain one or more heteroatoms or groups chosen from N, O and -CO- and/or optionally substituted with a C1-C10 hydrocarbon-based chain optionally containing one or more radicals chosen from hydroxyl and C1-C4 alkoxy;
**R14** denoting a radical chosen from:
a) -H;
b) a saturated linear C1-C10 or branched C3-C10 or cyclic C3-C8 alkyl group, optionally substituted with one or more groups, which may be identical or different,
   chosen from:
   i) -COOR16;
   ii) a C5-C12 (hetero)aryl radical, optionally containing one or more heteroatoms chosen from O, N and S, optionally substituted with one or more hydroxyls and/or with one or more C1-C8 alkoxy radicals;
c) a C5-C12 (hetero)aryl group, optionally containing one or more heteroatoms chosen from O, N and S, optionally substituted with one or more hydroxyls and/or with one or more C1-C8 alkoxy radicals;
**R15** being chosen from H, a saturated linear C1-C10 or branched C3-C10 or unsaturated C2-C10 or cyclic C3-C8 alkyl hydrocarbon-based group;
**R16** and **R17,** which may be identical or different, being chosen from H and a saturated linear C1-C10 or branched C3-C10 or unsaturated C2-C10 or cyclic C3-C8 alkyl hydrocarbon-based group; a (C1-C4)alkyl(hetero)(C6)aryl group optionally containing a nitrogen atom, especially a benzyl group; an acetyl radical;
**R16** and **R17** possibly forming, with the nitrogen that bears them, a 5- to 8-membered heterocycle, which may contain one or more heteroatoms or groups chosen from N, O and -CO- and/or optionally substituted with a C1-C10 hydrocarbon-based chain;
**R"** denotes a radical chosen from:
a) -H;
b) a linear saturated C1-C20 or unsaturated C2-C20 or branched C3-C20 or cyclic C3-C8 alkyl group, optionally interrupted with one or more heteroatoms or groups chosen from N, O, -CO- or a combination thereof such as -NHCO- or -NHCONH-, and/or optionally substituted with one or more identical or different groups chosen from:
   i) -OR25
   ii) -SR25
   iii) -NR26R27
   iv) -CONHR26
   v) -CONR26R27
   vi) -COOR26
   vii) -NHCONHR26
   viii) -C(O)-(C1-C4)alkyl
   ix) a C5-C12 (hetero)aryl group, optionally containing one or more heteroatoms chosen from O, N and S, optionally substituted with one or more hydroxyls and/or with one or more C1-C8 alkoxy radicals;
   x) a saturated or unsaturated non-aromatic 5- to 8-membered heterocycle, comprising one or more heteroatoms chosen from O, N and S, optionally substituted with one or more hydroxyls and/or with one or more C1-C8 alkoxy or C1-C4 alkyl radicals; one of the ring members possibly being a carbonyl group;
c) a C5-C12 (hetero)aryl group, optionally containing one or more heteroatoms chosen from O, N and S, optionally substituted with one or more hydroxyls and/or with one or more radicals chosen from C1-C8 alkoxy and C1-C8 alkyl groups;
**R25** being chosen from H, a saturated linear C1-C10 or branched C3-C10 or unsaturated C2-C10 or cyclic C3-C8 alkyl hydrocarbon-based group;
**R26** and **R27,** which may be identical or different, being chosen from H, a saturated linear C1-C10 or branched C3-C10 or unsaturated C2-C10 or cyclic C3-C8 alkyl hydrocarbon-based group; a (C1-C4)alkyl(hetero)(C6)aryl group optionally containing a nitrogen atom, especially a benzyl group; **R26** and **R27** possibly forming, with the nitrogen that bears them, a 5- to 8-membered heterocycle, which may contain one or more heteroatoms or groups chosen from N, O and -CO- and/or optionally substituted with a C1-C10 hydrocarbon-based chain;
it being understood that **A** and **R"** may form, with the nitrogen atom that bears them, a saturated or unsaturated 5- to 8-membered heterocycle, which may contain one or more heteroatoms or groups chosen from N, O and -CO- and/or optionally substituted with a C1-C10 hydrocarbon-based chain or C1-C10 hydroxyalkyl or CO2T, T denoting a hydrogen atom or a saturated linear C1-C10 or branched C3-C10 alkyl radical,
and the salts thereof, the solvates thereof, the optical isomers thereof and the racemic mixtures thereof, alone or as a mixture.

When a radical (1) may be substituted with one or more radicals (2) as specified previously, preferentially, the said radical (1) may be substituted with 1 to 3 radicals (2) as specified previously.

When a cyclic radical (1') optionally contains one or more heteroatoms or groups as specified previously, preferentially, the said cyclic radical (1') optionally contains 1 to 3 heteroatoms or groups as specified previously.

The salts of the compounds of formula (I) comprise conventional non-toxic salts of the said compounds, such as those formed from acid or base.

Mention may be made, as salts of the compound of formula (I) (when it comprises a quaternizable nitrogen atom), of:
a) the salts obtained by addition of the compound (I) to a mineral acid, chosen in particular from hydrochloric, boric, hydrobromic, hydriodic, sulfuric, nitric, carbonic, phosphoric or tetrafluoroboric acid;
b) or the salts obtained by addition of the compound (I) to an organic acid, chosen in particular from acetic, propionic, succinic, fumaric, lactic, glycolic, citric, gluconic, salicylic, tartaric, terephthalic, methylsulfonic, ethylsulfonic, benzenesulfonic, toluenesulfonic or triflic acid.

Mention may also be made of the salts obtained by addition of the compound of formula (I) (when it comprises an acid group) to an mineral base, such as sodium hydroxide, potassium hydroxide, calcium hydroxide, ammonium hydroxide, magnesium hydroxide, lithium hydroxide, and the carbonates or hydrogen carbonates of sodium, of potassium or of calcium, for example;
or to an organic base, such as a primary, secondary or tertiary alkylamine, for example triethylamine or butylamine. This primary, secondary or tertiary alkylamine can contain one or more nitrogen and/or oxygen atoms and can thus contain, for example, one or more alcohol functional groups; mention may in particular be made of 2-amino-2-methylpropanol, ethanolamine, triethanolamine, 2-(dimethylamino)propanol, 2-amino-2-(hydroxymethyl)-1,3-propanediol or 3-(dimethylamino)propylamine.

Mention may also be made of the salts of amino acids, such as, for example, lysine, arginine, guanidine, glutamic acid or aspartic acid.

Advantageously, the salts of the compound of formula (I) (when it comprises an acid group) can be chosen from alkali metal or alkaline-earth metal salts, such as sodium, potassium, calcium or magnesium; or ammonium salts.

Advantageously, the salts of the compound of formula (I) (when it comprises a quaternizable nitrogen atom) can be chosen from halides, such as chloride or bromide, citrate, acetate, succinate, phosphate, lactate or tartrate.

The acceptable solvates of the compounds described in the present invention comprise conventional solvates, such as those formed during the preparation of the said compounds due to the presence of solvents. Mention may be made, by way of example, of the solvates due to the presence of water or of linear or branched alcohols, such as ethanol or isopropanol.

The optical isomers are in particular enantiomers and diastereoisomers.

In the context of the present invention:
a **"(Cₓ-C_{y})alkyl group"** denotes an alkyl group comprising from x to y carbon atoms. Such an alkyl group can be linear and saturated and can typically include from 1 to 20 carbon atoms or also from 1 to 10 carbon atoms. It can also be linear and unsaturated and can typically include from 2 to 20 carbon atoms or also from 2 to 10 carbon atoms. It can also be branched and can typically include from 3 to 20 carbon atoms or also from 3 to 10 carbon atoms. An alkyl group can also be cyclic; it is then a cycloalkyl group, which can typically include from 3 to 8 carbon atoms. In such a case it is indicated in the claims as "cyclic alkyl". Unless otherwise indicated, a "(Cₓ-C_{y})alkyl group" denotes a linear and saturated alkyl group comprising from x to y carbon atoms.

Preferentially, the saturated or branched linear alkyl groups may be chosen from: methyl, ethyl, propyl, isopropyl, butyl, isobutyl, tert-butyl, pentyl, hexyl, heptyl, 2-ethylhexyl, octyl, nonyl, decyl, undecyl, dodecyl, tridecyl, tetradecyl, pentadecyl, hexadecyl, heptadecyl, octadecyl, nonadecyl and eicosyl.

More preferentially, the saturated or branched linear alkyl groups may be chosen from: methyl, ethyl, propyl, isopropyl, butyl, isobutyl, tert-butyl, pentyl, hexyl, heptyl, 2-ethylhexyl and octyl.

The cycloalkyl group may be chosen from: cyclopropyl, cyclobutyl, cyclopentyl, cyclohexyl, cycloheptyl and cyclooctyl. - A **"(Cₓ-C_{y})alkoxy group"** denotes a linear and if appropriate branched group of formula -O(Cₓ-C_{y})alkyl which can typically include from 1 to 8 carbon atoms or also from 1 to 4 carbon atoms.

The alkoxy group may be chosen from methoxy, ethoxy, propoxy and butoxy and can more particularly be a methoxy group. - A "saturated or unsaturated non-aromatic heterocycle group" denotes a monocyclic or bicyclic carbocyclic 5- to 8-membered group containing one to three heteroatoms or groups chosen from N, O, S and -C(O)-.

A heterocycle group may be chosen from piperidyl, morpholinyl, piperazinyl and pyrrolidinyl. Preferentially, it is the piperidyl or morpholinyl ring. - An **"aryl group"** denotes an unsaturated or partially unsaturated monocyclic or bicyclic carbocyclic group including from 5 to 12 carbon atoms.

The aryl radicals may be chosen from phenyl, naphthyl, indenyl, fluorenyl and anthracenyl. Preferably, it is the phenyl group.
- A "heteroaryl group" denotes a fused or non-fused, 5- to 22-membered monocyclic or polycyclic group, comprising from 1 to 6 heteroatoms chosen from nitrogen, oxygen and sulfur, and at least one ring of which is aromatic; preferentially, a heteroaryl radical is chosen from acridinyl, benzimidazolyl, benzobistriazolyl, benzopyrazolyl, benzopyridazinyl, benzoquinolyl, benzothiazolyl, benzotriazolyl, benzoxazolyl, pyridyl, tetrazolyl, dihydrothiazolyl, imidazopyridyl, imidazolyl, indolyl, isoquinolyl, naphthimidazolyl, naphthxazolyl, naphthpyrazolyl, oxadiazolyl, oxazolyl, oxazolopyridyl, phenazinyl, phenoxazolyl, pyrazinyl, pyrazolyl, pyrilyl, pyrazoyltriazyl, pyridyl, pyridinoimidazolyl, pyrrolyl, quinolyl, tetrazolyl, thiadiazolyl, thiazolyl, thiazolopyridyl, thiazoylimidazolyl, thiopyrylyl, triazolyl, xanthyl and the ammonium salt thereof;

The term "5- to 8-membered heterocycle which may contain one or more heteroatoms or groups chosen from N and O" means in particular, in a non-limiting manner, piperidine, morpholine, piperazine, imidazole and pyrrolidine rings. Preferentially, it is a piperidine or morpholine ring.

Preferably, the compounds of formula (I) have the following meanings:
**R** denotes a hydrogen atom;
**Y** denotes a radical chosen from OR' and NAR";
**R'** denotes a radical chosen from:
a) -H;
b) a linear saturated C1-C10 or unsaturated C2-C10 or branched C10 or cyclic C3-C8 alkyl group, optionally interrupted with one or more heteroatoms or groups chosen from N, O, -CO- or a combination thereof such as -NHCO- or -NHCONH-, and/or optionally substituted with one or more groups -OR15; **R15** being chosen from H and a saturated linear C1-C4 alkyl hydrocarbon-based group;
c) a C5-C12 (hetero)aryl group, optionally containing one or more heteroatoms chosen from O, N and S, optionally substituted with one or more hydroxyls and/or with one or more radicals chosen from C1-C8 alkoxy and C1-C8 alkyl groups;
**A** denotes a radical chosen from:
a) -H;
b) a linear saturated C1-C20 or unsaturated C2-C20 or branched C3-C20 or cyclic C3-C8 alkyl group, optionally interrupted with one or more heteroatoms or groups chosen from N, O, -CO- or a combination thereof such as -NHCO- or -NHCONH-, and/or optionally substituted with one or more identical or
   different groups chosen from:
   i) -OR15
   ii) -SR15
   iii) -NR16R17
   iv) -CONHR16
   vi) -COOR16
   ix) a C5-C12 (hetero)aryl group, optionally containing one or more heteroatoms chosen from O, N and S, optionally substituted with one or more hydroxyls and/or with one or more C1-C8 alkoxy radicals;
   x) a saturated or unsaturated non-aromatic 5- to 8-membered heterocycle, comprising one or more heteroatoms chosen from O, N and S, optionally substituted with one or more hydroxyls and/or with one or more C1-C8 alkoxy or C1-C4 alkyl radicals; one of the ring members possibly being a carbonyl group;
c) a C5-C12 (hetero)aryl group, optionally containing one or more heteroatoms chosen from O, N and S, optionally substituted with one or more hydroxyls and/or with one or more radicals chosen from C1-C8 alkoxy and C1-C8 alkyl groups;
d) -NR12R13;
e) -OR14;
**R12** and **R13,** which may be identical or different, denoting a radical chosen from:
a) -H;
b) a saturated linear C1-C4 alkyl group;
c) a phenyl group optionally substituted with one or more hydroxyls and/or with one or more C1-C8 alkoxy radicals;
**R12** and **R13** possibly forming, with the nitrogen that bears them, a 5- to 8-membered heterocycle, which may contain one or more heteroatoms or groups chosen from N, O and -CO- and/or optionally substituted with a C1-C10 hydrocarbon-based chain optionally containing one or more radicals chosen from hydroxyl and C1-C4 alkoxy;
**R14** denoting a radical chosen from:
a) -H;
b) a saturated linear C1-C10 alkyl group optionally substituted with one or more identical or different phenyl radicals, the said phenyl radicals being
optionally substituted with one or more hydroxyls and/or with one or more C1-C8 alkoxy radicals;
**R15** being chosen from H, a saturated linear C1-C10 or branched C3-C10 or unsaturated C2-C10 or cyclic C3-C8 alkyl hydrocarbon-based group;
**R16** and **R17,** which may be identical or different, being chosen from H and a saturated linear C1-C10 or branched C3-C10 or unsaturated C2-C10 or cyclic C3-C8 alkyl hydrocarbon-based group; a (C1-C4)alkyl(hetero)(C6)aryl group optionally containing a nitrogen atom, especially a benzyl group; an acetyl radical;
**R16** and **R17** possibly forming, with the nitrogen that bears them, a 5- to 8-membered heterocycle, which may contain one or more heteroatoms or groups chosen from N, O and -CO- and/or optionally substituted with a C1-C10 hydrocarbon-based chain;
**R"** denotes a radical chosen from:
a) -H;
b) a linear saturated C1-C10 or branched C3-C10 alkyl group, optionally substituted with one or more identical or different groups -OR25;
**R25** being chosen from H and a saturated linear C1-C10 alkyl hydrocarbon-based group;
it being understood that **A** and **R"** may form, with the nitrogen atom that bears them, a saturated or unsaturated 5- to 8-membered heterocycle, which may contain one or more heteroatoms or groups chosen from N, O and -CO- and/or optionally substituted with a C1-C4 hydrocarbon-based chain or C1-C4 hydroxyalkyl or CO2T, T denoting a hydrogen atom or a saturated linear C1-C6 or branched C3-C6 alkyl radical,
and the salts thereof, the solvates thereof, the optical isomers thereof and the racemic mixtures thereof, alone or as a mixture.

More preferentially, the compounds of formula (I) have the following meanings:
**R'** denotes a radical chosen from:
   a) -H;
   b) a linear saturated C1-C10 or unsaturated C2-C10 or branched C3-C10 or cyclic C3-C8 alkyl group, optionally interrupted with one or more oxygen atoms, and/or optionally substituted with one or more hydroxyl, methoxy or ethoxy groups;
**A** denotes a radical chosen from:
   a) -H;
   b) a linear saturated C1-C10 or unsaturated C2-C10 or branched C3-C10 or cyclic C3-C8 alkyl group, optionally interrupted with one or more oxygen atoms, and/or optionally substituted with one or more identical or different groups chosen from:
      i) -OR15
      ii) -NR16R17
      vi) -COOR16
      ix) a C5-C12 (hetero)aryl group, optionally containing one or more heteroatoms chosen from O, N and S, optionally substituted with one or more hydroxyls and/or with one or more C1-C8 alkoxy radicals;
      x) a saturated or unsaturated non-aromatic 5- to 8-membered heterocycle, comprising one or more heteroatoms chosen from O, N and S, optionally substituted with one or more hydroxyls and/or with one or more C1-C8 alkoxy or C1-C4 alkyl radicals;
   c) a phenyl group optionally substituted with one or more radicals chosen from hydroxyl and/or methoxy and/or ethoxy;
   d) -NR12R13;
   e) -OR14;
**R12** and **R13,** which may be identical or different, denoting a radical chosen from:
   a) -H;
   b) a saturated linear C1-C4 alkyl group;
   c) a phenyl group optionally substituted with one or more hydroxyls and/or with one or more C1-C8 alkoxy radicals;
**R14** denoting a radical chosen from:
   a) -H;
   b) a saturated linear C1-C4 alkyl group optionally substituted with a phenyl radical;
**R15** being chosen from H and a saturated linear C1-C4 or branched C3-C4 alkyl hydrocarbon-based group;
**R16** and **R17,** which may be identical or different, being chosen from H and a saturated linear C1-C10 or branched C3-C10 or unsaturated C2-C10 or cyclic C3-C8 alkyl hydrocarbon-based group; a (C1-C4)alkyl(hetero)(C6)aryl group optionally containing a nitrogen atom, especially a benzyl group; an acetyl radical;
**R16** and **R17** possibly forming, with the nitrogen that bears them, a 5- to 8-membered heterocycle, which may contain one or more heteroatoms or groups chosen from N, O and -CO- and/or optionally substituted with a C1-C10 hydrocarbon-based chain;
**R"** denotes a radical chosen from:
   a) -H;
   b) a linear saturated C1-C10 or branched C3-C10 alkyl group, optionally substituted with one or more identical or different groups -OR25; **R25** being chosen from H and a saturated linear C1-C4 alkyl hydrocarbon-based group;
it being understood that **A** and **R"** may form, with the nitrogen atom that bears them, a saturated or unsaturated 5- to 8-membered heterocycle, which may contain one or more heteroatoms or groups chosen from N, O and -CO- and/or optionally substituted with a C1-C4 hydrocarbon-based chain or C1-C4 hydroxyalkyl or CO2T, T denoting a hydrogen atom or a saturated linear C1-C6 or branched C3-C6 alkyl radical;
and the salts thereof, the solvates thereof, the optical isomers thereof and the racemic mixtures thereof, alone or as a mixture.

The particularly preferred products are chosen from:

| No. | Structure | Chemical name |
|---|---|---|
| 1 | 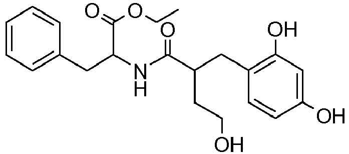 | ethyl N-[2-(2,4-dihydroxybenzyl)-4-hydroxybutanoyl]phenylalaninate |
| 2 | 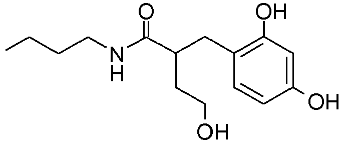 | N-butyl-2-(2,4-dihydroxybenzyl)-4-hydroxybutanamide |
| 3 | 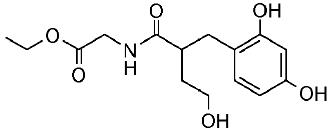 | ethyl N-[2-(2,4-dihydroxybenzyl)-4-hydroxybutanoyl]glycinate |
| 4 | 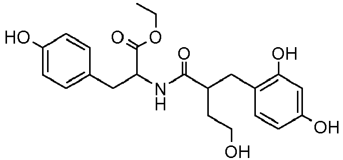 | ethyl N-[2-(2,4-dihydroxybenzyl)-4-hydroxybutanoyl]tyrosinate |
| 5 | 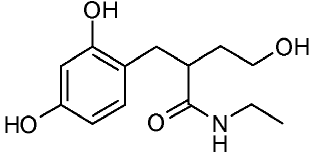 | 2-(2,4-dihydroxybenzyl)-N-ethyl-4-hydroxybutanamide |
| 6 | 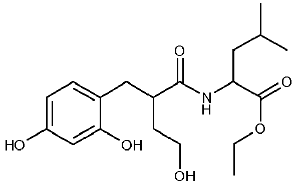 | ethyl N-[2-(2,4-dihydroxybenzyl)-4-hydroxybutanoyl]leucinate |
| 7 | 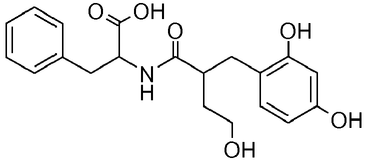 | N-[2-(2,4-dihydroxybenzyl)-4-hydroxybutanoyl]phenylalanine |
| 8 | 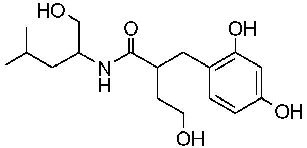 | 2-(2,4-dihydroxybenzyl)-4-hydroxy-N-(1-hydroxy-4-methylpentan-2-yl)butanamide |
| 9 | 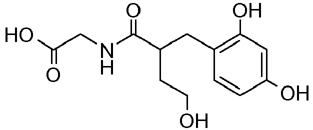 | N-[2-(2,4-dihydroxybenzyl)-4-hydroxybutanoyl]glycine |
| 10 | 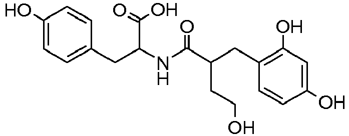 | N-[2-(2,4-dihydroxybenzyl)-4-hydroxybutanoyl]tyrosine |
| 11 | 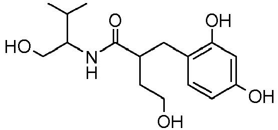 | 2-(2,4-dihydroxybenzyl)-4-hydroxy-N-(1-hydroxy-3-methylbutan-2-yl)butanamide |
| 12 | 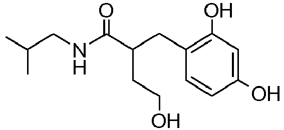 | 2-(2,4-dihydroxybenzyl)-4-hydroxy-N-(2-methylpropyl)butanamide |
| 13 | 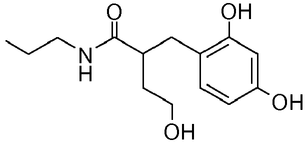 | 2-(2,4-dihydroxybenzyl)-4-hydroxy-N-propylbutanamide |
| 14 | 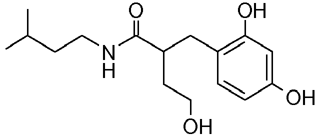 | 2-(2,4-dihydroxybenzyl)-4-hydroxy-N-(3-methylbutyl)butanam ide |
| 15 | 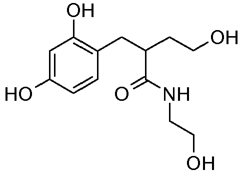 | 2-(2,4-dihydroxybenzyl)-4-hydroxy-N-(2-hydroxyethyl)butanamide |
| 16 | 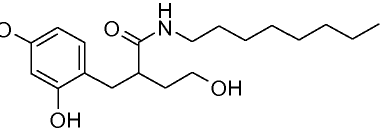 | 2-(2,4-dihydroxybenzyl)-4-hydroxy-N-octylbutanamide |
| 17 | 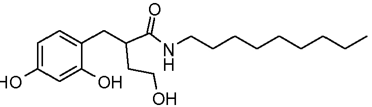 | 2-(2,4-dihydroxybenzyl)-4-hydroxy-N-nonylbutanamide |
| 18 | 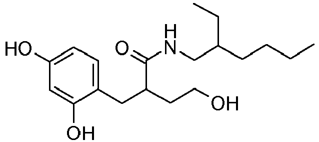 | 2-(2,4-dihydroxybenzyl)-N-(2-ethylhexyl)-4-hydroxybutanam ide |
| 19 | 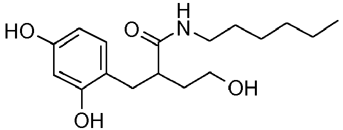 | 2-(2,4-dihydroxybenzyl)-N-hexyl-4-hydroxybutanamide |
| 20 | 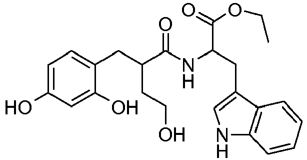 | ethyl N-[2-(2,4-dihydroxybenzyl)-4-hydroxybutanoyl]-3-(3H-indol-3-yl)-D-alaninate |
| 21 | 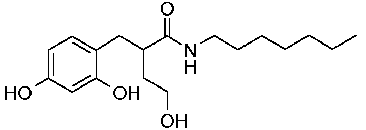 | 2-(2,4-dihydroxybenzyl)-N-heptyl-4-hydroxybutanamide |
| 22 | 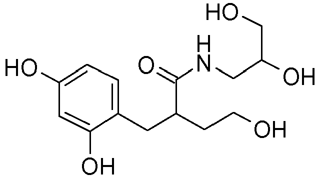 | 2-(2,4-dihydroxybenzyl)-N-(2,3-dihydroxypropyl)-4-hydroxybutanamide |
| 23 | 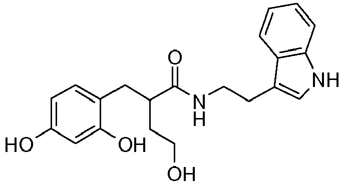 | 2-(2,4-dihydroxybenzyl)-4-hydroxy-N-[2-(1H-indol-3-yl)ethyl]butanamide |
| 24 | 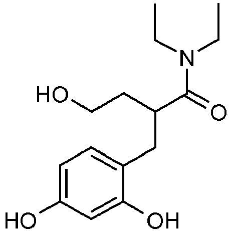 | 2-(2,4-dihydroxybenzyl)-N,N-diethyl-4-hydroxybutanam ide |
| 25 | 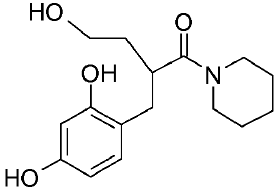 | 2-(2,4-dihydroxybenzyl)-4-hydroxy-1-(piperidin-1-yl)butan-1-one |
| 26 | 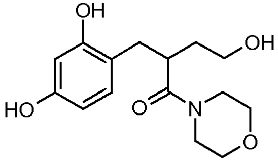 | 2-(2,4-dihydroxybenzyl)-4-hydroxy-1-(morpholin-4-yl)butan-1-one |
| 27 | 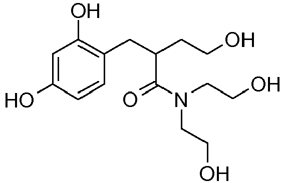 | 2-(2,4-dihydroxybenzyl)-4-hydroxy-N,N-bis(2-hydroxyethyl)butanamide |
| 28 | 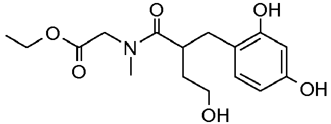 | ethyl N,N'-methyl[2-(2,4-dihydroxybenzyl)-4-hydroxybutanoyl]glycinate |
| 29 | 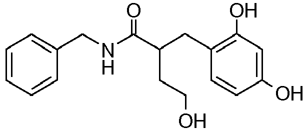 | 2-(2,4-dihydroxybenzyl)-4-hydroxy-N-benzylbutanamide |
| 30 | 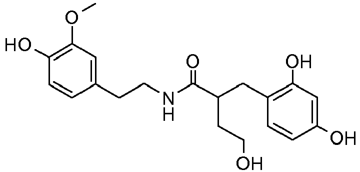 | 2-(2,4-dihydroxybenzyl)-4-hydroxy-N-[2-(4-hydroxy-3-methoxyphenyl)-ethyl]butyramide |
| 31 | 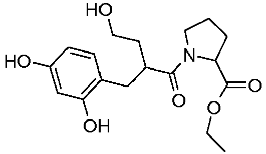 | ethyl N-[2-(2,4-dihydroxybenzyl)-4-hydroxybutanoyl]prolinate |
| 32 | 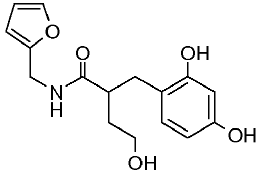 | 2-(2,4-dihydroxybenzyl)-N-(2,3-dihydroxypropyl)furfurylbutanamide |
| 33 | 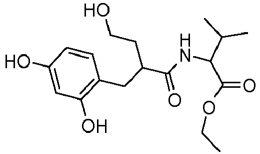 | ethyl N-[2-(2,4-dihydroxybenzyl)-4-hydroxybutanoyl]valinate |
| 34 | 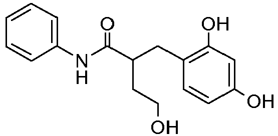 | 2-(2,4-dihydroxybenzyl)-N-(2,3-dihydroxypropyl)phenylbutanamide |
| 35 | 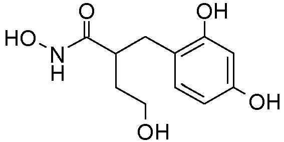 | 2-(2,4-dihydroxybenzyl)-N-(2,3-dihydroxypropyl)oxybutanamide |
| 36 | 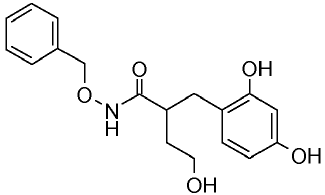 | 2-(2,4-dihydroxybenzyl)-N-(2,3-dihydroxypropyl)benzyloxy-butanamide |
| 37 | 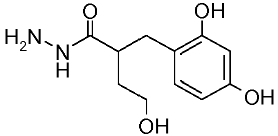 | 2-(2,4-dihydroxybenzyl)-N-(2,3-dihydroxypropyl)butyric acid hydrazide |
| 38 | 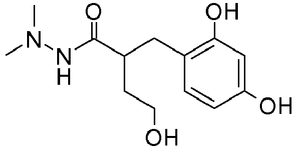 | 2-(2,4-dihydroxybenzyl)-N-(2,3-dihydroxypropyl)butyric acid N',N'-dimethylhydrazide |
| 39 | 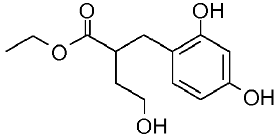 | 2-(2,4-dihydroxybenzyl)-4-hydroxyethyl butanoate |
| 40 | 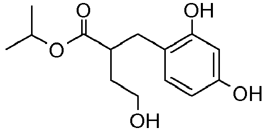 | 2-(2,4-dihydroxybenzyl)-4-hydroxyisopropyl butanoate |
| 41 | 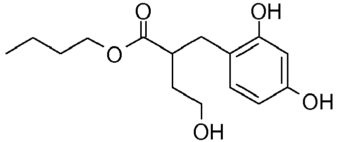 | 2-(2,4-dihydroxybenzyl)-4-hydroxybutyl butanoate |
| 42 | 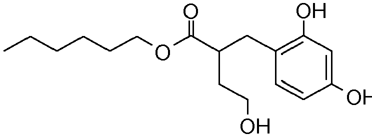 | 2-(2,4-dihydroxybenzyl)-4-hydroxyhexyl butanoate |
| 43 | 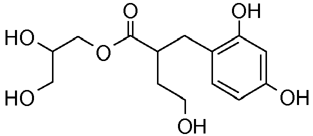 | 2-(2,4-dihydroxybenzyl)-4-hydroxybutyric acid 2,3-dihydroxypropyl ester |
| 44 | 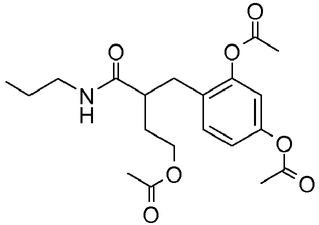 | acetic acid 5-acetoxy-2-(4-acetoxy-2-propylcarbamoylbutyl)phenyl ester |
| 45 | 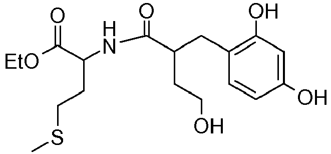 | ethyl N-[2-(2,4-dihydroxybenzyl)-4-hydroxybutanoyl]methioninate |
| 46 | 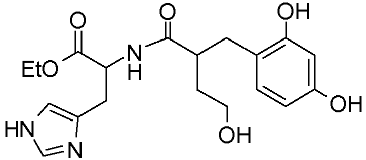 | ethyl N-[2-(2,4-dihydroxybenzyl)-4-hydroxybutanoyl]histidinate |
| 47 | 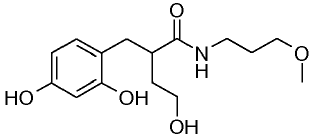 | 2-(2,4-dihydroxybenzyl)-N-methoxypropamino-4-hydroxybutanamide |
| 48 | 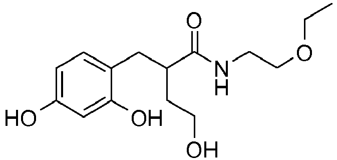 | 2-(2,4-dihydroxybenzyl)-N-(2-ethoxyethyl)-4-hydroxybutanamide |
| 49 | 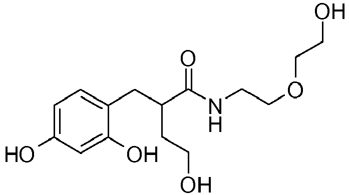 | 2-(2,4-dihydroxybenzyl)-N-[2-(2-hydroxyethoxy)ethyl]-4-hydroxybutanamide |
| 50 | 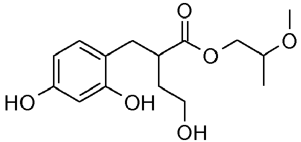 | 2-(2,4-dihydroxybenzyl)-4-hydroxybutyric acid 2-methoxypropyl ester |
| 51 | 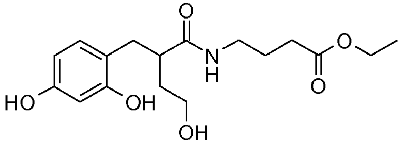 | 4-[2-(2,4-dihydroxybenzyl)-4-hydroxybutyrylamino]butyric acid ethyl ester |
| 52 | 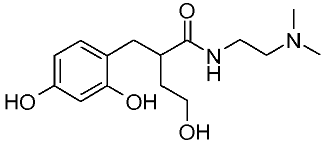 | 2-(2,4-dihydroxybenzyl)-N-(2-dimethylaminoethyl)-4-hydroxybutanamide |
| 53 | 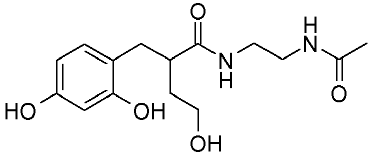 | 2-(2,4-dihydroxybenzyl)-N-(2-acetylaminoethyl)4-hydroxybutanamide |
| 54 | 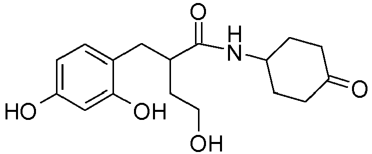 | 2-(2,4-dihydroxybenzyl)-N-(4-oxocyclohexyl)-4-hydroxybutanamide |
| 55 | 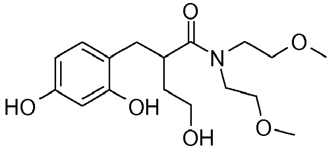 | 2-(2,4-dihydroxybenzyl)-N,N-bis(2-methoxyethyl)-4-hydroxybutanamide |
| 56 | 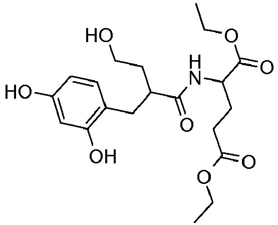 | 2-[2-(2,4-dihydroxybenzyl)-4-hydroxybutyrylamino]pentanedioic acid diethyl ester |
| 57 | 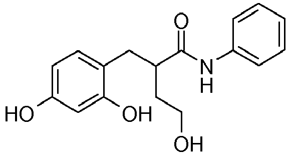 | 2-(2,4-dihydroxybenzyl)-N-phenyl-4-hydroxybutanamide |
| 58 | 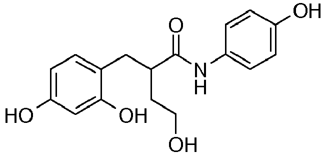 | 2-(2,4-dihydroxybenzyl)-N-para-hydroxyphenyl-4-hydroxybutanamide |
| 59 | 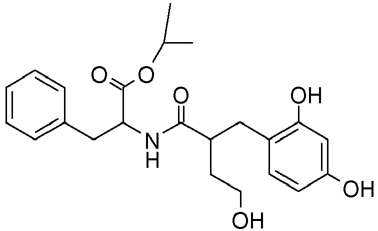 | isopropyl N-[2-(2,4-dihydroxybenzyl)-4-hydroxybutanoyl]phenylalaninate |

and the salts thereof, the solvates thereof, the optical isomers thereof and the racemic mixtures thereof, alone or as a mixture.

Among these compounds, the most particularly preferred compounds in the context of the present invention are the following: compounds 1 to 6, 12 to 25, 31, 33 and 59, and salts thereof, solvates thereof, isomers thereof and racemic mixtures thereof, taken alone or as a mixture.

The compounds of the invention of formula (I) may be prepared according to the following synthetic routes:

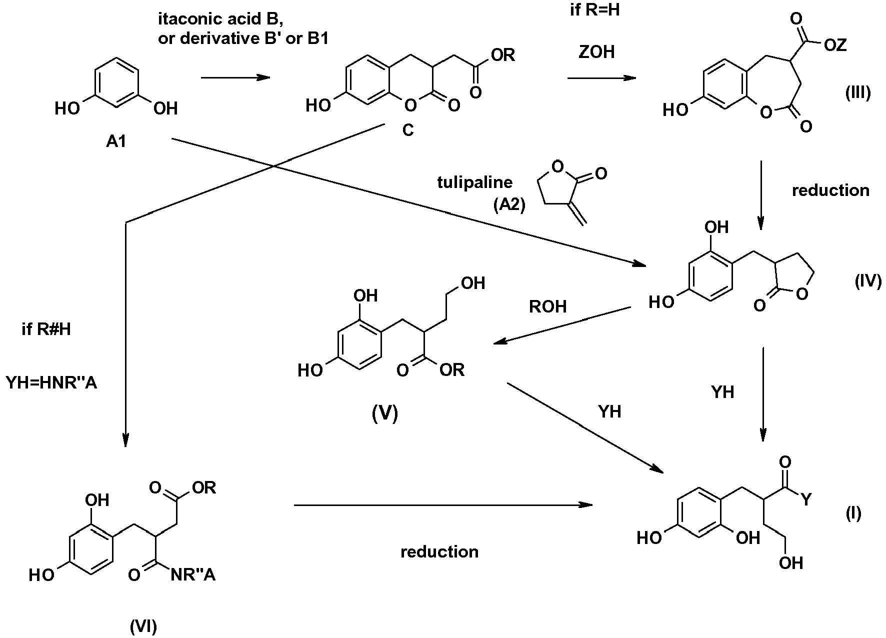

Resorcinol (A1) is reacted with itaconic acid (B) or its anhydride (B') or one of its esters of formula (B1) 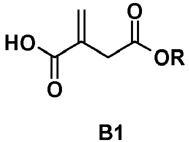 in which R denotes H or a linear C1-C6 or branched C3-C6 alkyl group,
to form a compound C, and this compound is then activated to form a heterocycle of formula (III), which is then subjected to a reduction step to form the intermediate (IV) which gives access to the compounds (V).

The intermediate of formula (IV) may also be accessed directly by reacting resorcinol (A1) with tulipaline (3-methylenedihydrofuran-2-one) (A2).

This step makes it possible to arrive directly at the compounds of formula (I) by reaction of derivatives YH. One variant consists in treating (IV) with an alcohol ROH to give the compounds (V), which, by reaction with YH, lead to (I).

To obtain the other compounds of formula (I),
compound C (R other than H) is reacted with a derivative YH = NHR"A in which A, R' and R" have the same meaning as described previously for the compounds of formula (I), optionally in the presence of a basic or acidic catalyst, optionally by heating to a temperature of between 15 and 200°C. This leads to the compounds of formula (VI), which, when subjected to a selective reduction of ester versus amide, makes it possible to obtain the compounds (I).

The reaction of resorcinol (A1) in the presence of itaconic acid (B) or its anhydride (B') or one of its esters of formula (B1) which are described above, in order to form the compound C, is especially performed in the presence of an organic solvent which may be chosen from toluene, tetrahydrofuran, heptane, isooctane, methyltetrahydrofuran, methyl ethyl ketone, methyl isobutyl ketone, dioxane, ethyl acetate, isopropyl acetate, isododecane and mixtures thereof, in particular at a temperature of between 15 and 200°C, optionally in the presence of a catalyst (acidic or basic) as described in the publications: Synthesis of 7-hydroxycoumarins via the Pechmann reaction using Nafion resin/silica nanocomposites as catalysts: Laufer MC, Hausmann H and Hölderich WF, J. of Catalysis, 2003, 218, 315-320; Synthesis of 7-hydroxycoumarins catalysed by solid acid catalysts: Hoefnagel A, Gunnewegh E, Downing R and van Bekkum H, J. Chem. Soc., Chem. Commun., 1995, 225-226; in particular in the presence of an acid catalyst, such as methanesulfonic acid, triflic acid, para-toluenesulfonic acid or sulfonic resins, such as the Dowex® products or the Amberlyst® products (sold by the company Aldrich).

The compounds (B1) may be obtained in a known way by selective esterification in an acidic medium of itaconic acid with one or more alcohols of formula ROH, as described in the literature (Selective esterification of nonconjugated carboxylic acids in the presence of conjugated or aromatic carboxylic acids over active carbon supported methanesulfonic acid; Feng, Ze Wang, Zhao, Xin Qi and Bi, Hua, Science in China, Series B: Chemistry (2008), 1(10), 990-992 / An efficient and regiospecific esterification of dioic acids using PTSA; Devi, A. Rama and Rajaram, S., Indian Journal of Chemistry, Section B: Organic Chemistry Including Medicinal Chemistry (2000), 39B(4), 294-296 / A simple method for the preparation of monomethyl esters of dicarboxylic acids by selective esterification of the nonconjugated carboxyl group in the presence of an aromatic or conjugated carboxyl group; Ram, Ram N. and Meher, Nabin Kumar, Journal of Chemical Research, Synopses (2000), (6), 282-283).

According to a specific embodiment of the process of synthesis, when the R group of the compound C denotes H, the compound of formula (I) may be obtained by activation of the acid C according to the known acid activation methods, described especially in Comprehensive Organic Transformations by R. Larock, published by Wiley VCH, in the chapter Interconversion of nitriles, carboxylic acids and derivatives.

Compound C in acid form is then esterified in the presence of ethanol under standard conditions of heating (between 15 and 100°C) and of acid catalysis to give a carboxylic acid ethyl ester derivative (A3), which, when activated according to the known acid activation methods, described especially in Comprehensive Organic Transformations by R. Larock, published by Wiley VCH, in the chapter Interconversion of nitriles, carboxylic acids and derivatives, leads to the heptacyclic lactone of formula (III). This lactone, subjected to a reduction with hydride, gives the compound of formula (IV) after acidic work-up. This reduction may be performed between -30 and +60°C in protic or aprotic solvents (tetrahydrofuran, dioxane, ethanol, methanol) with metal hydride donors, such as sodium borohydride, sodium triacetoxyborohydride or diisobutylaluminium hydride.

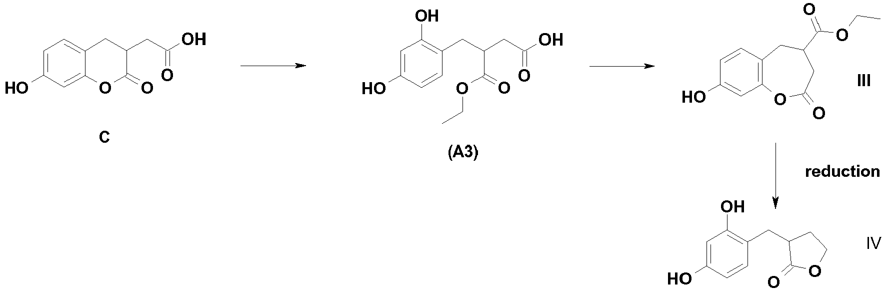

The reaction of resorcinol (A1) in the presence of tulipaline (3-methylene-dihydrofuran-2-one) (A2), and leading to compound (IV), is especially performed in the presence of an organic solvent which may be chosen from toluene, tetrahydrofuran, heptane, isooctane, methyltetrahydrofuran, methyl ethyl ketone, methyl isobutyl ketone, dioxane, ethyl acetate, isopropyl acetate and isododecane, and mixtures thereof, especially at a temperature of between 15 and 200°C, optionally in the presence of a catalyst (acidic or basic) as described in the publications: Synthesis of 7-hydroxycoumarins by Pechmann reaction using Nafion resin/silica nanocomposites as catalysts: Laufer MC, Hausmann H and Hölderich WF, J. of Catalysis, 2003, 218, 315-320; Synthesis of 7-hydroxycoumarins catalysed by solid acid catalysts: Hoefnagel A, Gunnewegh E, Downing R and van Bekkum H, J. Chem. Soc., Chem. Commun., 1995, 225-226; in particular in the presence of an acid catalyst, such as methanesulfonic acid, triflic acid, para-toluenesulfonic acid or sulfonic resins, such as the Dowex® products or the Amberlyst® products (sold by the company Aldrich).

The reaction of the compound of formula (IV) with a compound of formula YH 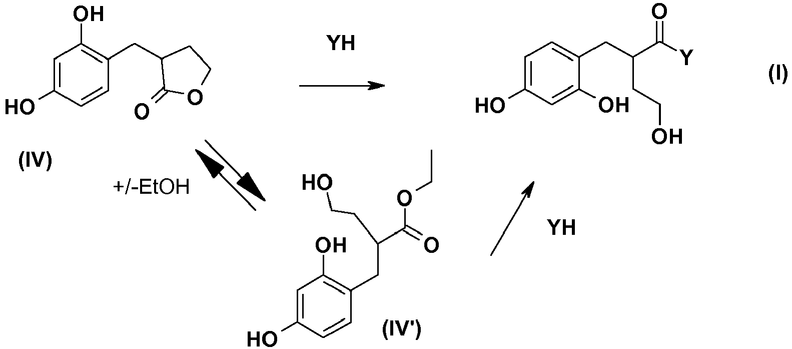 is optionally performed in the presence of an organic solvent, in particular tetrahydrofuran, dioxane, dimethylformamide, dimethyl sulfoxide, 2-methyltetrahydrofuran, dichloromethane, toluene, methanol or ethanol,
optionally in the presence of a catalyst chosen from acid catalysts of Lewis or Bronsted type or basic catalysts, such as potassium carbonate, triethylamine or diisopropylethylamine;
optionally by heating to a temperature of between 15°C and 200°C, in particular between 30°C and 150°C.

It should be noted that the lactone (IV) may be in equilibrium with the ethyl ester form (IV') in the presence of ethanol. The acyclic ester can then react with YH compounds in order to result in the compounds (I).

Alternatively, if R is other than H, the compounds of formula (I) may be obtained by opening the lactone C with derivatives YH, for example and especially the amines (YH = NHR"A), having the same meaning as described previously for the compounds of formula (I), optionally in the presence of a protic or aprotic organic solvent, especially tetrahydrofuran, dioxane, dimethylformamide, dimethyl sulfoxide, 2-methyltetrahydrofuran, dichloromethane, methanol or ethanol;
optionally in the presence of a catalyst chosen from acid catalysts of Lewis or Bronsted type or basic catalysts, such as potassium carbonate, triethylamine or diisopropylethylamine;
optionally by heating to a temperature of between 15°C and 200°C, in particular between 30°C and 150°C.

The compounds (VI) for which R = ethyl are thus obtained. These compounds are then subjected to a reduction with hydride to give the compounds I after acidic work-up. This reduction may be performed at between -30 and +60°C in protic or aprotic solvents (tetrahydrofuran, dioxane, ethanol, methanol) with metal hydride donors, such as sodium borohydride, sodium triacetoxyborohydride or diisobutylaluminium hydride.

Mention may be made, as acid activation methods, of:
- the intermediate formation of acid chloride (for example by using thionyl or oxalyl chloride, or 1-chloro-N,N,2-trimethyl-1-propenamine),
- the intermediate formation of a mixed anhydride (for example by using a C₂-C₆ alkyl chloroformate, such as isobutyl chloroformate, in the presence of a base, for instance triethylamine or diisopropylethylamine, ;
- the intermediate formation of carbamimidate or of acylphosphonate (for example by using carbodiimides or diethyl cyanophosphate; Phosphorus in organic synthesis-XI, Amino acids and peptides-XXI, Reaction of diethyl phosphorocyanidate with carboxylic acids. A new synthesis of carboxylic esters and amides, Tetrahedron, 32, 1976, 2211-2217).

All of these steps may also resort to protection/deprotection strategies normally used in organic chemistry and compiled in the book Protecting Groups in Organic Synthesis, Greene and Wuts, Wiley Interscience, as a function of the nature of the radicals R', A and R".

In addition, when the final compounds (I) bear a free carboxylic acid on the radical A or R", these compounds may be obtained by saponification using mineral bases, for instance NaOH or LiOH, in the presence of protic or aprotic solvents, for instance ethanol or tetrahydrofuran or water, at temperatures ranging between 0 and 100°C. The salts obtained are then re-acidified in the presence of conventional mineral or organic acids, for instance hydrochloric acid or citric acid.

The compounds of formula (I) according to the invention have a very particular application in the cosmetics field.

The composition according to the invention comprises, in a physiologically acceptable medium, a compound of formula (I) as described above.

The term "physiologically acceptable medium" is understood to mean a medium that is compatible with human keratin materials, such as bodily skin or facial skin, the lips, mucous membranes, the eyelashes, the nails, the scalp and/or the hair.

Compound (I) may be present in the composition according to the invention in an amount that may be between 0.01% and 10% by weight, preferably between 0.1% and 5% by weight and especially from 0.5% to 3% by weight relative to the total weight of the composition.

The composition according to the invention is advantageously a cosmetic composition: it can comprise adjuvants normally employed in the cosmetics field.

Mention may especially be made of water, organic solvents, in particular C₂-C₆ alcohols, oils, especially hydrocarbon-based oils or silicone oils, waxes, pigments, fillers, dyes, surfactants, emulsifiers, cosmetic active agents, UV screening agents, polymers, thickeners, preserving agents, fragrances, bactericides, odour absorbers or antioxidants.

These optional cosmetic adjuvants may be present in the composition in a proportion of 0.001% to 80% by weight and especially 0.1% to 40% by weight relative to the total weight of the composition. In any case, these adjuvants, and the proportions thereof, will be chosen by a person skilled in the art so that the advantageous properties of the compounds according to the invention are not, or not substantially, adversely affected by the envisaged addition.

As active agents, it will be advantageous to introduce, into the composition according to the invention, at least one compound chosen from: desquamating agents; calmatives; organic or mineral photoprotective agents; moisturizers; depigmenting agents; antiglycation agents; NO-synthase inhibitors; agents which stimulate the synthesis of dermal or epidermal macromolecules and/or which prevent their decomposition; agents which stimulate the proliferation of fibroblasts and/or keratinocytes or which stimulate the differentiation of keratinocytes; muscle relaxants and/or dermo-decontracting agents; tensioning agents; antipollution agents and/or free-radical scavengers; agents which act on the capillary circulation; agents which act on the energy metabolism of cells; and mixtures thereof.

Examples of such additional compounds are: retinol and derivatives thereof, such as retinyl palmitate; ascorbic acid and derivatives thereof, such as magnesium ascorbyl phosphate and ascorbyl glucoside; tocopherol and derivatives thereof, such as tocopheryl acetate; nicotinic acid and precursors thereof, such as nicotinamide; ubiquinone; glutathione and precursors thereof, such as L-2-oxothiazolidine-4-carboxylic acid; plant extracts, and in particular plant proteins and hydrolysates thereof, and also phytohormones; marine extracts, such as algal extracts; bacterial extracts; sapogenins such as diosgenin and extracts of wild yam containing the same; ceramides; hydroxy acids, such as salicylic acid and 5-n-octanoylsalicylic acid; resveratrol; oligopeptides and pseudodipeptides and acylated derivatives thereof; manganese salts and magnesium salts, in particular gluconates; and mixtures thereof.

The term "desquamating agent" is understood to mean any compound capable of having an effect:
- either directly on desquamation by promoting exfoliation, such as β-hydroxy acids, in particular salicylic acid and its derivatives (including 5-(n-octanoyl)salicylic acid); α-hydroxy acids, such as glycolic, citric, lactic, tartaric, malic or mandelic acid; urea; gentisic acid; oligofucoses; cinnamic acid; *Saphora japonica* extract; or resveratrol;
- or on the enzymes involved in desquamation or decomposition of the corneodesmosomes, such as glycosidases, stratum corneum chymotryptic enzyme (SCCE) or indeed even other proteases (trypsin, chymotrypsin-like). Mention may be made of agents which chelate mineral salts: EDTA; N-acyl-N,N',N'-ethylenediaminetriacetic acid; aminosulfonic compounds, in particular N-(2-hydroxyethyl)piperazine-N-2-ethanesulfonic acid (HEPES); 2-oxothiazolidine-4-carboxylic acid (procysteine) derivatives; derivatives of α-amino acids of glycine type (such as described in EP 0 852 949, and also sodium methyl glycine diacetate, sold by BASF under the trade name Trilon M); honey; or sugar derivatives, such as O-octanoyl-6-D-maltose and N-acetylglucosamine.

The desquamating agents are generally present in the composition according to the invention in proportions ranging from 0.01% to 15% by weight and preferably ranging from 0.1% to 10% by weight relative to the total weight of the composition.

As calmatives that may be used in the composition according to the invention, mention may be made of: pentacyclic triterpenes and extracts of plants (for example *Glycyrrhiza glabra*) comprising the same, such as β-glycyrrhetinic acid and its salts and/or its derivatives (glycyrrhetinic acid monoglucuronide, stearyl glycyrrhetinate, 3-(stearoyloxy)glycyrrhetic acid), ursolic acid and its salts, oleanolic acid and its salts, betulinic acid and its salts, a *Paeonia suffruticosa* and/or *lactiflora* extract, salicylic acid salts and in particular zinc salicylate, phycosaccharides from the company Codif, a *Laminaria saccharina* extract, canola oil, bisabolol, camomile extracts, allantoin, Sepivital EPC (phosphoric diester of vitamins E and C) from SEPPIC, omega-3 unsaturated oils, such as musk rose oil, blackcurrant oil, ecchium oil or fish oil, plankton extracts, capryloyl glycine, Seppicalm VG (sodium palmitoylproline and *Nymphaea alba*) from SEPPIC, a *Pygeum* extract, a *Boswellia serrata* extract, a *Centipeda cunninghamii* extract, a *Helianthus annuus* extract, a *Linum usitatissimum* extract, tocotrienols, *Cola nitida* extracts, piperonal, a clove extract, an *Epilobium angustifolium* extract, aloe vera, a *Bacopa moniera* extract, phytosterols, cortisone, hydrocortisone, indomethacin and betamethasone. The calmatives are generally present in the composition according to the invention in proportions ranging from 0.01% to 15% by weight and preferably ranging from 0.1% to 10% by weight, relative to the total weight of the composition.

The organic photoprotective agents are chosen in particular from anthranilates; cinnamic derivatives; dibenzoylmethane derivatives; salicylic derivatives; camphor derivatives; triazine derivatives, such as those described in patent applications US 4 367 390, EP 863 145, EP 517 104, EP 570 838, EP 796 851, EP 775 698, EP 878 469, EP 933 376, EP 507 691, EP 507 692, EP 790 243 and EP 944 624; benzophenone derivatives; β,β-diphenylacrylate derivatives; benzotriazole derivatives; benzalmalonate derivatives; benzimidazole derivatives; imidazolines; bis-benzazolyl derivatives, such as described in patents EP 669 323 and US 2 463 264; p-aminobenzoic acid (PABA) derivatives; methylenebis(hydroxyphenylbenzotriazole) derivatives, such as described in patent applications US 5 237 071, US 5 166 355, GB 2 303 549, DE19726184 and EP 893 119; screening polymers and screening silicones, such as those described in particular in patent application WO-93/04665; α-alkylstyrene-based dimers, such as those described in patent application DE19855649; and merocyanin derivatives, such as those described in patent applications WO 2011/113719 and FR 2 957 251.

The mineral photoprotective agents may be chosen in particular from coated or non-coated metal oxide pigments or nanopigments (mean size of the primary particles generally of between 5 nm and 100 nm, preferably between 10 nm and 50 nm), for instance titanium oxide (amorphous or crystallized in rutile and/or anatase form), iron oxide, zinc oxide, zirconium oxide or cerium oxide nanopigments, which are all well-known UV photoprotective agents. Conventional coating agents are furthermore alumina and/or aluminium stearate. Such coated or non-coated metal oxide nanopigments are described in particular in patent applications EP 518 772 and EP 518 773.

The photoprotective agents are generally present in the composition according to the invention in proportions ranging from 0.1% to 20% by weight and preferably ranging from 0.2% to 15% by weight relative to the total weight of the composition.

The composition according to the invention may be in any galenical form normally used in the cosmetics field and in particular in the form of an aqueous or aqueous/alcoholic solution, which is optionally gelled, a dispersion of the lotion type, optionally comprising two phases, an oil-in-water or water-in-oil or multiple (W/O/W or O/W/O, for example) emulsion, an aqueous gel, a dispersion of oil in an aqueous phase using spherules, it being possible for these spherules to be polymeric nanoparticles, such as nanospheres and nanocapsules or better still lipid vesicles of ionic and/or nonionic type, or an aqueous or oily gel. These compositions are prepared according to the usual methods. Use is preferably made, according to this invention, of a composition in the form of an emulsion, in particular an oil-in-water emulsion.

The composition according to the invention can constitute a composition for caring for the skin and in particular a cleansing, protecting, treating or care cream for the face, for the hands, for the feet, for the major anatomical folds or for the body (for example, day creams, night creams, make-up-removing creams, foundation creams or anti-sun creams); a fluid foundation; a make-up-removing milk, a protective or care body milk or an anti-sun milk; or a lotion, gel or mousse for caring for the skin, such as a cleansing lotion.

The invention is illustrated in more detail by the following non-limiting examples.

### Method 1 for obtaining the compounds (I):

a) Synthesis of 3-(2,4-dihydroxybenzyl)dihydrofuran-2(3H)-one of formula (IV) 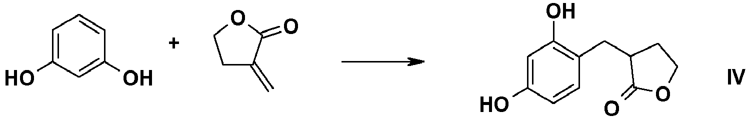
   To a solution of 11 g of resorcinol and 10 g of 3-methylenedihydrofuran-2-one (CAS 547-65-9) in 100 ml of a 50/50 toluene/isooctane mixture are added 20 g of Amberlyst 15 resin from Aldrich. The reaction mixture is heated at 120°C for 72 hours with, if necessary, addition of further resin. After cooling, 100 ml of methyltetrahydrofuran are added and the reaction medium is stirred for 1 hour. The mixture is filtered and the filtrate is concentrated under vacuum before purifying on a column of silica (eluent: 60/40 heptane/ethyl acetate) to give a colourless oil which may solidify, corresponding to compound IV (14% yield). The ¹H NMR and mass spectra are in accordance with the structure of the expected product (IV).
b) Compound IV is reacted with compound YH, used as reagent and solvent when YH is liquid, for example when YH is an amine (3 equivalents), and the reaction medium is then maintained at 60°C for 2 hours.

In the case where YH is solid, methyltetrahydrofuran can be added in a sufficient amount to dissolve the reaction medium.

At the end of the reaction, the reaction medium is concentrated under vacuum. The crude product is dissolved in methyltetrahydrofuran, washed with a 0.1N hydrochloric acid solution and then washed with water. The organic phase is collected, dried over magnesium sulfate and concentrated under vacuum. The crude product is purified on a chromatography column to give the expected product.

### Method 2 for obtaining the compounds (I):

a) Production of the lactone (III) in acid form 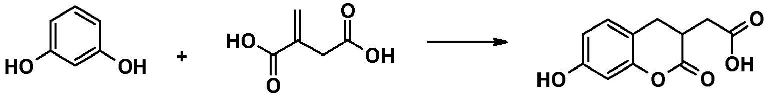
   10 g of resorcinol and 11.8 g of itaconic acid were dissolved in 150 ml of a toluene/dioxane mixture (1/1 ratio by volume) in the presence of Amberlyst 15 resin from Aldrich in a round-bottomed flask equipped with a Dean and Stark apparatus. The reaction medium was heated at 100°C for 3 hours. After cooling, the crude reaction product was filtered and the filtrate was concentrated under vacuum. The crude product was recrystallized under hot conditions from ethyl acetate. 10 g of a white powder corresponding to the expected product were obtained (yield of 50%).
   Melting point: 174-175°C. The ¹H NMR and mass spectra are in accordance with the structure of the expected product.
b) Production of the heptacyclic lactone III 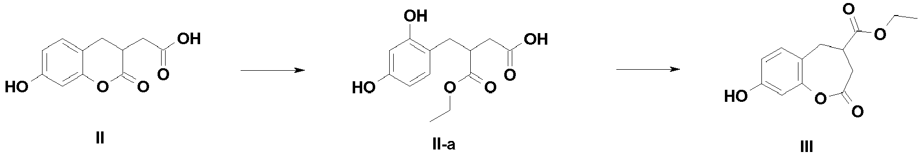
   Compound II (1.6 mmol) in acid form is refluxed in ethanol (5 ml) for 2 hours. After concentrating under vacuum, the residue is purified on a column of silica (eluent: 1/1 heptane/ethyl acetate) to give 300 mg of a colourless oil (70% yield) characterized by NMR and MS as compound II-a: 2-(2,4-Dihydroxybenzyl)succinic acid 1-ethyl ester. This synthesis may be extrapolated to a larger scale enabling the intermediate to be accumulated in larger amount.
   Compound II-a (2 g) is dissolved in 20 ml of dichloromethane, followed by addition of 1.25 eq. of EDCI (1-ethyl-3-(3-dimethylaminopropyl)carbodiimide), 1.25 eq. of HOBT (hydroxybenzotriazole) and 3 eq. of triethylamine. The reaction medium is stirred at room temperature for 2 hours. After concentrating under vacuum, the crude product is purified on a column of silica (2/1 heptane/ethyl acetate) to give compound III in the form of a pale yellow oil (44% yield). The ¹H NMR and mass spectra are in accordance with the structure of the expected product (III).
c)Synthesis of compound (IV) from (III): 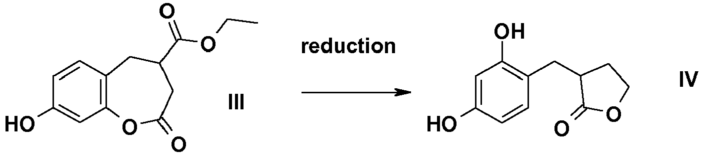
   To a solution of 0.34 mmol of III in 2 ml of tetrahydrofuran are added 1.1 eq. (0.37 mmol) of sodium borohydride at room temperature. After stirring for 1 hour, the reaction is stopped by addition of 1N hydrochloric acid. The mixture is then extracted three times with ethyl acetate. The organic phase is dried over sodium sulfate and then concentrated to dryness under vacuum. The crude product is purified on a column of silica (heptane/ethyl acetate) to give compound IV in the form of a very pale yellow pasty solid (60% yield). The ¹H NMR and mass spectra are in accordance with the structure of the expected product (IV).
d) Compound (IV) is added to compound YH, used as reagent and solvent, for example an amine (3 equivalents), and the reaction medium is then maintained at 60°C for 2 hours.

In the case where YH is solid, methyltetrahydrofuran is added in a sufficient amount to dissolve the reaction medium.

At the end of the reaction, the medium is concentrated under vacuum. The crude product is dissolved in methyltetrahydrofuran, washed with a 0.1N hydrochloric acid solution and then washed with water. The organic phase is collected, dried over sodium sulfate and concentrated under vacuum. The crude product is purified on a chromatography column to give the expected product.

### Method 3 for obtaining the compounds (I): in the case where YH is a secondary amino derivative, for example

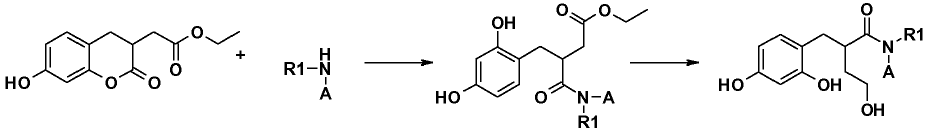

a) Synthesis of ethyl 7-hydroxy-2-oxo-3,4-dihydro-2H-chromen-3-yl)acetate (II in ethyl ester form): 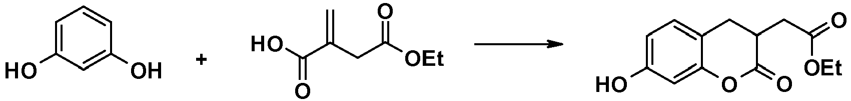
   4.4 g (0.04 mol) of resorcinol and 6.32 g (0.04 mol) of itaconic acid ethyl half-ester, and also 8.8 g of Amberlyst 15 resin from Aldrich, were added to 100 ml of toluene. The reaction mixture was refluxed and stirred for 2 hours and then filtered after cooling. The filtrate was concentrated and purified by chromatography on a column of silica (eluent: 50/1 CH₂Cl₂/MeOH) to give, after recrystallization from a 3/1 hexane/ethyl acetate mixture, 4.6 g (46% yield) of the expected lactone in the form of a white solid.
   Melting point: 102-103°C
   The ¹H NMR and mass spectra are in accordance with the structure of the expected product.
b) Synthesis of the acyclic amido-ethyl ester:
   To a solution of 2 g (8 mmol) of ethyl(7-hydroxy-2-oxo-3,4-dihydro-2H-chromen-3-yl)acetate are added 20 ml of the secondary amine as reagent and solvent. The mixture is heated at 80°C for 3 hours. After concentrating under vacuum, the crude reaction product is purified by chromatography on a column of silica (eluent: 30/1 dichloromethane/methanol) to give the desired compound.
c) Reduction of the ester function:
   The amido-ethyl ester (4.27 mmol) obtained previously is dissolved in 30 ml of tetrahydrofuran. 3 equivalents of lithium aluminium hydride are added at room temperature. After stirring for 3 hours, methanol is added and the reaction medium is then concentrated under vacuum. The crude reaction product is then purified on a column of silica (eluent: dichloromethane/methanol) to give the desired product (I).

### Example 1: synthesis of compound 1: Method 1 starting with compound (IV)

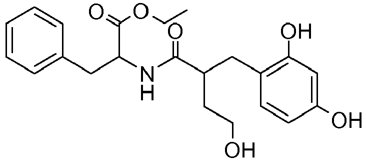

12 g of compound IV and 6 equivalents of ethyl phenylalaninate (67 g) are placed in a round-bottomed flask. The reaction medium is heated at 140°C for 3 hours. After cooling, the reaction medium is filtered. The filtrate is taken up in ethyl acetate and washed twice with 1N hydrochloric acid. After drying the organic phase over sodium sulfate and concentrating under vacuum, an orange-coloured oil is obtained (18 g). This oil is taken up in diisopropyl ether to give, after precipitation and filtration, 12.6 g (52% yield) of a pale yellow solid corresponding to compound 1, in the form of a mixture of two diastereoisomers in a 52/48 ratio determined by ¹H NMR.

The ¹H NMR and mass spectra are in accordance with the structure of the expected mixture of diastereoisomers.

### Example 2: synthesis of compound 3: Method 2 starting with compound IV

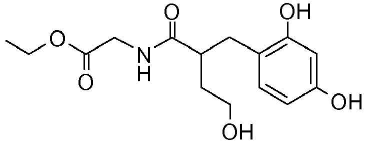

3.3 g of compound IV and 4.4 equivalents of ethyl glycinate (1.5 g) are placed in a round-bottomed flask. The reaction medium is heated at 130°C for 2 hours. After cooling, the reaction medium is diluted in methanol and filtered. The filtrate is concentrated under vacuum to give, after purification on a column of silica (eluent: 40/1 dichloromethane/methanol), 260 mg of a white solid corresponding to compound 3 (25% yield).

The ¹H NMR and mass spectra are in accordance with the structure of the expected product.
m.p.: 51-54°C

### Example 3: synthesis of compound 9: saponification of compound 3

800 mg of compound 3 are placed in 15 ml of tetrahydrofuran, and 4.5 ml of 2N lithium hydroxide solution are added dropwise. After stirring for 2 hours, the reaction is acidified to pH 2 with 1N hydrochloric acid. After concentrating under vacuum, the residue is purified by column chromatography on silica (eluent: 1/30 methanol/dichloromethane) to give the acid compound 4 in the form of a white solid. (Yield = 44%).

The ¹H NMR and mass spectra are in accordance with the structure of the expected product.
m.p.: 55-60°C

### Example 4: synthesis of compound 24: method 3

A mixture of 2 g of compound II (ethyl ester form) and 20 ml of diethylamine is heated at 80°C for 3 hours. After cooling, the mixture is concentrated under vacuum. The crude product obtained is purified by chromatography on a column of silica (eluent: 30/1 dichloromethane/methanol). The desired amido-ester is thus obtained in the form of a white solid in a yield of 43%.

1.37 g of this product are then placed in 30 ml of tetrahydrofuran, and 0.47 g (3 equivalents) of LiAlH₄ is added portionwise. After stirring at room temperature for 3 hours, cold methanol is added to the reaction medium. The medium is concentrated under vacuum and the residue is then purified on a column of silica (30/1 dichloromethane/methanol), to give 192 mg of a white solid corresponding to compound 24 (15% yield).

The ¹H NMR and mass spectra are in accordance with the structure of the expected product.
m.p.: 128-130°C

### Examples 5 to 32: Synthesis of the compounds (including those mentioned previously)

The same procedure was carried out as in the examples described above, using a different amine specified in the table below:

| No. | Structure | Reacting amine | Synthetic method | Final stage yield (%) | Analyses |
|---|---|---|---|---|---|
| 1 | 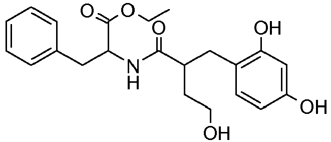 | Ethyl phenylalaninate | 1 | 52 | NMR/MS Compliant |
| 2 | 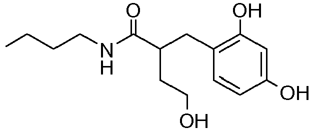 | Butylamine | 2 | 31 | NMR/MS Compliant m.p. 125-126°C |
| 3 | 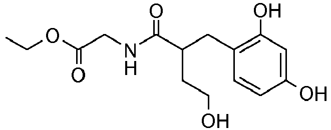 | Ethyl glycinate | 2 | 25 | NMR/MS Compliant m.p. 51-54°C |
| 4 | 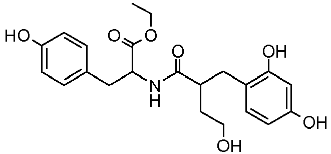 | Ethyl tyrosinate | 2 | 28 | NMR/MS Compliant m.p. 61-65°C |
| 5 | 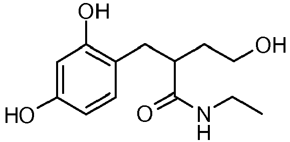 | Ethylamine | 2 | 42 | NMR/MS Compliant m.p. 133-135°C |
| 6 | 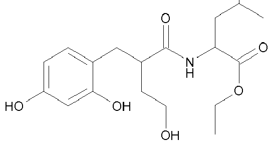 | Ethyl leucinate | 1 | 36 | NMR/MS Compliant |
| 7 | 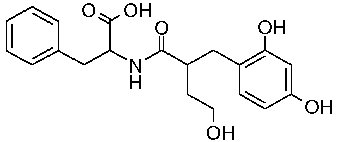 | Ethyl leucinate, followed by sapon ification | 2 | 43 | NMR/MS Compliant m.p. 86-91°C |
| 8 | 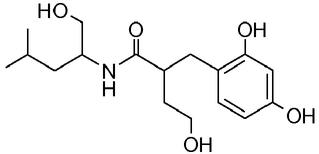 | Leucinol | 2 | 20 | NMR/MS Compliant |
| 9 | 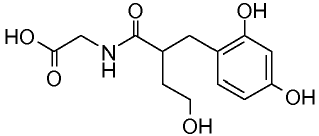 | Ethyl glycinate, followed by sapon ification | 2 | 46 | NMR/MS Compliant m.p. 55-60°C |
| 10 | 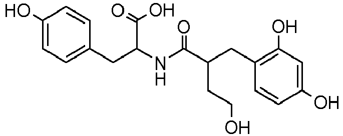 | Ethyl tyrosinate, followed by sapon ification | 2 | 14 | NMR/MS Compliant m.p. 152-156°C |
| 11 | 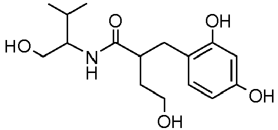 | Valinol | 2 | 43 | NMR/MS Compliant |
| 12 | 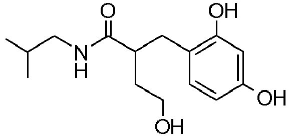 | Isobutylamine | 2 | 34 | NMR/MS Compliant |
| 13 | 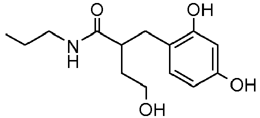 | Propylamine | 2 | 38 | NMR/MS Compliant |
| 14 | 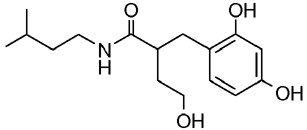 | Isoamylamine | 2 | 61 | NMR/MS Compliant |
| 15 | 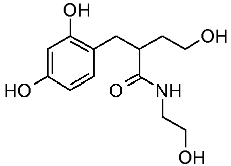 | Ethanolamine | 2 | 26 | NMR/MS Compliant |
| 16 | 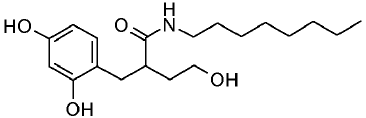 | Octylamine | 1 | 46 | NMR/MS Compliant |
| 17 | 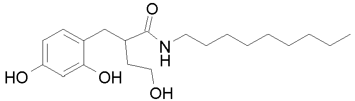 | Nonylamine | 1 | 39 | NMR/MS Compliant |
| 18 | 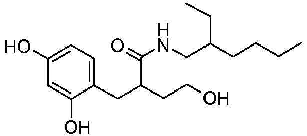 | 2-Ethylhexylamine | 1 | 35 | NMR/MS Compliant |
| 19 | 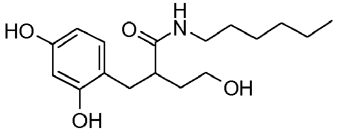 | Hexylamine | 1 | (loss of product) | NMR/MS Compliant |
| 20 | 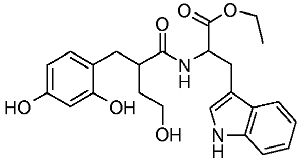 | Ethyl tryptophanate | 1 | 33 | NMR/MS Compliant |
| 21 | 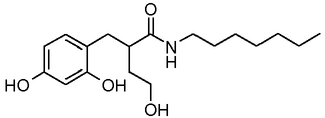 | Heptylamine | 1 | 42 | NMR/MS Compliant |
| 22 | 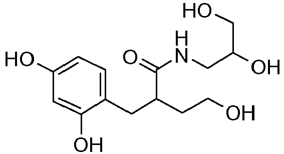 | 2,3-Dihydroxypropylamine | 1 | 32 | NMR/MS Compliant |
| 23 | 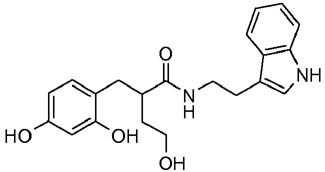 | Tryptamine | 1 | 20 | NMR/MS Compliant |
| 24 | 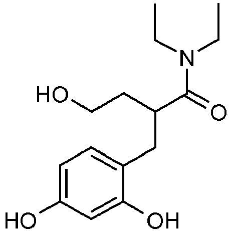 | Diethylamine | 3 | 15 | NMR/MS Compliant m.p. 128-130°C |
| 25 | 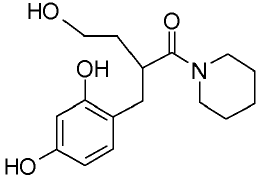 | Piperidine | 3 | 10 | NMR/MS Compliant |
| 26 | 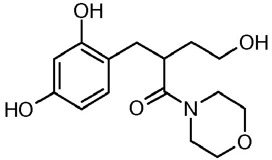 | Morpholine | 3 | 12 | NMR/MS Compliant m.p. 133-136°C |
| 27 | 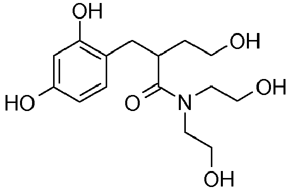 | Diethanolamine | 3 | 13 | NMR/MS Compliant |

### Example 33: Example of separation of the diastereoisomers and characterization of the latter

Compound 1 is subjected to a preferential crystallization in hot dichloromethane (20 volumes w/w). A diastereoisomer precipitates out (1-a) during the temperature decrease. The second one is present in the filtrate (1-b).

Another method consists in and performing a separation using a preparative HPLC system.
Instrument: SFC-80 (Thar, Waters)
Column: Chiralpak OJ-H 30*250 mm 5 um (Daicel)
Column temperature: 40°C
Mobile phase: CO₂/ Ethanol = 85/15
Flow: 80 g/min
Back pressure: 100 bar
Cycle time of stack injection: 4.4 min
Load per injection: 23 mg
Retention time of the first diastereoisomer (1-a): 3.07 min
Retention time of the second diastereoisomer (1-b): 3.95 min

The ¹H NMR and mass spectra are in accordance with the structures of the 2 expected products. Product 1 indeed corresponds to mixtures of 1-a and 1-b.

### Example 34: Demonstration of the activity with regard to constitutive melanogenesis

A biological test demonstrated the depigmenting activity of the compounds I. The modulating effect on melanogenesis of compound 1 was measured according to the method described in patent FR-A-2 734 825, and in the article by R. Schmidt, P. Krien and M. Régnier, Anal. Biochem., 235(2), 113-18, 1996. This test is carried out on a coculture of keratinocytes and melanocytes.

For the test compound, the following were determined:
- the cytotoxicity, by estimating the incorporation of leucine,
- the inhibitory activity on melanin synthesis, by estimating the ratio of the incorporation of thiouracil to the incorporation of leucine, relative to 100% for the control (the control corresponds to the test performed without test compound). The IC₅₀ (concentration for which 50% of the synthesis of the melanin is inhibited) values were determined.

The test was also carried out with arbutin and kojic acid, which are known depigmenting compounds.

The results are collated in the following table:

| **Compound** | **Cytotoxicity on coculture** | **IC₅₀** |
|---|---|---|
| Arbutin | Non-cytotoxic | Not reached (or greater than 500 µM) |
| Kojic acid | 100 µM | Not reached (or greater than 500 µM) |
| 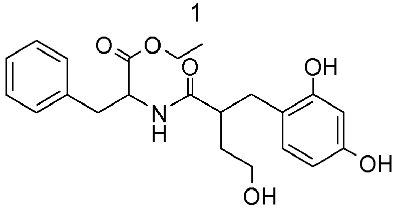 | Non-cytotoxic | 0.15 µM |
| 1-a | Non-cytotoxic | 6.9 µM |
| 1- b | Non-cytotoxic | 25.3 µM |
| 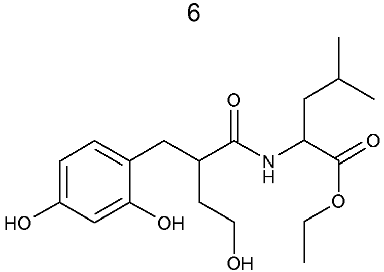 | Non-cytotoxic | 100 µM |
| 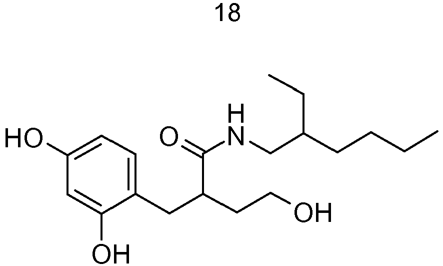 | 50 µM | 25.2 µM |

The compounds according to the invention are thus shown to be effective in inhibiting melanogenesis and are furthermore more effective than arbutin and kojic acid.

### TEST on pigmented reconstructed epidermis samples

Compositions comprising 300 µM of compound 1 in DMSO were applied to samples of pigmented reconstructed epidermis (cf. EP 1 878 790). The control is DMSO. The melanin was quantified by image analysis on histological slices after staining with Fontana Masson dye. Each sample of coloured epidermis is photographed over its entire length using a camera connected to a microscope. The melanin is thresholded and the number of melanin pixels is measured in each field using automated image analysis software. A non-parametric statistical test is performed in order to determine the significance of the measurements (Mann-Whitney test).

The pigmented reconstructed epidermis standard study model was published by:
Regnier M, Duval C, Galey JB, Philippe M, Lagrange A, Tuloup R, Schmidt R, Cellular and Molecular Biology, 1999, 45, 7, 969-980: "Keratinocyte-Melanocyte co-cultures and pigmented reconstructed human epidermis: models to study modulation of melanogenesis".

| Topical application | DMSO 1/1000 | Compound 1 300 uM |
|---|---|---|
| Number of images | 60 | 78 |
| Average area | 368.5 | 264.2 |
| 2SEM | 13.8 | 10.28 |
| Ttest | | 0.0006 |
| Percentage (normalized) | 100 | 71.7 |
| 2SEM (normalized) | 3.74 | 2.89 |

Significant depigmenting activity was evaluated at 300 µM for compound 1 (pValue < 0.05: significant depigmenting activity).

### Example 35: Use of a cosmetic formulation

A depigmenting gel for the skin is prepared, comprising (% by weight):

| | | |
|---|---|---|
| Compound 1 (Example 1) | | 2% |
| Carbomer (Carbopol 981 from Lubrizol) | | 1% |
| Preserving agent | qs | |
| Water | qs | 100% |

The composition, applied to the skin, makes it possible to attenuate brown blemishes.

Similar compositions are prepared with compounds 5, 6 and 18.

## Claims

1. Compounds of formula (I): 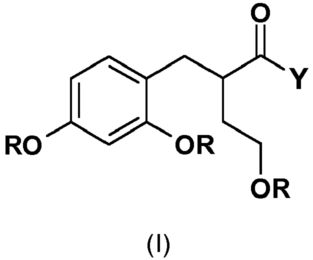 in which:
• **R** denotes a hydrogen atom;
• **Y** denotes a radical chosen from OR' and NAR";
**R'** denotes a radical chosen from:
a) -H;
b) a linear saturated C1-C20 or unsaturated C2-C20 or branched C3-C20 or cyclic C3-C8 alkyl group, optionally interrupted with one or more heteroatoms
or groups chosen from N, O, -CO- or a combination thereof such as -NHCO- or -NHCONH-, and/or optionally substituted with one or more identical or
different groups chosen from:
i) -OR5
ii) -SR5
iii) -NR6R7
iv) -CONHR6
v) -CONR6R7
vi) -COOR6
vii) -NHCONHR6
viii) -C(O)-(C1-C4)alkyl
ix) a C5-C12 (hetero)aryl group, optionally containing one or more heteroatoms chosen from O, N and S, optionally substituted with one or more hydroxyls and/or with one or more C1-C8 alkoxy radicals;
x) a saturated or unsaturated non-aromatic 5- to 8-membered heterocycle, comprising one or more heteroatoms chosen from O, N and S, optionally substituted with one or more hydroxyls and/or with one or more C1-C8 alkoxy or C1-C4 alkyl radicals; one of the ring members possibly being a carbonyl group;
c) a C5-C12 (hetero)aryl group, optionally containing one or more heteroatoms chosen from O, N and S, optionally substituted with one or more hydroxyls and/or with one or more radicals chosen from C1-C8 alkoxy
and C1-C8 alkyl groups;
**R5** being chosen from H, a saturated linear C1-C10 or branched C3-C10 or unsaturated C2-C10 or cyclic C3-C8 alkyl hydrocarbon-based group;
**R6** and **R7,** which may be identical or different, being chosen from H, a saturated linear C1-C10 or branched C3-C10 or unsaturated C2-C10 or cyclic C3-C8 alkyl hydrocarbon-based group; a (C1-C4)alkyl(hetero)(C6)aryl group optionally containing a nitrogen atom, especially a benzyl group;
**R6** and **R7** possibly forming, with the nitrogen atom that bears them, a 5-to 8-membered heterocycle which may contain one or more heteroatoms or groups chosen from
N, O and-CO- and/or optionally substituted with a C1-C10 hydrocarbon-based chain;
**A** denotes a radical chosen from:
a) -H;
b) a linear saturated C1-C20 or unsaturated C2-C20 or branched C3-C20 or cyclic C3-C8 alkyl group, optionally interrupted with one or more heteroatoms
or groups chosen from N, O, -CO- or a combination thereof such as -NHCO- or -NHCONH-, and/or optionally substituted with one or more identical or
different groups chosen from:
i) -OR15
ii) -SR15
iii) -NR16R17
iv) -CONHR16
v) -CONR16R17
vi) -COOR16
vii) -NHCONHR16
viii) -C(O)-(C1-C4)alkyl
ix) a C5-C12 (hetero)aryl group, optionally containing one or more heteroatoms chosen from O, N and S, optionally substituted with one or more hydroxyls and/or with one or more C1-C8 alkoxy radicals;
x) a saturated or unsaturated non-aromatic 5- to 8-membered heterocycle, comprising one or more heteroatoms chosen from O, N and S, optionally substituted with one or more hydroxyls and/or with one or more C1-C8 alkoxy or C1-C4 alkyl radicals; one of the ring members possibly being a carbonyl group;
c) a C5-C12 (hetero)aryl group, optionally containing one or more heteroatoms chosen from O, N and S, optionally substituted with one or more hydroxyls and/or with one or more radicals chosen from C1-C8 alkoxy
and C1-C8 alkyl groups;
xi)
-NH-C=NH(NH₂) (guanidine group)
d) -NR12R13;
e) -OR14;
f) -C(O)NHR14;
g) C(O)-(C1-C10)alkyl
**R12** and **R13,** which may be identical or different, denoting a radical chosen from:
a) -H;
b) a saturated linear C1-C10 or unsaturated C2-C10 or branched C3-C10 or cyclic C3-C8 alkyl group, optionally interrupted with one or more heteroatoms or groups chosen from N, O, -CO- or a combination thereof such as -NHCO- or -NHCONH-, and/or optionally substituted with one or more groups, which may be identical or different, chosen from -OR5;
c) a C5-C12 (hetero)aryl group, optionally containing one or more heteroatoms chosen from O, N and S, optionally substituted with one or more hydroxyls and/or with one or more C1-C8 alkoxy radicals; R2 and R3 possibly forming, with the nitrogen that bears them, a 5- to 8-membered heterocycle, which may contain one or more heteroatoms or groups chosen from N, O and -CO- and/or optionally substituted with a C1-C10 hydrocarbon-based chain optionally containing one or more radicals chosen from hydroxyl and C1-C4 alkoxy;
**R14** denoting a radical chosen from:
a) -H;
b) a saturated linear C1-C10 or branched C3-C10 or cyclic C3-C8 alkyl group, optionally substituted with one or more groups, which may be identical or different, chosen from:
i) -COOR16;
ii) a C5-C12 (hetero)aryl radical, optionally containing one or more heteroatoms chosen from O, N and S, optionally substituted with one or more hydroxyls and/or with one or more C1-C8 alkoxy radicals;
c) a C5-C12 (hetero)aryl group, optionally containing one or more heteroatoms chosen from O, N and S, optionally substituted with one or more hydroxyls and/or with one or more C1-C8 alkoxy radicals;
**R15** being chosen from H, a saturated linear C1-C10 or branched C3-C10 or unsaturated C2-C10 or cyclic C3-C8 alkyl hydrocarbon-based group; R16 and R17, which may be identical or different, being chosen from H and a saturated linear C1-C10 or branched C3-C10 or unsaturated C2-C10 or cyclic C3-C8 alkyl hydrocarbon-based group; a (C1-C4)alkyl(hetero)(C6)aryl group optionally containing a nitrogen atom, especially a benzyl group;
**R16** and **R17,** which may be identical or different, being chosen from H and a saturated linear C1-C10 or branched C3-C10 or unsaturated C2-C10 or cyclic C3-C8 alkyl hydrocarbon-based group; a (C1-C4)alkyl(hetero)(C6)aryl group optionally containing a nitrogen atom, especially a benzyl group; an acetyl radical;
**R16** and **R17** possibly forming, with the nitrogen that bears them, a 5- to 8-membered heterocycle, which may contain one or more heteroatoms or groups chosen from N, O and -CO- and/or optionally substituted with a C1-C10 hydrocarbon-based chain;
**R"** denotes a radical chosen from:
a) -H;
b) a linear saturated C1-C20 or unsaturated C2-C20 or branched C3-C20 or cyclic C3-C8 alkyl group, optionally interrupted with one or more heteroatoms or groups chosen from N, O, -CO- or a combination thereof such as -NHCO- or -NHCONH-, and/or optionally substituted with one or more identical or different groups chosen from:
i) -OR25
ii) -SR25
iii) -NR26R27
iv) -CONHR26
v) -CONR26R27
vi) -COOR26
vii) -NHCONHR26
viii) -C(O)-(C1-C4)alkyl
ix) a C5-C12 (hetero)aryl group, optionally containing one or more heteroatoms chosen from O, N and S, optionally substituted with one or more hydroxyls and/or with one or more C1-C8 alkoxy radicals;
x) a saturated or unsaturated non-aromatic 5- to 8-membered heterocycle, comprising one or more heteroatoms chosen from O, N and S, optionally substituted with one or more hydroxyls and/or with one or more C1-C8 alkoxy or C1-C4 alkyl radicals; one of the ring members possibly being a carbonyl group;
c) a C5-C12 (hetero)aryl group, optionally containing one or more heteroatoms chosen from O, N and S, optionally substituted with one or more hydroxyls and/or with one or more radicals chosen from C1-C8 alkoxy and C1-C8 alkyl groups;
**R25** being chosen from H, a saturated linear C1-C10 or branched C3-C10 or unsaturated C2-C10 or cyclic C3-C8 alkyl hydrocarbon-based group;
**R26** and **R27,** which may be identical or different, being chosen from H, a saturated linear C1-C10 or branched C3-C10 or unsaturated C2-C10 or cyclic C3-C8 alkyl hydrocarbon-based group; a (C1-C4)alkyl(hetero)(C6)aryl group optionally containing a nitrogen atom, especially a benzyl group; **R26** and **R27** possibly forming, with the nitrogen that bears them, a 5- to 8-membered heterocycle, which may contain one or more heteroatoms or groups chosen from N, O and -CO- and/or optionally substituted with a C1-C10 hydrocarbon-based chain;
it being understood that **A** and **R"** may form, with the nitrogen atom that bears them, a saturated or unsaturated 5- to 8-membered heterocycle, which may contain one or more heteroatoms or groups chosen from N, O and -CO- and/or optionally substituted with a C1-C10 hydrocarbon-based chain or C1-C10 hydroxyalkyl or CO2T, T denoting a hydrogen atom or a saturated linear C1-C10 or branched C3-C10 alkyl radical,
and the salts thereof, the solvates thereof, the optical isomers thereof and the racemic mixtures thereof, alone or as a mixture.

2. Compounds according to the preceding claim, in which:
**R** denotes a hydrogen atom;
**Y** denotes a radical chosen from OR' and NAR";
**R'** denotes a radical chosen from:
a) -H;
b) a linear saturated C1-C10 or unsaturated C2-C10 or branched C3-C10 or cyclic C3-C8 alkyl group, optionally interrupted with one or more heteroatoms or groups chosen from N, O, -CO- or a combination thereof such as -NHCO- or -NHCONH-, and/or optionally substituted with one or more groups -OR5; **R5** being chosen from H and a saturated linear C1-C4 alkyl hydrocarbon-based group;
c) a C5-C12 (hetero)aryl group, optionally containing one or more heteroatoms chosen from O, N and S, optionally substituted with one or more hydroxyls and/or with one or more radicals chosen from C1-C8 alkoxy and C1-C8 alkyl groups;
**A** denotes a radical chosen from:
a) -H;
b) a linear saturated C1-C20 or unsaturated C2-C20 or branched C3-C20 or cyclic C3-C8 alkyl group, optionally interrupted with one or more heteroatoms or groups chosen from N, O, -CO- or a combination thereof such as -NHCO- or -NHCONH-, and/or optionally substituted with one or more identical or
different groups chosen from:
i) -OR15
ii) -SR15
iii) -NR16R17
iv) -CONHR16
vi) -COOR16
ix) a C5-C12 (hetero)aryl group, optionally containing one or more heteroatoms chosen from O, N and S, optionally substituted with one or more hydroxyls and/or with one or more C1-C8 alkoxy radicals;
x) a saturated or unsaturated non-aromatic 5- to 8-membered heterocycle, comprising one or more heteroatoms chosen from O, N and S, optionally substituted with one or more hydroxyls and/or with one or more C1-C8 alkoxy or C1-C4 alkyl radicals; one of the ring members possibly being a carbonyl group;
c) a C5-C12 (hetero)aryl group, optionally containing one or more heteroatoms chosen from O, N and S, optionally substituted with one or more hydroxyls and/or with one or more radicals chosen from C1-C8 alkoxy and C1-C8 alkyl groups;
d) -NR12R13;
e) -OR14;
**R12** and **R13,** which may be identical or different, denoting a radical chosen from:
a) -H;
b) a saturated linear C1-C4 alkyl group;
c) a phenyl group optionally substituted with one or more hydroxyls and/or with one or more C1-C8 alkoxy radicals;
**R12** and **R13** possibly forming, with the nitrogen that bears them, a 5- to 8-membered heterocycle, which may contain one or more heteroatoms or groups chosen from N, O and -CO- and/or optionally substituted with a C1-C10 hydrocarbon-based chain optionally containing one or more radicals chosen from hydroxyl and C1-C4 alkoxy;
**R14** denoting a radical chosen from:
a) -H;
b) a saturated linear C1-C10 alkyl group optionally substituted with one or more identical or different phenyl radicals, the said phenyl radicals being
optionally substituted with one or more hydroxyls and/or with one or more C1-C8 alkoxy radicals;
**R15** being chosen from H, a saturated linear C1-C10 or branched C3-C10 or unsaturated C2-C10 or cyclic C3-C8 alkyl hydrocarbon-based group;
**R16** and **R17,** which may be identical or different, being chosen from H and a saturated linear C1-C10 or branched C3-C10 or unsaturated C2-C10 or cyclic C3-C8 alkyl hydrocarbon-based group; a (C1-C4)alkyl(hetero)(C6)aryl group optionally containing a nitrogen atom, especially a benzyl group; an acetyl radical;
**R16** and **R17** possibly forming, with the nitrogen that bears them, a 5- to 8-membered heterocycle, which may contain one or more heteroatoms or groups chosen from N, O and -CO- and/or optionally substituted with a C1-C10 hydrocarbon-based chain;
**R"** denotes a radical chosen from:
a) -H;
b) a linear saturated C1-C10 or branched C3-C10 alkyl group, optionally substituted with one or more identical or different groups -OR25;
**R25** being chosen from H and a saturated linear C1-C10 alkyl hydrocarbon-based group.

3. Compounds according to either of the preceding claims, in which:
• **R** denotes a hydrogen atom;
• **Y** denotes a radical chosen from OR' and NAR";
**R'** denotes a radical chosen from:
a) -H;
b) a linear saturated C1-C10 or unsaturated C2-C10 or branched C3-C10 or cyclic C3-C8 alkyl group, optionally interrupted with one or more oxygen atoms, and/or optionally substituted with one or more hydroxyl, methoxy or ethoxy groups;
**A** denotes a radical chosen from:
a) -H;
b) a linear saturated C1-C10 or unsaturated C2-C10 or branched C3-C10 or cyclic C3-C8 alkyl group, optionally interrupted with one or more oxygen atoms, and/or optionally substituted with one or more identical or different groups chosen from:
i) -OR15
ii) -NR16R17
vi) -COOR16
ix) a C5-C12 (hetero)aryl group, optionally containing one or more heteroatoms chosen from O, N and S, optionally substituted with one or more hydroxyls and/or with one or more C1-C8 alkoxy radicals;
x) a saturated or unsaturated non-aromatic 5- to 8-membered heterocycle, comprising one or more heteroatoms chosen from O, N and S, optionally substituted with one or more hydroxyls and/or with one or more C1-C8 alkoxy or C1-C4 alkyl radicals;
c) a phenyl group optionally substituted with one or more radicals chosen from hydroxyls and/or methoxy and/or ethoxy;
d) -NR12R13;
e) -OR14;
**R12** and **R13,** which may be identical or different, denoting a radical chosen from:
a) -H;
b) a saturated linear C1-C4 alkyl group;
c) a phenyl group optionally substituted with one or more hydroxyls and/or with one or more C1-C8 alkoxy radicals;
**R14** denoting a radical chosen from:
a) -H;
b) a saturated linear C1-C4 alkyl group optionally substituted with a phenyl radical;
**R15** being chosen from H and a saturated linear C1-C4 or branched C3-C4 alkyl hydrocarbon-based group;
**R16** and **R17,** which may be identical or different, being chosen from H and a saturated linear C1-C10 or branched C3-C10 or unsaturated C2-C10 or cyclic C3-C8 alkyl hydrocarbon-based group; a (C1-C4)alkyl(hetero)(C6)aryl group optionally containing a nitrogen atom, especially a benzyl group; an acetyl radical;
**R16** and **R17** possibly forming, with the nitrogen that bears them, a 5- to 8-membered heterocycle, which may contain one or more heteroatoms or groups chosen from N, O and -CO- and/or optionally substituted with a C1-C10 hydrocarbon-based chain;
**R"** denotes a radical chosen from:
a) -H;
b) a linear saturated C1-C10 or branched C3-C10 alkyl group, optionally substituted with one or more identical or different groups -OR25; **R25** being chosen from H and a saturated linear C1-C4 alkyl hydrocarbon-based group.

4. Compounds according to one of the preceding claims, chosen from:
| No. | Structure |
|---|---|
| 1 | 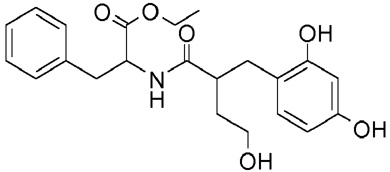 |
| 2 | 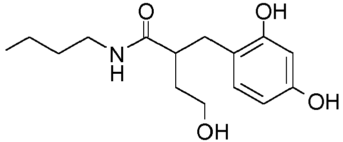 |
| 3 | 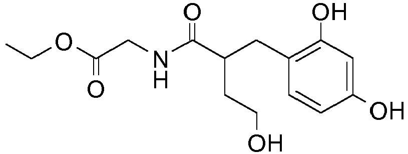 |
| 4 | 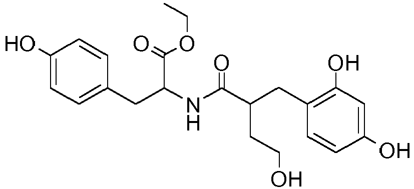 |
| 5 | 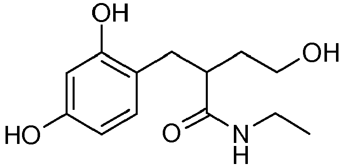 |
| 6 | 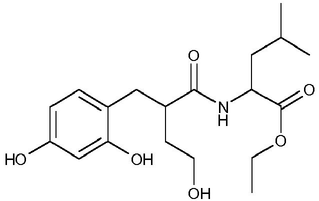 |
| 7 | 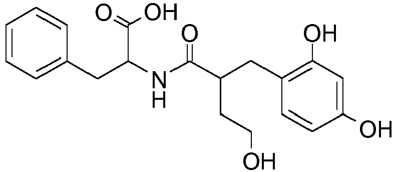 |
| 8 | 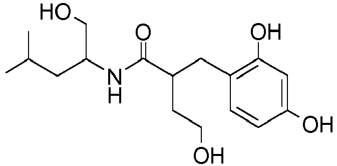 |
| 9 | 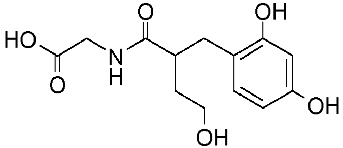 |
| 10 | 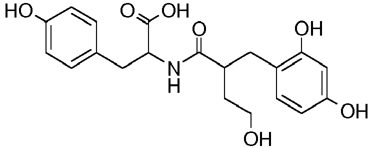 |
| 11 | 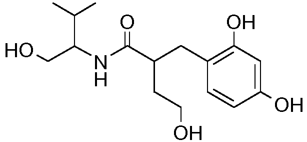 |
| 12 | 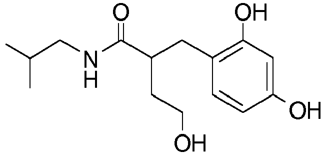 |
| 13 | 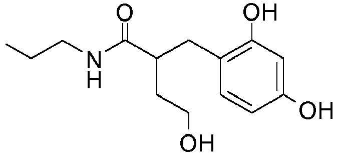 |
| 14 | 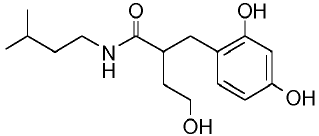 |
| 15 | 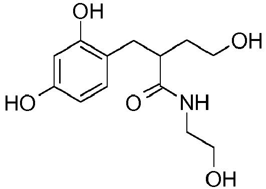 |
| 16 | 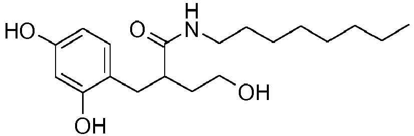 |
| 17 | 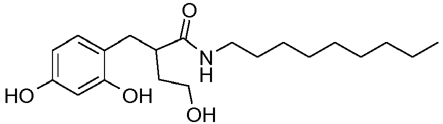 |
| 18 | 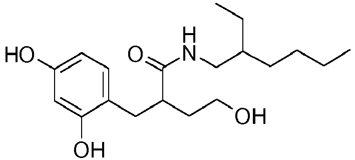 |
| 19 | 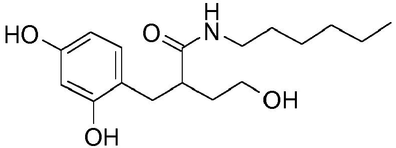 |
| 20 | 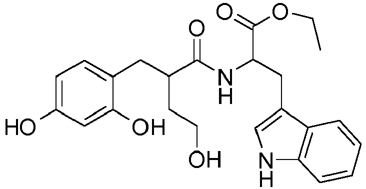 |
| 21 | 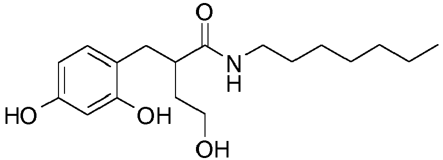 |
| 22 | 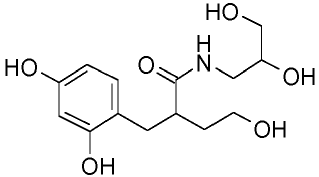 |
| 23 | 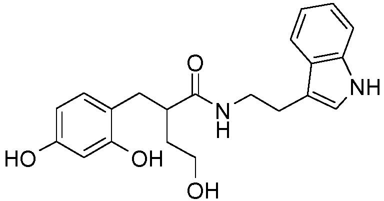 |
| 24 | 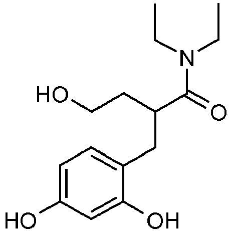 |
| 25 | 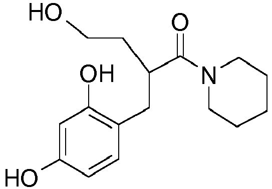 |
| 26 | 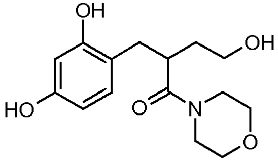 |
| 27 | 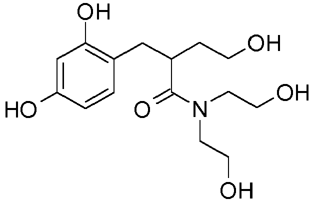 |
| 28 | 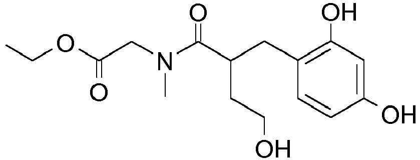 |
| 29 | 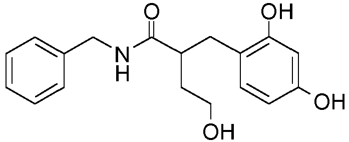 |
| 30 | 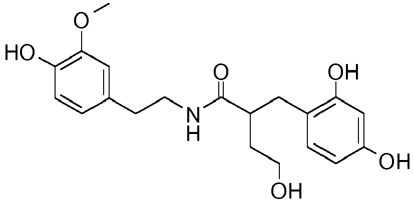 |
| 31 | 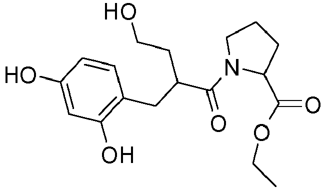 |
| 32 | 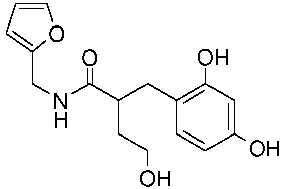 |
| 33 | 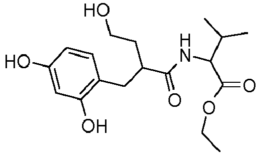 |
| 34 | 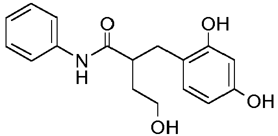 |
| 35 | 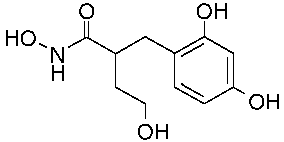 |
| 36 | 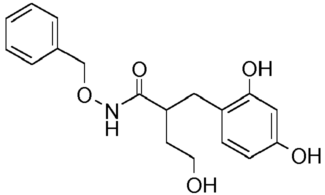 |
| 37 | 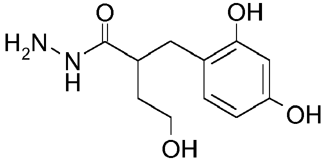 |
| 38 | 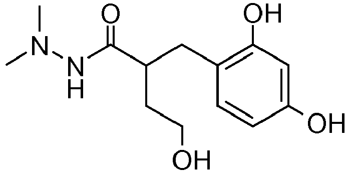 |
| 39 | 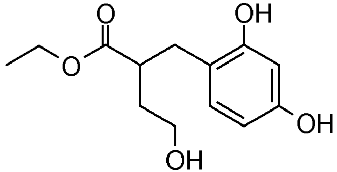 |
| 40 | 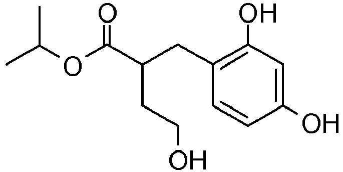 |
| 41 | 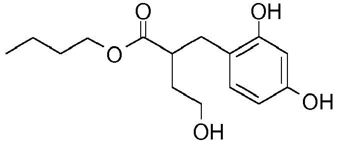 |
| 42 | 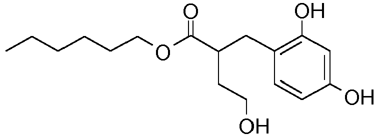 |
| 43 | 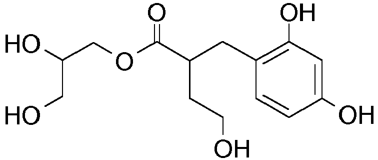 |
| 45 | 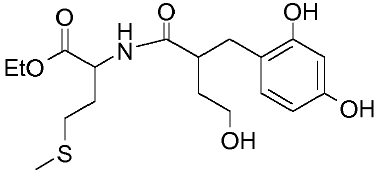 |
| 46 | 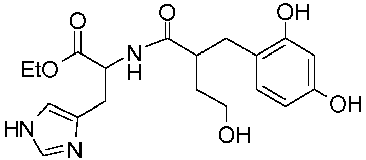 |
| 47 | 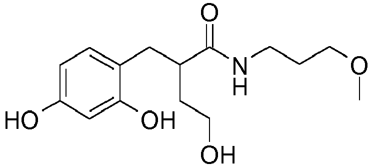 |
| 48 | 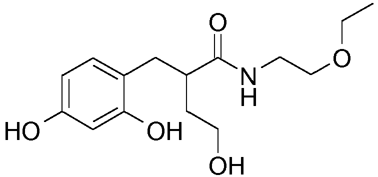 |
| 49 | 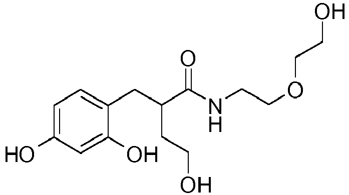 |
| 50 | 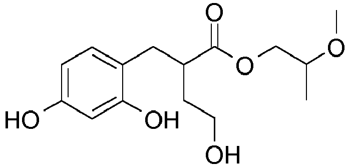 |
| 51 | 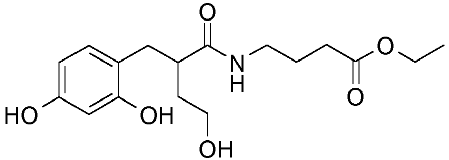 |
| 52 | 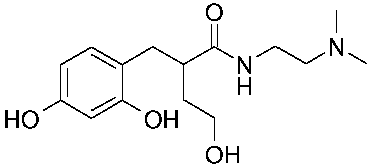 |
| 53 | 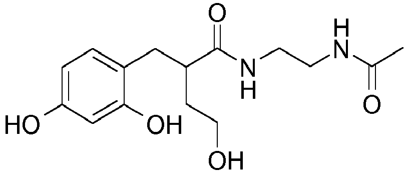 |
| 54 | 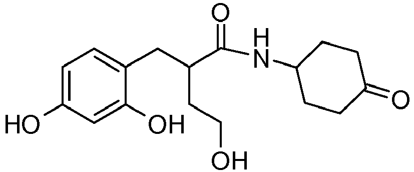 |
| 55 | 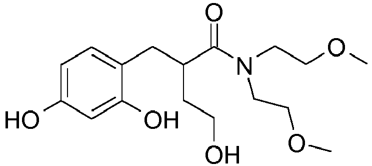 |
| 56 | 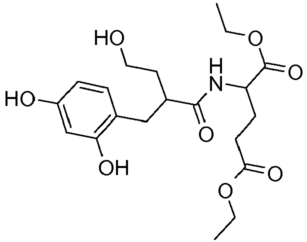 |
| 57 | 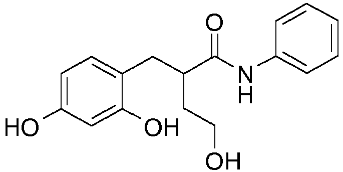 |
| 58 | 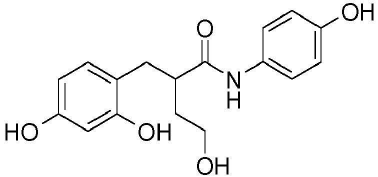 |
| 59 | 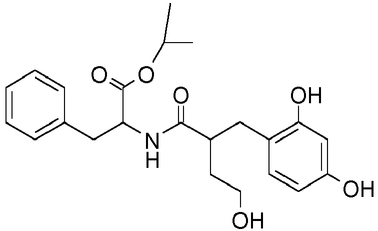 |
and the salts thereof, the solvates thereof, the optical isomers thereof and the racemic mixtures thereof, alone or as a mixture.

5. Composition comprising, in a physiologically acceptable medium, a compound of formula (I) according to any one of the preceding claims.

6. Composition according to the preceding claim, **characterized in that** compound (I) is present in a content of between 0.01% and 10% by weight relative to the total weight of the composition.

7. Composition according to the preceding claims 5 or 6, **characterized in that** compound (I) is present in a content of between 0.1% and 5% by weight relative to the total weight of the composition.

8. Composition according to either of Claims 5 to 7, **characterized in that** it comprises at least one adjuvant chosen from the group formed by: water, organic solvents, hydrocarbon-based oils, silicone oils, waxes, pigments, fillers, dyes, surfactants, emulsifiers; cosmetic active agents, UV screening agents, polymers, thickeners, preserving agents, fragrances, bactericides, ceramides, odour absorbers and antioxidants.

9. Composition according to any one of Claims 5 to 8, **characterized in that** it comprises at least one active agent chosen from: desquamating agents; calmatives; organic or mineral photoprotective agents; moisturizers; depigmenting agents; antiglycation agents; NO-synthase inhibitors; agents which stimulate the synthesis of dermal or epidermal macromolecules and/or which prevent their decomposition; agents which stimulate the proliferation of fibroblasts and/or keratinocytes or which stimulate the differentiation of keratinocytes; muscle relaxants and/or dermo-decontracting agents; tensioning agents; antipollution agents and/or free-radical scavengers; agents which act on the capillary circulation; agents which act on the energy metabolism of cells; and mixtures thereof.

10. Non-therapeutic cosmetic process for depigmenting, lightening and/or bleaching keratin materials, comprising the application of a composition according to any one of Claims 5 to 9.

11. Process according to the preceding claim, for depigmenting, lightening and/or bleaching the skin.

12. Non-therapeutic cosmetic use of a compound of formula (I) as defined according to any one of Claims 1 to 4, as an agent for bleaching, lightening and/or depigmenting keratin materials.

13. Process for preparing the compounds of formula (I) according to one of Claims 1 to 4, comprising the following stages: 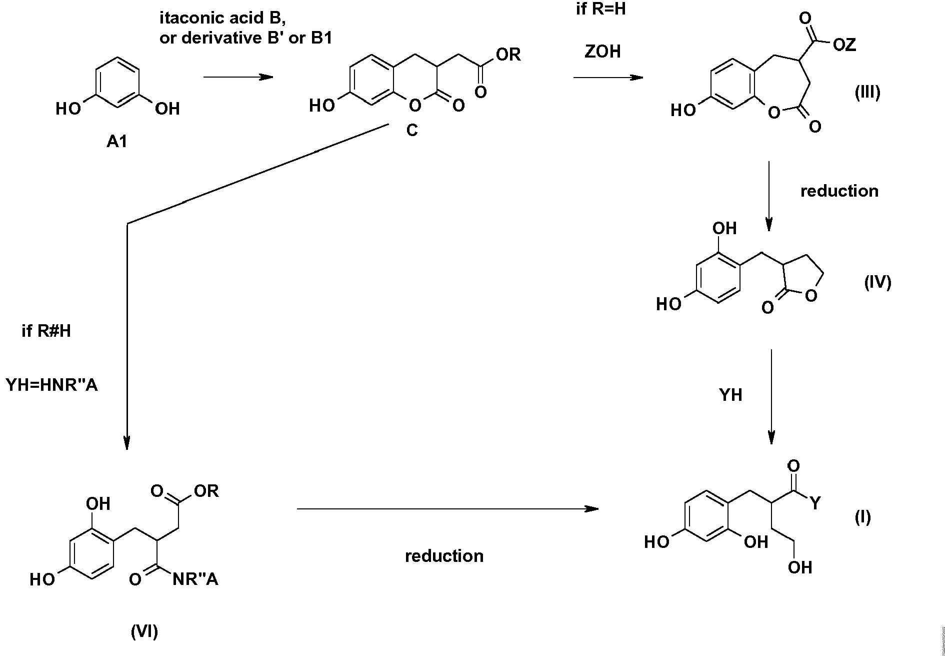 comprising the following steps:
a) Reaction of resorcinol (A1) with itaconic acid (B) or with its anhydride (B') or with one of its esters of formula (B1) 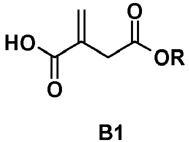 in which R denotes H or a linear C1-C6 or branched C3-C6 alkyl group, to form a compound C, and this compound is then activated to form a heterocycle of formula (III), which is then subjected to a reduction step to form the intermediate (IV) which gives access to the compounds (I),
Z denotes H or a linear (C₁-C₆)alkyl group or a branched (C₃-C₆)alkyl group,
**Y** denotes a radical chosen from OR' and NAR".

14. Intermediate compounds of formulae (III) and (IV) 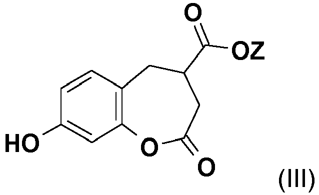 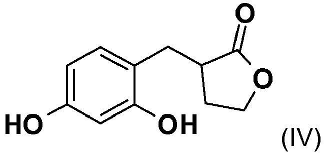 in which Z denotes H or a linear (C₁-C₆)alkyl group or a branched (C₃-C₆)alkyl group.

## Patentansprüche

1. Verbindungen der Formel (I): 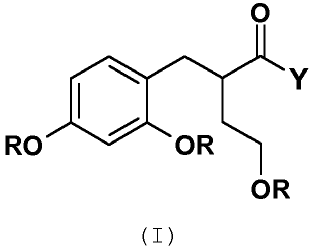 in der:
• **R** für ein Wasserstoffatom steht;
• **Y** für einen Rest, der aus OR' und NAR" ausgewählt ist, steht;
**R'** für einen Rest, der aus:
a) -H;
b) einer linearen gesättigten C1-C20- oder ungesättigten C2-C20- oder verzweigten C3-C20- oder cyclischen C3-C8-Alkylgruppe, die gegebenenfalls durch ein oder mehrere Heteroatome oder eine oder mehrere Gruppen, die aus N, O, -CO- oder einer Kombination davon wie -NHCO- oder - NHCONH- ausgewählt sind, unterbrochen ist und/oder gegebenenfalls durch eine oder mehrere gleiche oder verschiedene Gruppen, die aus:
i) -OR5
ii) -SR5
iii) -NR6R7
iv) -CONHR6
v) -CONR6R7
vi) -COOR6
vii) -NHCONHR6
viii) -C(O)-(C1-C4)Alkyl
ix) einer C5-C12-(Hetero)arylgruppe, die gegebenenfalls ein oder mehrere Heteroatome, die aus O, N und S ausgewählt sind, enthält und gegebenenfalls durch eine oder mehrere Hydroxylgruppen und/oder durch einen oder mehrere C1-C8-Alkoxyreste substituiert ist;
x) einem gesättigten oder ungesättigten nichtaromatischen 5- bis 8-gliedrigen Heterocyclus, der ein oder mehrere Heteroatome, die aus O, N und S ausgewählt sind, umfasst und gegebenenfalls durch eine oder mehrere Hydroxylgruppen und/oder durch einen oder mehrere C1-C8-Alkoxy- oder C1-C4-Alkylreste substituiert ist; wobei eines der Ringglieder gegebenenfalls eine Carbonylgruppe ist;
ausgewählt sind, substituiert ist;
c) einer C5-C12-(Hetero)arylgruppe, die gegebenenfalls ein oder mehrere Heteroatome, die aus O, N und S ausgewählt sind, enthält und gegebenenfalls durch eine oder mehrere Hydroxylgruppen und/oder durch einen oder mehrere Reste, die aus C1-C8-Alkoxy- und C1-C8-Alkylgruppen ausgewählt sind, substituiert ist; ausgewählt ist, steht;
wobei **R5** aus H, einer gesättigten linearen C1-C10- oder verzweigten C3-C10- oder ungesättigten C2-C10- oder cyclischen C3-C8-Alkylgruppe auf Kohlenwasserstoffbasis ausgewählt ist;
wobei **R6** und **R7,** die gleich oder verschieden sein können, aus H, einer gesättigten linearen C1-C10- oder verzweigten C3-C10- oder ungesättigten C2-C10- oder cyclischen C3-C8-Alkylgruppe auf Kohlenwasserstoffbasis; einer (C1-C4)Alkyl(hetero)(C6)arylgruppe, die gegebenenfalls ein Stickstoffatom enthält, insbesondere einer Benzylgruppe, ausgewählt sind;
wobei **R6** und **R7** gegebenenfalls mit dem Stickstoffatom, das sie trägt, einen 5- bis 8-gliedrigen Heterocyclus bilden, der ein oder mehrere Heteroatome oder eine oder mehrere Gruppen, die aus N, O und -CO- ausgewählt sind, enthalten kann und/oder gegebenenfalls durch eine C1-C10-Kette auf Kohlenwasserstoffbasis substituiert sein kann;
**A** für einen Rest, der aus:
a) -H;
b) einer linearen gesättigten C1-C20- oder ungesättigten C2-C20- oder verzweigten C3-C20- oder cyclischen C3-C8-Alkylgruppe, die gegebenenfalls durch ein oder mehrere Heteroatome oder eine oder mehrere Gruppen, die aus N, O, -CO- oder einer Kombination davon wie -NHCO- oder - NHCONH- ausgewählt sind, unterbrochen ist und/oder gegebenenfalls durch eine oder mehrere gleiche oder verschiedene Gruppen, die aus:
i) -OR15
ii) -SR15
iii) -NR16R17
iv) -CONHR16
v) -CONR16R17
vi) -COOR16
vii) -NHCONHR16
viii) -C(O)-(C1-C4)Alkyl
ix) einer C5-C12-(Hetero)arylgruppe, die gegebenenfalls ein oder mehrere Heteroatome, die aus O, N und S ausgewählt sind, enthält und gegebenenfalls durch eine oder mehrere Hydroxylgruppen und/oder durch einen oder mehrere C1-C8-Alkoxyreste substituiert ist;
x) einem gesättigten oder ungesättigten nichtaromatischen 5- bis 8-gliedrigen Heterocyclus, der ein oder mehrere Heteroatome, die aus O, N und S ausgewählt sind, umfasst und gegebenenfalls durch eine oder mehrere Hydroxylgruppen und/oder durch einen oder mehrere C1-C8-Alkoxy- oder C1-C4-Alkylreste substituiert ist; wobei eines der Ringglieder gegebenenfalls eine Carbonylgruppe ist;
ausgewählt sind, substituiert ist;
c) einer C5-C12 (Hetero)arylgruppe, die gegebenenfalls ein oder mehrere Heteroatome, die aus O, N und S ausgewählt sind, enthält und gegebenenfalls durch eine oder mehrere Hydroxylgruppen und/oder durch einen oder mehrere aus C1-C8-Alkoxy-und C1-C8-Alkylgruppen ausgewählte Reste substituiert ist;
xi)
-NH-C=NH(NH₂) (Guanidingruppe)
d) -NR12R13;
e) -OR14;
f) -C(O)NHR14;
g) C(O)-(C1-C10)Alkyl
ausgewählt ist, steht;
wobei **R12** und **R13,** die gleich oder verschieden sein können, für einen Rest, der aus:
a) -H;
b) einer gesättigten linearen C1-C10- oder ungesättigten C2-C10- oder verzweigten C3-C10- oder cyclischen C3-C8-Alkylgruppe, die gegebenenfalls durch ein oder mehrere Heteroatome oder eine oder mehrere Gruppen, die aus N, O, -CO- oder einer Kombination davon wie -NHCO- oder - NHCONH- ausgewählt sind, unterbrochen ist und/oder gegebenenfalls durch eine oder mehrere Gruppen, die gleich oder verschieden sein können und aus - OR5 ausgewählt ist, substituiert ist;
c) einer C5-C12 (Hetero)arylgruppe, die gegebenenfalls ein oder mehrere Heteroatome, die aus O, N und S ausgewählt sind, enthält und gegebenenfalls durch eine oder mehrere Hydroxylgruppen und/oder durch einen oder mehrere C1-C8-Alkoxyreste substituiert ist; wobei R2 und R3 gegebenenfalls mit dem Stickstoff, der sie trägt, einen 5- bis 8-gliedrigen Heterocyclus bilden, der ein oder mehrere Heteroatome oder eine oder mehrere Gruppen, die aus N, O und -COausgewählt sind, enthalten kann und/oder gegebenenfalls durch eine C1-C10-Kette auf Kohlenwasserstoffbasis, die gegebenenfalls einen oder mehrere Reste, die aus Hydroxyl und C1-C4-Alkoxy ausgewählt sind, enthält, substituiert ist; ausgewählt ist, stehen;
wobei **R14** für einen Rest, der aus:
a) -H;
b) einer gesättigten linearen C1-C10- oder verzweigten C3-C10- oder cyclischen C3-C8-Alkylgruppe, die gegebenenfalls durch eine oder mehrere Gruppen, die gleich oder verschieden sein können und aus:
i) -COOR16;
ii) einem C5-C12-(Hetero)arylrest, der gegebenenfalls ein oder mehrere Heteroatome, die aus O, N und S ausgewählt sind, enthält und gegebenenfalls durch eine oder mehrere Hydroxylgruppen und/oder durch einen oder mehrere C1-C8-Alkoxyreste substituiert ist;
ausgewählt sind, substituiert ist;
c) einer C5-C12 (Hetero)arylgruppe, die gegebenenfalls ein oder mehrere Heteroatome, die aus O, N und S ausgewählt sind, enthält und gegebenenfalls durch eine oder mehrere Hydroxylgruppen und/oder durch einen oder mehrere C1-C8-Alkoxyreste substituiert ist;
ausgewählt ist, steht;
wobei **R15** aus H, einer gesättigten linearen C1-C10- oder verzweigten C3-C10- oder ungesättigten C2-C10- oder cyclischen C3-C8-Alkylgruppe auf Kohlenwasserstoffbasis ausgewählt ist; wobei R16 und R17, die gleich oder verschieden sein können, aus H und einer gesättigten linearen C1-C10- oder verzweigten C3-C10- oder ungesättigten C2-C10- oder cyclischen C3-C8-Alkylgruppe auf Kohlenwasserstoffbasis; einer (C1-C4)Alkyl(hetero)(C6)arylgruppe, die gegebenenfalls ein Stickstoffatom enthält, insbesondere einer Benzylgruppe, ausgewählt sind;
wobei **R16** und **R17,** die gleich oder verschieden sein können, aus H und einer gesättigten linearen C1-C10- oder verzweigten C3-C10- oder ungesättigten C2-C10- oder cyclischen C3-C8-Alkylgruppe auf Kohlenwasserstoffbasis; einer (C1-C4)Alkyl(hetero)(C6)arylgruppe, die gegebenenfalls ein Stickstoffatom enthält, insbesondere einer Benzylgruppe; einem Acetylrest ausgewählt sind; wobei **R16** und **R17** gegebenenfalls mit dem Stickstoff, der sie trägt, einen 5- bis 8-gliedrigen Heterocyclus bilden, der ein oder mehrere Heteroatome oder eine oder mehrere Gruppen, die aus N, O und -CO- ausgewählt sind,
enthalten kann und/oder gegebenenfalls durch eine C1-C10-Kette auf Kohlenwasserstoffbasis substituiert sein kann;
R" für einen Rest, der aus:
a) -H;
b) einer linearen gesättigten C1-C20- oder ungesättigten C2-C20- oder verzweigten C3-C20- oder cyclischen C3-C8-Alkylgruppe, die gegebenenfalls durch ein oder mehrere Heteroatome oder eine oder mehrere Gruppen, die aus N, O, -CO- oder einer Kombination davon wie -NHCO- oder - NHCONH- ausgewählt sind, unterbrochen ist und/oder gegebenenfalls durch eine oder mehrere gleiche oder verschiedene Gruppen, die aus:
i) -OR25
ii) -SR25
iii) -NR26R27
iv) -CONHR26
v) -CONR26R27
vi) -COOR26
vii) -NHCONHR26
viii) -C(O)-(C1-C4)Alkyl
ix) einer C5-C12-(Hetero)arylgruppe, die gegebenenfalls ein oder mehrere Heteroatome, die aus O, N und S ausgewählt sind, enthält und gegebenenfalls durch eine oder mehrere Hydroxylgruppen und/oder durch einen oder mehrere C1-C8-Alkoxyreste substituiert ist;
x) einem gesättigten oder ungesättigten nichtaromatischen 5- bis 8-gliedrigen Heterocyclus, der ein oder mehrere Heteroatome, die aus O, N und S ausgewählt sind, umfasst und gegebenenfalls durch eine oder mehrere Hydroxylgruppen und/oder durch einen oder mehrere C1-C8-Alkoxy- oder C1-C4-Alkylreste substituiert ist; wobei eines der Ringglieder gegebenenfalls eine Carbonylgruppe ist;
ausgewählt sind, substituiert ist;
c) einer C5-C12-(Hetero)arylgruppe, die gegebenenfalls ein oder mehrere Heteroatome, die aus O, N und S ausgewählt sind, enthält und gegebenenfalls durch eine oder mehrere Hydroxylgruppen und/oder durch einen oder mehrere aus C1-C8-Alkoxy- und C1C8-Alkylgruppen ausgewählte Reste substituiert ist;
ausgewählt ist, steht;
wobei **R25** aus H, einer gesättigten linearen C1-C10- oder verzweigten C3-C10- oder ungesättigten C2-C10- oder cyclischen C3-C8-Alkylgruppe auf Kohlenwasserstoffbasis ausgewählt ist;
wobei **R26** und **R27,** die gleich oder verschieden sein können, aus H, einer gesättigten linearen C1-C10- oder verzweigten C3-C10- oder ungesättigten C2-C10- oder cyclischen C3-C8-Alkylgruppe auf Kohlenwasserstoffbasis; einer (C1-C4)Alkyl(hetero)(C6)arylgruppe, die gegebenenfalls ein Stickstoffatom enthält, insbesondere einer Benzylgruppe, ausgewählt sind; wobei **R26** und **R27** gegebenenfalls mit dem Stickstoff, der sie trägt, einen 5- bis 8-gliedrigen Heterocyclus bilden, der ein oder mehrere Heteroatome oder ein oder mehrere Heteroatome oder eine oder mehrere Gruppen, die aus N, O und -CO- ausgewählt sind, enthalten kann und/oder gegebenenfalls durch eine C1-C10-Kette auf Kohlenwasserstoffbasis substituiert sein kann;
mit der Maßgabe, dass **A** und **R"** mit dem Stickstoffatom, das sie trägt, einen gesättigten oder ungesättigten 5-bis 8-gliedrigen Heterocyclus bilden können, der ein oder mehrere Heteroatome oder ein oder mehrere Gruppen, die aus N, O und -CO- ausgewählt sind, enthalten kann und/oder gegebenenfalls durch eine C1-C10-Kette auf Kohlenwasserstoffbasis oder C1-C10-Hydroxyalkyl oder CO2T substituiert sein kann, wobei T für ein Wasserstoffatom oder einen gesättigten linearen C1-C10- oder verzweigten C3-C10-Alkylrest steht,
und die Salze davon, die Solvate davon, die optischen Isomere davon und die racemischen Gemische davon alleine oder als Gemisch.

2. Verbindungen nach dem vorhergehenden Anspruch, in denen:
**R** für ein Wasserstoffatom steht;
**Y** für einen Rest, der aus OR' und NAR" ausgewählt ist, steht;
**R'** für einen Rest, der aus:
a) -H;
b) einer linearen gesättigten C1-C10- oder ungesättigten C2-C10- oder verzweigten C3-C10- oder cyclischen C3-C8-Alkylgruppe, die gegebenenfalls durch ein oder mehrere Heteroatome oder eine oder mehrere Gruppen, die aus N, O, -CO- oder einer Kombination davon wie -NHCO- oder - NHCONH- ausgewählt sind, unterbrochen ist und/oder gegebenenfalls durch eine oder mehrere Gruppen - OR5 substituiert ist; wobei **R5** aus H und einer gesättigten linearen C1-C4-Alkylgruppe auf Kohlenwasserstoffbasis ausgewählt ist;
c) einer C5-C12-(Hetero)arylgruppe, die gegebenenfalls ein oder mehrere Heteroatome, die aus O, N und S ausgewählt sind, enthält und gegebenenfalls durch eine oder mehrere Hydroxylgruppen und/oder durch einen oder mehrere Reste, die aus C1-C8-Alkoxy- und C1-C8-Alkylgruppen ausgewählt sind, substituiert ist; ausgewählt ist, steht;
**A** für einen Rest, der aus:
a) -H;
b) einer linearen gesättigten C1-C20- oder ungesättigten C2-C20- oder verzweigten C3-C20- oder cyclischen C3-C8-Alkylgruppe, die gegebenenfalls durch ein oder mehrere Heteroatome oder eine oder mehrere Gruppen, die aus N, O, -CO- oder einer Kombination davon wie -NHCO- oder - NHCONH- ausgewählt sind, unterbrochen ist und/oder gegebenenfalls durch eine oder mehrere gleiche oder verschiedene Gruppen, die aus:
i) -OR15
ii) -SR15
iii) -NR16R17
iv) -CONHR16
vi) -COOR16
ix) einer C5-C12-(Hetero)arylgruppe, die gegebenenfalls ein oder mehrere Heteroatome, die aus O, N und S ausgewählt sind, enthält und gegebenenfalls durch eine oder mehrere Hydroxylgruppen und/oder durch einen oder mehrere C1-C8-Alkoxyreste substituiert ist;
x) einem gesättigten oder ungesättigten nichtaromatischen 5- bis 8-gliedrigen Heterocyclus, der ein oder mehrere Heteroatome, die aus O, N und S ausgewählt sind, umfasst und gegebenenfalls durch eine oder mehrere Hydroxylgruppen und/oder durch einen oder mehrere C1-C8-Alkoxy- oder C1-C4-Alkylreste substituiert ist; wobei eines der Ringglieder gegebenenfalls eine Carbonylgruppe ist;
ausgewählt sind, substituiert ist;
c) einer C5-C12-(Hetero)arylgruppe, die gegebenenfalls ein oder mehrere Heteroatome, die aus O, N und S ausgewählt sind, enthält und gegebenenfalls durch eine oder mehrere Hydroxylgruppen und/oder durch einen oder mehrere aus C1-C8-Alkoxy- und C1-C8-Alklgruppen ausgewählte Reste substituiert ist;
d) -NR12R13;
e) -OR14;
ausgewählt ist, steht;
wobei **R12** und **R13,** die gleich oder verschieden sein können, für einen Rest, der aus:
a) -H;
b) einer gesättigten linearen C1-C4-Alkylgruppe;
c) einer Phenylgruppe, die gegebenenfalls durch eine oder mehrere Hydroxylgruppen und/oder durch einen oder mehrere C1-C8-Alkoxyreste substituiert ist;
ausgewählt ist, stehen;
wobei **R12** und **R13** gegebenenfalls mit dem Stickstoff, der sie trägt, einen 5- bis 8-gliedrigen Heterocyclus bilden, der ein oder mehrere Heteroatome oder eine oder mehrere Gruppen, die aus N, O und -CO- ausgewählt sind, enthalten kann und/oder gegebenenfalls durch eine C1-C10-Kette auf Kohlenwasserstoffbasis, die gegebenenfalls einen oder mehrere Reste, die aus Hydroxyl und C1-C4-Alkoxy ausgewählt sind, enthält, substituiert sein kann;
wobei **R14** für einen Rest, der aus:
a) -H;
b) einer gesättigten linearen C1-C10-Alkylgruppe, die gegebenenfalls durch einen oder mehrere gleiche oder verschiedene Phenylreste substituiert ist, wobei die Phenylreste gegebenenfalls durch eine oder mehrere Hydroxylgruppen und/oder durch einen oder mehrere C1-C8-Alkoxyreste substituiert sind;
ausgewählt ist, steht;
wobei **R15** aus H, einer gesättigten linearen C1-C10- oder verzweigten C3-C10- oder ungesättigten C2-C10- oder cyclischen C3-C8-Alkylgruppe auf Kohlenwasserstoffbasis ausgewählt ist;
wobei **R16** und **R17,** die gleich oder verschieden sein können, aus H und einer gesättigten linearen C1-C10- oder verzweigten C3-C10- oder ungesättigten C2-C10- oder cyclischen C3-C8-Alkylgruppe auf Kohlenwasserstoffbasis; einer (C1-C4)Alkyl(hetero)(C6)arylgruppe, die gegebenenfalls ein Stickstoffatom enthält, insbesondere einer Benzylgruppe; einem Acetylrest ausgewählt sind; wobei **R16** und **R17** gegebenenfalls mit dem Stickstoff, der sie trägt, einen 5- bis 8-gliedrigen Heterocyclus bilden, der ein oder mehrere Heteroatome oder eine oder mehrere Gruppen, die aus N, O und -CO- ausgewählt sind, enthalten kann und/oder gegebenenfalls durch eine C1-C10-Kette auf Kohlenwasserstoffbasis substituiert sein kann;
**R"** für einen Rest, der aus:
a) -H;
b) einer linearen gesättigten C1-C10- oder verzweigten C3-C10-Alkylgruppe, die gegebenenfalls durch eine oder mehrere gleiche oder verschiedene Gruppen -OR25 substituiert ist;
ausgewählt ist, steht;
wobei **R25** aus H und einer gesättigten linearen C1-C10-Alkylgruppe auf Kohlenwasserstoffbasis ausgewählt ist.

3. Verbindungen nach einem der vorhergehenden Ansprüche, in denen:
• **R** für ein Wasserstoffatom steht;
• **Y** für einen Rest, der aus OR' und NAR" ausgewählt ist, steht;
**R'** für einen Rest, der aus:
a) -H;
b) einer linearen gesättigten C1-C10- oder ungesättigten C2-C10- oder verzweigten C3-C10- oder cyclischen C3-C8-Alkylgruppe, die gegebenenfalls durch ein oder mehrere Sauerstoffatome unterbrochen ist und/oder gegebenenfalls durch eine oder mehrere Hydroxyl-, Methoxy- oder Ethoxygruppen substituiert ist; ausgewählt ist, steht;
**A** für einen Rest, der aus:
a) -H;
b) einer linearen gesättigten C1-C10- oder ungesättigten C2-C10- oder verzweigten C3-C10- oder cyclischen C3-C8-Alkylgruppe, die gegebenenfalls durch ein oder mehrere Sauerstoffatome unterbrochen ist und/oder gegebenenfalls durch eine oder mehrere gleiche oder verschiedene Gruppen, die aus:
i) -OR15
ii) -NR16R17
vi) -COOR16
ix) einer C5-C12-(Hetero)arylgruppe, die gegebenenfalls ein oder mehrere Heteroatome, die aus O, N und S ausgewählt sind, enthält und gegebenenfalls durch eine oder mehrere Hydroxylgruppen und/oder durch einen oder mehrere C1-C8-Alkoxyreste substituiert ist;
x) einem gesättigten oder ungesättigten nichtaromatischen 5- bis 8-gliedrigen Heterocyclus, der ein oder mehrere Heteroatome, die aus O, N und S ausgewählt sind, umfasst und gegebenenfalls durch eine oder mehrere Hydroxylgruppen und/oder durch einen oder mehrere C1-C8-Alkoxy- oder C1-C4-Alkylreste substituiert ist;
ausgewählt sind, substituiert ist;
c) einer Phenylgruppe, die gegebenenfalls durch einen oder mehrere Reste, die aus Hydroxylgruppen und/oder Methoxy und/oder Ethoxy ausgewählt sind, substituiert ist;
d) -NR12R13;
e) -OR14;
ausgewählt ist, steht;
wobei **R12** und **R13,** die gleich oder verschieden sein können, für einen Rest, der aus:
a) -H;
b) einer gesättigten linearen C1-C4-Alkylgruppe;
c) einer Phenylgruppe, die gegebenenfalls durch eine oder mehrere Hydroxylgruppen und/oder durch einen oder mehrere C1-C8-Alkoxyreste substituiert ist;
ausgewählt ist, stehen;
wobei **R14** für einen Rest, der aus:
a) -H;
b) einer gesättigten linearen C1-C4-Alkylgruppe, die gegebenenfalls durch einen Phenylrest substituiert ist;
ausgewählt ist, steht;
wobei **R15** aus H und einer gesättigten linearen C1-C4- oder verzweigten C3-C4-Alkylgruppe auf Kohlenwasserstoffbasis ausgewählt ist;
wobei **R16** und **R17,** die gleich oder verschieden sein können, aus H und einer gesättigten linearen C1-C10- oder verzweigten C3-C10- oder ungesättigten C2-C10- oder cyclischen C3-C8-Alkylgruppe auf Kohlenwasserstoffbasis; einer (C1-C4)Alkyl(hetero)(C6)arylgruppe, die gegebenenfalls ein Stickstoffatom enthält, insbesondere einer Benzylgruppe; einem Acetylrest ausgewählt sind;
wobei **R16** und **R17** gegebenenfalls mit dem Stickstoff, der sie trägt, einen 5- bis 8-gliedrigen Heterocyclus bilden, der ein oder mehrere Heteroatome oder eine oder mehrere Gruppen, die aus N, O und -CO- ausgewählt sind,
enthalten kann und/oder gegebenenfalls durch eine C1-C10-Kette auf Kohlenwasserstoffbasis substituiert sein kann;
**R"** für einen Rest, der aus:
a) -H;
b) einer linearen gesättigten C1-C10- oder verzweigten C3-C10-Alkylgruppe, die gegebenenfalls durch ein oder mehrere gleiche oder verschiedene Gruppen -OR25 substituiert ist; wobei **R25** aus H und einer gesättigten linearen C1-C4-Alkylgruppe auf Kohlenwasserstoffbasis ausgewählt ist;
ausgewählt ist, steht.

4. Verbindungen nach einem der vorhergehenden Ansprüche, ausgewählt aus:
| Nr. | Struktur |
|---|---|
| 1 | 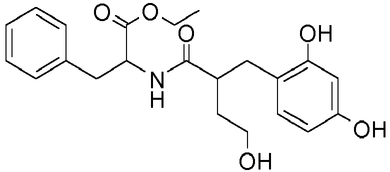 |
| 2 | 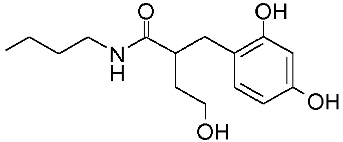 |
| 3 | 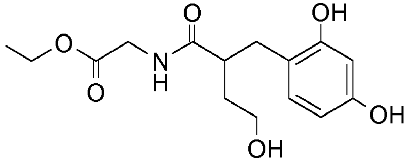 |
| 4 | 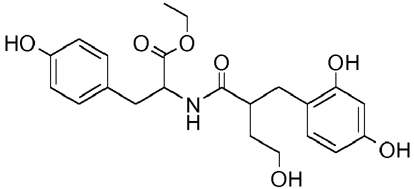 |
| 5 | 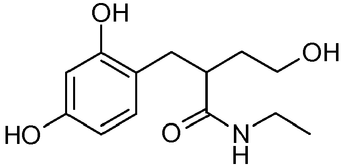 |
| 6 | 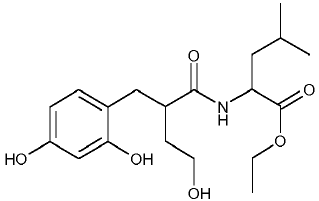 |
| 7 | 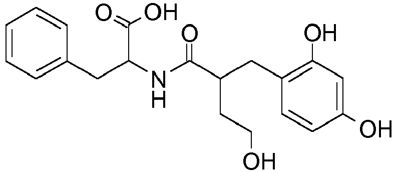 |
| 8 | 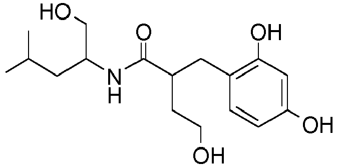 |
| 9 | 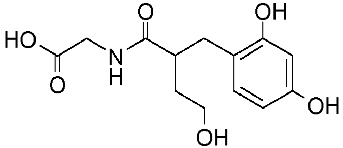 |
| 10 | 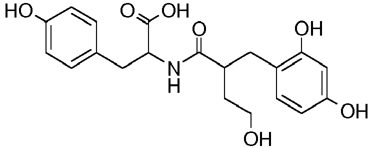 |
| 11 | 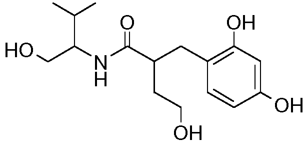 |
| 12 | 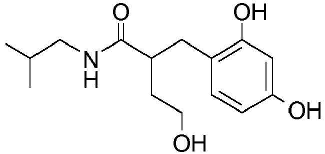 |
| 13 | 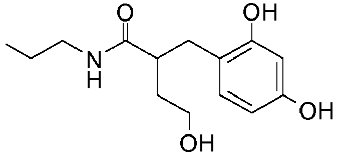 |
| 14 | 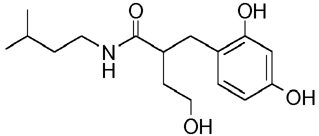 |
| 15 | 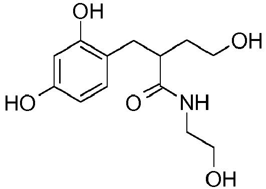 |
| 16 | 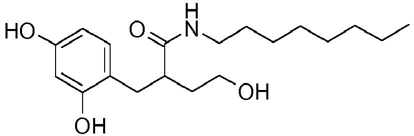 |
| 17 | 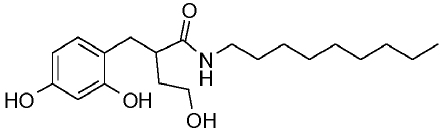 |
| 18 | 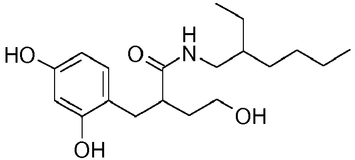 |
| 19 | 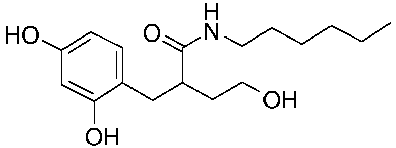 |
| 20 | 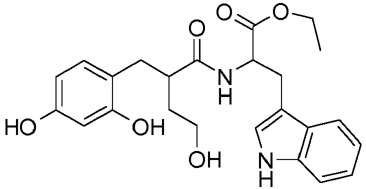 |
| 21 | 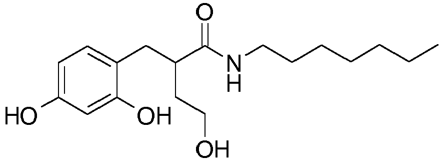 |
| 22 | 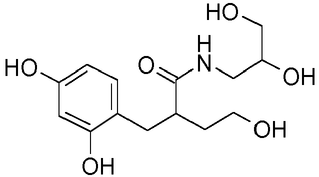 |
| 23 | 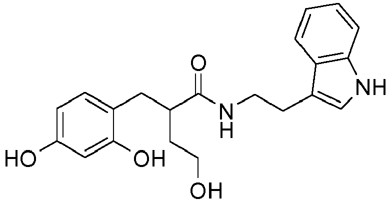 |
| 24 | 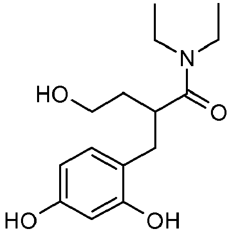 |
| 25 | 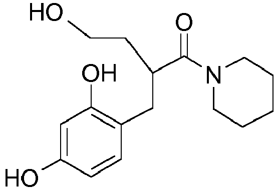 |
| 26 | 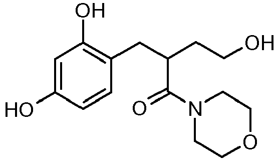 |
| 27 | 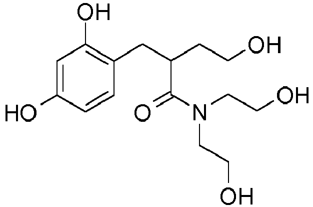 |
| 28 | 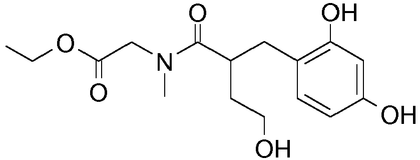 |
| 29 | 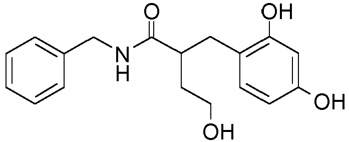 |
| 30 | 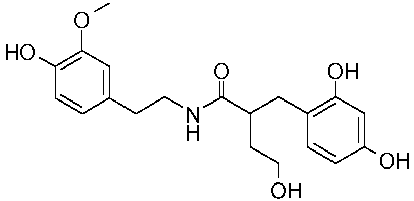 |
| 31 | 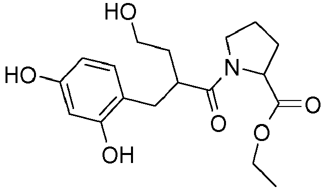 |
| 32 | 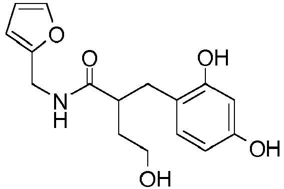 |
| 33 | 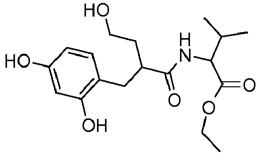 |
| 34 | 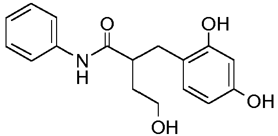 |
| 35 | 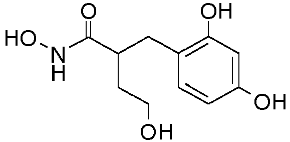 |
| 36 | 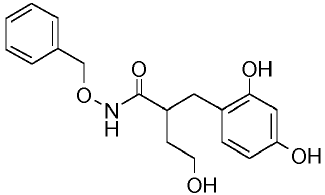 |
| 37 | 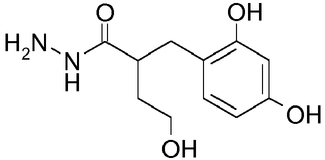 |
| 38 | 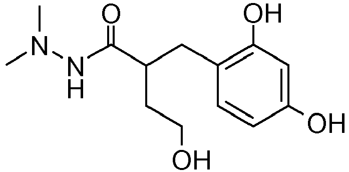 |
| 39 | 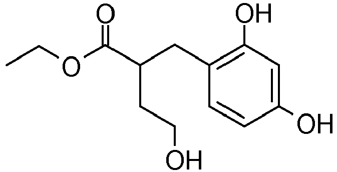 |
| 40 | 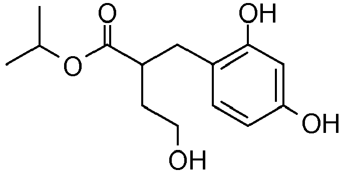 |
| 41 | 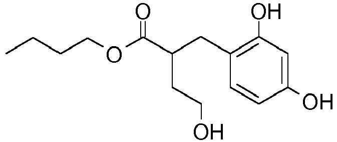 |
| 42 | 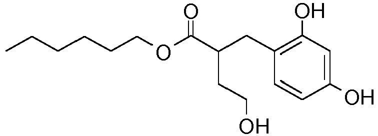 |
| 43 | 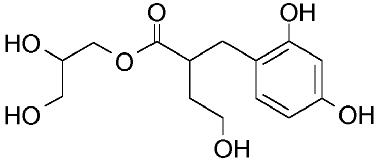 |
| | |
| 45 | 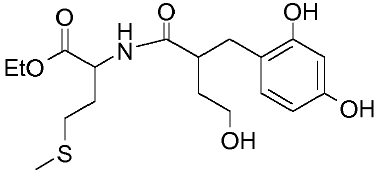 |
| 46 | 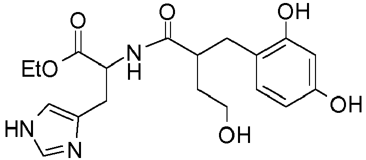 |
| 47 | 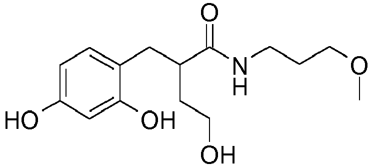 |
| 48 | 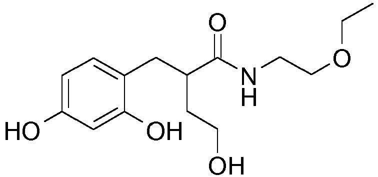 |
| 49 | 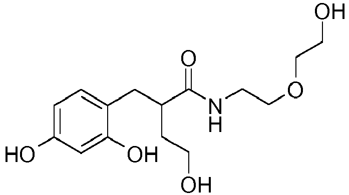 |
| 50 | 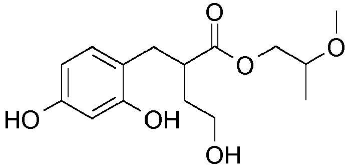 |
| 51 | 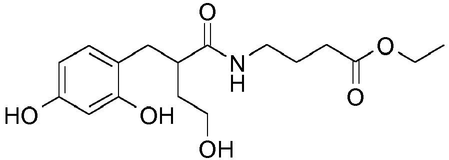 |
| 52 | 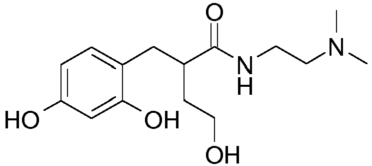 |
| 53 | 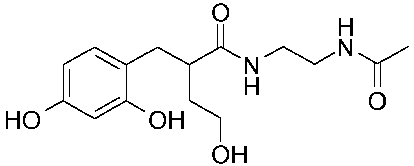 |
| 54 | 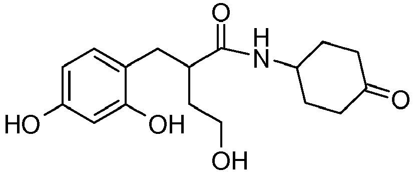 |
| 55 | 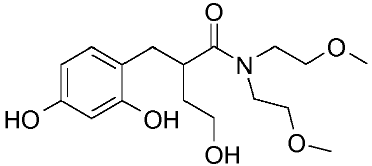 |
| 56 | 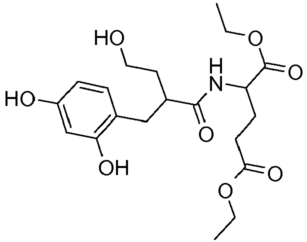 |
| 57 | 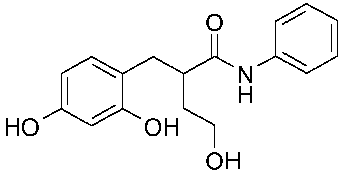 |
| 58 | 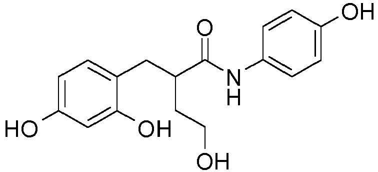 |
| 59 | 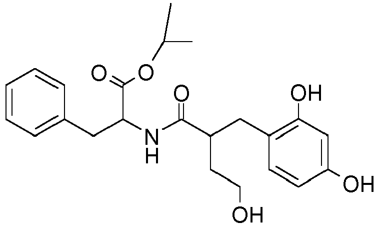 |
und die Salze davon, die Solvate davon, die optischen Isomere davon und die racemischen Gemische davon alleine oder als Gemisch.

5. Zusammensetzung, die in einem physiologisch unbedenklichen Medium eine Verbindung der Formel (I) nach einem der vorhergehenden Ansprüche umfasst.

6. Zusammensetzung nach dem vorhergehenden Anspruch, **dadurch gekennzeichnet, dass** Verbindung (I) in einem Gehalt zwischen 0,01 und 10 Gew.-%, bezogen auf das Gesamtgewicht der Zusammensetzung, vorliegt.

7. Zusammensetzung nach den vorhergehenden Ansprüchen 5 oder 6, **dadurch gekennzeichnet, dass** Verbindung (I) in einem Gehalt zwischen 0,1 und 5 Gew.-%, bezogen auf das Gesamtgewicht der Zusammensetzung, vorliegt.

8. Zusammensetzung nach den vorhergehenden Ansprüchen 5 bis 7, **dadurch gekennzeichnet, dass** sie mindestens einen Hilfsstoff aus der Gruppe bestehend aus Wasser; organischen Lösungsmitteln, auf Kohlenstoff basierenden Ölen, Silikonölen, Wachsen, Pigmenten, Füllstoffen, Farbstoffen, Tensiden, Emulgatoren; kosmetischen Wirkstoffen, UV-Schutzmitteln, Polymeren, Verdickungsmitteln, Konservierungsmitteln, Duftstoffen, Bakteriziden, Ceramiden, Geruchsabsorbern und Antioxidantien umfasst.

9. Zusammensetzung nach einem der Ansprüche 5 bis 8, **dadurch gekennzeichnet, dass** sie mindestens einen Wirkstoff umfasst, der aus Entschuppungsmitteln; beruhigend wirkenden Mitteln; organischen oder mineralischen Lichtschutzmitteln; Feuchtigkeitsmitteln; Depigmentierungsmitteln; Antiglykierungsmitteln; NO-Synthase-Inhibitoren; Mitteln, die die Synthese von dermalen oder epidermalen Makromolekülen stimulieren und/oder ihre Zersetzung verhindern; Mitteln, die die Proliferation von Fibroblasten und/oder Keratinozyten stimulieren oder die die Differenzierung von Keratinozyten stimulieren; Muskelrelaxantien und/oder Hautdekontraktionsmitteln; Straffungsmitteln; Mitteln gegen Schadstoffe und/oder Radikalfängern; Mitteln, die auf die Kapillarzirkulation wirken; Mitteln, die auf den Energiestoffwechsel von Zellen wirken; und Mischungen davon ausgewählt ist.

10. Nichttherapeutisches kosmetisches Verfahren zur Depigmentierung, Aufhellung und/oder Bleichung von Keratinmaterialien, bei dem man eine Zusammensetzung nach einem der Ansprüche 5 bis 9 aufbringt.

11. Verfahren nach dem vorhergehenden Anspruch zur Depigmentierung, Aufhellung und/oder Bleichung der Haut.

12. Nichttherapeutische kosmetische Verwendung einer Verbindung der Formel (I) gemäß einem der Ansprüche 1 bis 4 als Mittel zur Bleichung, Aufhellung und/oder Depigmentierung von Keratinmaterialien.

13. Verfahren zur Herstellung der Verbindungen der Formel (I) nach einem der Ansprüche 1 bis 4, das die folgenden Stufen umfasst: 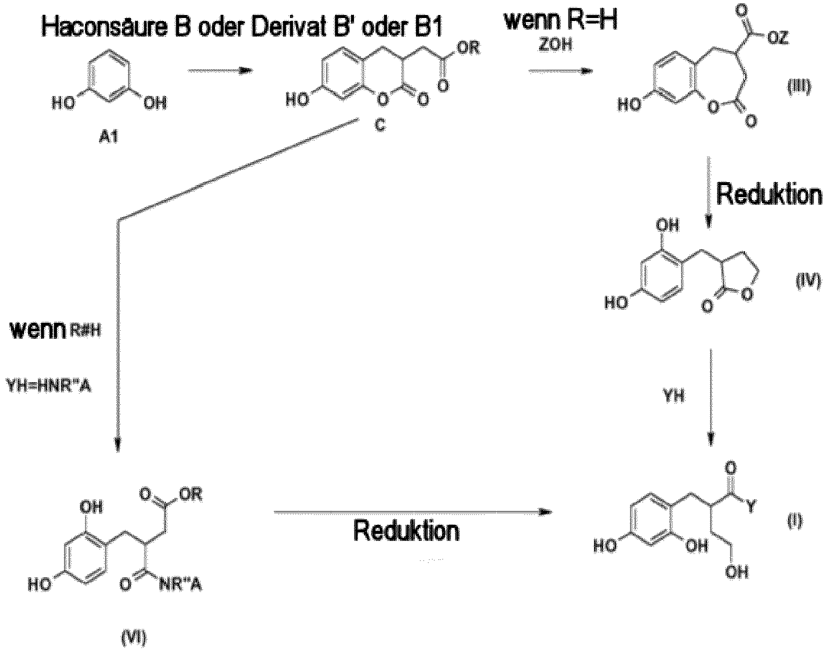 das die folgenden Schritte umfasst:
a) Umsetzung von Resorcinol (A1) mit Itaconsäure (B) oder mit ihrem Anhydrid (B') oder mit einem ihrer Ester der Formel (B1) 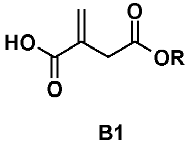 in der R für H oder eine lineare C1-C6- oder verzweigte C3-C6-Alkylgruppe steht, zu einer Verbindung C, wonach diese Verbindung zur Bildung eines Heterocyclus der Formel (III) aktiviert wird, welcher dann einem Reduktionsschritt unterworfen wird, der das Intermediat (IV) ergibt, welches Zugang zu den Verbindungen (I) gibt,
Z für H oder eine lineare (C₁-C₆)Alkylgruppe oder eine verzweigte (C₃-C₆)Alkylgruppe steht,
**Y** für einen Rest, der aus OR' und NAR'' ausgewählt ist, steht.

14. Intermediate der Formeln (III) und (IV) 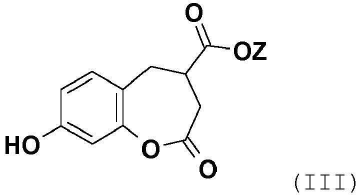 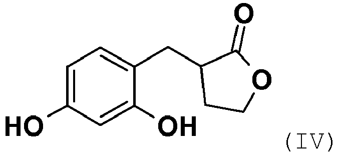 in denen Z für H oder eine lineare (C₁-C₆)Alkylgruppe oder eine verzweigte (C₃-C₆)Alkylgruppe steht.

## Revendications

1. Composés de formule (I) : 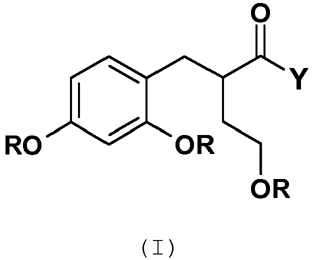 dans laquelle :
• **R** désigne un atome d'hydrogène;
• **Y** désigne un radical choisi parmi OR' ou NAR";
**R'** désigne un radical choisi parmi :
a) -H ;
b) - un groupe alkyle linéaire saturé en C1-C20 ou insaturé en C2-C20 ou ramifié en C3- C20 ou cyclique en C3-C8, éventuellement interrompu par un ou plusieurs hétéroatomes
ou groupements choisis parmi N, O, -CO- ou leur combinaison telle que -NHCO-, -NHCONH- , et/ou éventuellement substitué par un ou plusieurs groupes identiques ou
différents choisi(s) parmi :
i) -OR5
ii) -SR5
iii) -NR6R7
iv) -CONHR6
v) -CONR6R7
vi) -COOR6
vii) -NHCONHR6
viii) -C(O)alkyleC1-C4
ix) un groupe (hétéro)aryle en C5-C12, contenant éventuellement un ou plusieurs hétéroatomes choisis parmi O, N, S, éventuellement substitué par un ou plusieurs hydroxyles et/ou par un ou plusieurs radicaux alcoxy en C1-C8 ;
x) un hétérocycle saturé ou insaturé non aromatique ayant de 5 à 8 chaînons, comprenant un ou plusieurs hétéroatomes choisis parmi O, N, S, éventuellement substitué par un ou plusieurs hydroxyles et/ou par un ou plusieurs radicaux alcoxy en C1-C8 ou alkyl en C1-C4 ; l'un des chaînons pouvant être un groupement carbonyle ;
c) un groupe (hétéro)aryle en C5-C12, contenant éventuellement un ou plusieurs hétéroatomes choisis parmi O, N, S, éventuellement substitué par un ou plusieurs hydroxyles et/ou par un ou plusieurs radicaux choisis parmi les groupes alcoxy en C1-C8
ou alkyl en C1-C8 ;
**R5** étant choisi parmi H, un groupe hydrocarboné alkyle saturé linéaire en C1-C10 ou ramifié en C3-C10 ou insaturé en C2-C10 ou cyclique en C3-C8 ;
**R6**, **R7** identiques ou différents, étant choisis parmi H, un groupe hydrocarboné alkyle saturé linéaire en C1-C10 ou ramifié en C3-C10 ou insaturé en C2-C10 ou cyclique en C3-C8 ; un groupe alkyle(C1-C4)(hétéro)aryle en C6 contenant éventuellement un atome d'azote, notamment un groupe benzyle ;
**R6**, **R7** pouvant former avec l'azote qui les porte un hétérocycle ayant de 5 à 8 chaînons, pouvant contenir un ou plusieurs hétéroatomes ou groupements choisis parmi
N, O, -CO- et/ou éventuellement substitué par une chaîne hydrocarbonée en C1-C10 ;
**A** désigne un radical choisi parmi :
a) -H ;
b) un groupe alkyle linéaire saturé en C1-C20 ou insaturé en C2-C20 ou ramifié en C3-C20 ou cyclique en C3-C8, éventuellement interrompu par un ou plusieurs hétéroatomes
ou groupements choisis parmi N, O, -CO- ou leur combinaison telle que -NHCO-, -NHCONH- , et/ou éventuellement substitué par un ou plusieurs groupes identiques ou
différents choisi(s) parmi :
i) -OR15
ii) -SR15
iii) -NR16R17
iv) -CONHR16
v) -CONR16R17
vi) -COOR16
vii) -NHCONHR16
viii) -C(O)alkyleC1-C4
ix) un groupe (hétéro)aryle en C5-C12, contenant éventuellement un ou plusieurs hétéroatomes choisis parmi O, N, S, éventuellement substitué par un ou plusieurs hydroxyles et/ou par un ou plusieurs radicaux alcoxy en C1-C8 ;
x) un hétérocycle saturé ou insaturé non aromatique ayant de 5 à 8 chaînons, comprenant un ou plusieurs hétéroatomes choisis parmi O, N, S, éventuellement substitué par un ou plusieurs hydroxyles et/ou par un ou plusieurs radicaux alcoxy en C1-C8 ou alkyl en C1-C4 ; l'un des chaînons pouvant être un groupement carbonyle ;
c) un groupe (hétéro)aryle en C5-C12, contenant éventuellement un ou plusieurs hétéroatomes choisis parmi O, N, S, éventuellement substitué par un ou plusieurs hydroxyles et/ou par un ou plusieurs radicaux choisis parmi les groupes alcoxy en C1-C8
ou alkyl en C1-C8 ;
xi)-
NH-C=NH(NH2) (groupement guanidine)
d) -NR12R13 ;
e) -OR14 ;
f) -C(O)NHR14 ;
g) C(O) alkyle C1-C10
**R12, R13**, identiques ou différents, désignant un radical choisi parmi :
a) -H
b) un groupe alkyle saturé linéaire en C1-C10 ou insaturé en C2-C10 ou ramifié en C3-C10 ou cyclique en C3-C8, éventuellement interrompu par un ou plusieurs hétéroatomes ou groupements choisis parmi N, O, -CO- ou leur combinaison telle que -NHCO-, -NHCONH- , et/ou éventuellement substitués par un ou plusieurs groupes, identiques ou différents, choisi(s) parmi -OR5 ;
c) un groupe (hétéro)aryle en C5-C12 , contenant éventuellement un ou plusieurs hétéroatomes choisis parmi O, N, S, éventuellement substitué par un ou plusieurs hydroxyles et/ou par un ou plusieurs radicaux alcoxy en C1-C8 ; R2 et R3 pouvant former avec l'azote qui les porte un hétérocycle ayant de 5 à 8 chaînons, pouvant contenir un ou plusieurs hétéroatomes ou groupements choisis parmi N, O, -CO- et/ou éventuellement substitué par une chaîne hydrocarbonée en C1-C10 contenant éventuellement un ou plusieurs radicaux choisis parmi hydroxyle ou alcoxy C1-C4;
**R14** désignant un radical choisi parmi :
a) -H
b) un groupe alkyle saturé linéaire en C1-C10 ou ramifié en C3-C10 ou cyclique en C3-C8, éventuellement substitué par un ou plusieurs groupes, identiques ou différents,
choisis parmi :
i) -COOR16 ,
ii) un radical (hétéro)aryle en C5-C12 , contenant éventuellement un ou plusieurs hétéroatomes choisis parmi O, N, S, éventuellement substitué par un ou plusieurs hydroxyles et/ou par un ou plusieurs radicaux alcoxy en C1-C8 ;
c) un groupe (hétéro)aryle en C5-C12 , contenant éventuellement un ou plusieurs hétéroatomes choisis parmi O, N, S, éventuellement substitué par un ou plusieurs hydroxyles et/ou par un ou plusieurs radicaux alcoxy en C1-C8 ;
**R15** étant choisi parmi H, un groupe hydrocarboné alkyle saturé linéaire en C1-C10 ou ramifié en C3-C10 ou insaturé en C2-C10 ou cyclique en C3-C8 ; R16 , R17 identiques ou différents, étant choisis parmi H, un groupe hydrocarboné alkyle saturé linéaire en C1-C10 ou ramifié en C3-C10 ou insaturé en C2-C10 ou cyclique en C3-C8 ; un groupe alkyle(C1-C4)(hétéro)aryle en C6 contenant éventuellement un atome d'azote, notamment un groupe benzyle ;
**R16** , **R17** identiques ou différents, étant choisis parmi H, un groupe hydrocarboné alkyle saturé linéaire en C1-C10 ou ramifié en C3-C10 ou insaturé en C2-C10 ou cyclique en C3-C8 ; un groupe alkyle(C1-C4)(hétéro)aryle en C6 contenant éventuellement un atome d'azote, notamment un groupe benzyle ; un radical acétyl ;
**R16** , **R17** pouvant former avec l'azote qui les porte un hétérocycle ayant de 5 à 8 chaînons, pouvant contenir un ou plusieurs hétéroatomes ou groupements choisis parmi N, O, -CO- et/ou éventuellement substitué par une chaîne hydrocarbonée en C1-C10 ;
**R"** désigne un radical choisi parmi :
a) -H ;
b) un groupe alkyle linéaire saturé en C1-C20 ou insaturé en C2-C20 ou ramifié en C3-C20 ou cyclique en C3-C8, éventuellement interrompu par un ou plusieurs hétéroatomes ou groupements choisis parmi N, O, -CO- ou leur combinaison telle que -NHCO-, -NHCONH- , et/ou éventuellement substitué par un ou plusieurs groupes identiques ou différents choisi(s) parmi :
i) -OR25
ii) -SR25
iii) -NR26R27
iv) -CONHR26
v) -CONR26R27
vi) -COOR26
vii) -NHCONHR26
viii) -C(O)alkyleC1-C4
ix) un groupe (hétéro)aryle en C5-C12, contenant éventuellement un ou plusieurs hétéroatomes choisis parmi O, N, S, éventuellement substitué par un ou plusieurs hydroxyles et/ou par un ou plusieurs radicaux alcoxy en C1-C8 ;
x) un hétérocycle saturé ou insaturé non aromatique ayant de 5 à 8 chaînons, comprenant un ou plusieurs hétéroatomes choisis parmi O, N, S, éventuellement substitué par un ou plusieurs hydroxyles et/ou par un ou plusieurs radicaux alcoxy en C1-C8 ou alkyl en C1-C4 ; l'un des chaînons pouvant être un groupement carbonyle ;
c) un groupe (hétéro)aryle en C5-C12, contenant éventuellement un ou plusieurs hétéroatomes choisis parmi O, N, S, éventuellement substitué par un ou plusieurs hydroxyles et/ou par un ou plusieurs radicaux choisis parmi les groupes alcoxy en C1-C8 ou alkyl en C1-C8 ;
**R25** étant choisi parmi H, un groupe hydrocarboné alkyle saturé linéaire en C1-C10 ou ramifié en C3-C10 ou insaturé en C2-C10 ou cyclique en C3-C8 ;
**R26** , **R27** identiques ou différents, étant choisis parmi H, un groupe hydrocarboné alkyle saturé linéaire en C1-C10 ou ramifié en C3-C10 ou insaturé en C2-C10 ou cyclique en C3-C8 ; un groupe alkyle(C1-C4) (hétéro)aryle en C6 contenant éventuellement un atome d'azote, notamment un groupe benzyle ; **R26** , **R27** pouvant former avec l'azote qui les porte un hétérocycle ayant de 5 à 8 chaînons, pouvant contenir un ou plusieurs hétéroatomes ou groupements choisis parmi N, O,-CO- et/ou éventuellement substitué par une chaîne hydrocarbonée en C1-C10 ;
étant entendu que **A et R''** peuvent former avec l'atome d'azote qui les porte un hétérocycle saturé ou insaturé de 5 à 8 chaînons, pouvant contenir un ou plusieurs hétéroatomes ou groupements choisis parmi N, O, -CO-et/ou éventuellement substitué par une chaîne hydrocarbonée en C1-C10 ou hydroxyalkyle en C1-C10 ou CO2T, T désignant un atome d'hydrogène ou un radical alkyle saturé linéaire en C1-C10 ou ramifié en C3-C10.
ainsi que leurs sels, leurs solvates, leurs isomères optiques et leurs racémiques, seuls ou en mélange.

2. Composés selon la revendication précédente, dans lesquels :
**R** désigne un atome d'hydrogène ou un groupe acétyle ;
**Y** désigne un radical choisi parmi OR' ou NAR"
**R'** désigne un radical choisi parmi :
a) -H ;
b) un groupe alkyle linéaire saturé en C1-C10 ou insaturé en C2-C10 ou ramifié en C3-C10 ou cyclique en C3-C8, éventuellement interrompu par un ou plusieurs hétéroatomes ou groupements choisis parmi N, O, -CO- ou leur combinaison telle que -NHCO-, -NHCONH- , et/ou éventuellement substitué par un ou plusieurs groupes -OR5 ; **R5** étant choisi parmi H, un groupe hydrocarboné alkyle saturé linéaire en C1-C4 ;
c) un groupe (hétéro)aryle en C5-C12, contenant éventuellement un ou plusieurs hétéroatomes choisis parmi O, N, S, éventuellement substitué par un ou plusieurs hydroxyles et/ou par un ou plusieurs radicaux choisis parmi les groupes alcoxy en C1-C8 ou alkyl en C1-C8 ;
**A** désigne un radical choisi parmi :
a) -H ;
b) un groupe alkyle linéaire saturé en C1-C20 ou insaturé en C2-C20 ou ramifié en C3- C20 ou cyclique en C3-C8, éventuellement interrompu par un ou plusieurs hétéroatomes ou groupements choisis parmi N, O, -CO- ou leur combinaison telle que -NHCO-, -NHCONH- , et/ou éventuellement substitué par un ou plusieurs groupes identiques ou
différents choisi(s) parmi :
i) -OR15
ii) -SR15
iii) -NR16R17
iv) -CONHR16
vi) -COOR16
ix) un groupe (hétéro)aryle en C5-C12, contenant éventuellement un ou plusieurs hétéroatomes choisis parmi O, N, S, éventuellement substitué par un ou plusieurs hydroxyles et/ou par un ou plusieurs radicaux alcoxy en C1-C8 ;
x) un hétérocycle saturé ou insaturé non aromatique ayant de 5 à 8 chaînons, comprenant un ou plusieurs hétéroatomes choisis parmi O, N, S, éventuellement substitué par un ou plusieurs hydroxyles et/ou par un ou plusieurs radicaux alcoxy en C1-C8 ou alkyl en C1-C4 ; l'un des chaînons pouvant être un groupement carbonyle ;
c) un groupe (hétéro)aryle en C5-C12, contenant éventuellement un ou plusieurs hétéroatomes choisis parmi O, N, S, éventuellement substitué par un ou plusieurs hydroxyles et/ou par un ou plusieurs radicaux choisis parmi les groupes alcoxy en C1-C8 ou alkyl en C1-C8 ;
d) -NR12R13 ;
e) -OR14 ;
**R12, R13** , identiques ou différents, désignant un radical choisi parmi :
a) -H
b) un groupe alkyle saturé linéaire en C1-C4
c) un groupe phényle éventuellement substitué par un ou plusieurs hydroxyles et/ou par un ou plusieurs radicaux alcoxy en C1-C8 ;
**R12 et R13** pouvant former avec l'azote qui les porte un hétérocycle ayant de 5 à 8 chaînons, pouvant contenir un ou plusieurs hétéroatomes ou groupements choisis parmi N, O, -CO- et/ou éventuellement substitué par une chaîne hydrocarbonée en C1-C10 contenant éventuellement un ou plusieurs radicaux choisis parmi hydroxyle ou alcoxy C1-C4 ;
**R14** désignant un radical choisi parmi :
a) -H
b) un groupe alkyle saturé linéaire en C1-C10 éventuellement substitué par un ou plusieurs radicaux phényle, identiques ou différents, lesdits radicaux phényle étant éventuellement substitués par un ou plusieurs hydroxyles et/ou par un ou plusieurs radicaux alcoxy en C1-C8 ;
**R15** étant choisi parmi H, un groupe hydrocarboné alkyle saturé linéaire en C1-C10 ou ramifié en C3-C10 ou insaturé en C2-C10 ou cyclique en C3-C8 ;
**R16** , **R17** identiques ou différents, étant choisis parmi H, un groupe hydrocarboné alkyle saturé linéaire en C1-C10 ou ramifié en C3-C10 ou insaturé en C2-C10 ou cyclique en C3-C8 ; un groupe alkyle(C1-C4) (hétéro)aryle en C6 contenant éventuellement un atome d'azote, notamment un groupe benzyle ; un radical acétyle ;
**R16**, **R17** pouvant former avec l'azote qui les porte un hétérocycle ayant de 5 à 8 chaînons, pouvant contenir un ou plusieurs hétéroatomes ou groupements choisis parmi N, O, -CO- et/ou éventuellement substitué par une chaîne hydrocarbonée en C1-C10 ;
**R"** désigne un radical choisi parmi :
a) -H ;
b) un groupe alkyle linéaire saturé en C1-C10 ou ramifié en C3-C10 éventuellement substitué par un ou plusieurs groupes identiques ou différents-OR25
**R25** étant choisi parmi H, un groupe hydrocarboné alkyle saturé linéaire en C1-C10 .

3. Composés selon l'une des revendications précédentes, dans lesquels :
• **R** désigne un atome d'hydrogène;
• **Y** désigne un radical choisi parmi OR' ou NAR"
**R'** désigne un radical choisi parmi :
a) -H ;
b) un groupe alkyle linéaire saturé en C1-C10 ou insaturé en C2-C10 ou ramifié en C3- C10 ou cyclique en C3-C8, éventuellement interrompu par un ou plusieurs atomes d'oxygène , et/ou éventuellement substitué par un ou plusieurs groupes hydroxyle, méthoxy ou éthoxy.
**A** désigne un radical choisi parmi :
a) -H ;
b) un groupe alkyle linéaire saturé en C1-C10 ou insaturé en C2-C10 ou ramifié en C3- C10 ou cyclique en C3-C8, éventuellement interrompu par un ou plusieurs atomes d'oxygène et/ou éventuellement substitué par un ou plusieurs groupes identiques ou différents choisi(s) parmi :
i) -OR15
ii) -NR16R17
vi) -COOR16
ix) un groupe (hétéro)aryle en C5-C12, contenant éventuellement un ou plusieurs hétéroatomes choisis parmi O, N, S, éventuellement substitué par un ou plusieurs hydroxyles et/ou par un ou plusieurs radicaux alcoxy en C1-C8 ;
x) un hétérocycle saturé ou insaturé non aromatique ayant de 5 à 8 chaînons, comprenant un ou plusieurs hétéroatomes choisis parmi O, N, S, éventuellement substitué par un ou plusieurs hydroxyles et/ou par un ou plusieurs radicaux alcoxy en C1-C8 ou alkyl en C1-C4
c) un groupe phényle éventuellement substitué par un ou plusieurs radicaux choisis parmi hydroxyles et/ou méthoxy et/ou éthoxy;
d) -NR12R13 ;
e) -OR14 ;
**R12, R13** , identiques ou différents, désignant un radical choisi parmi :
a) -H
b) un groupe alkyle saturé linéaire en C1-C4
c) un groupe phényle éventuellement substitué par un ou plusieurs hydroxyles et/ou par un ou plusieurs radicaux alcoxy en C1-C8 ;
**R14** désignant un radical choisi parmi :
a) -H
b) un groupe alkyle saturé linéaire en C1-C4 éventuellement substitué par un radical phényle;
**R15** étant choisi parmi H, un groupe hydrocarboné alkyle saturé linéaire en C1-C4 ou ramifié en C3-C4;
**R16** , **R17** identiques ou différents, étant choisis parmi H, un groupe hydrocarboné alkyle saturé linéaire en C1-C10 ou ramifié en C3-C10 ou insaturé en C2-C10 ou cyclique en C3-C8 ; un groupe alkyle(C1-C4) (hétéro)aryle en C6 contenant éventuellement un atome d'azote, notamment un groupe benzyle ; un radical acétyle
**R16** , **R17** pouvant former avec l'azote qui les porte un hétérocycle ayant de 5 à 8 chaînons, pouvant contenir un ou plusieurs hétéroatomes ou groupements choisis parmi N, O, -CO- et/ou éventuellement substitué par une chaîne hydrocarbonée en C1-C10 ;
**R"** désigne un radical choisi parmi :
a) -H ;
b) un groupe alkyle linéaire saturé en C1-C10 ou ramifié en C3-C10 et/ou éventuellement substitué par un ou plusieurs groupes identiques ou différents -OR25 ; **R25** étant choisi parmi H, un groupe hydrocarboné alkyle saturé linéaire en C1-C4 ,

4. Composés selon l'une des revendications précédentes, choisis parmi :
| N° | Structure |
|---|---|
| 1 | 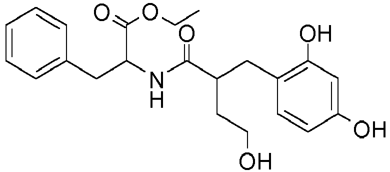 |
| 2 | 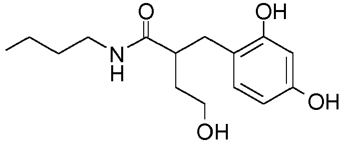 |
| 3 | 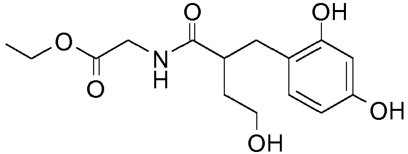 |
| 4 | 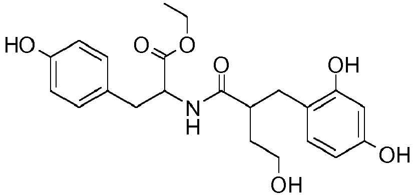 |
| 5 | 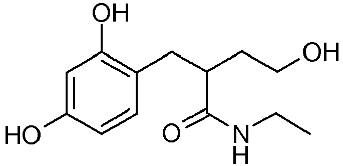 |
| 6 | 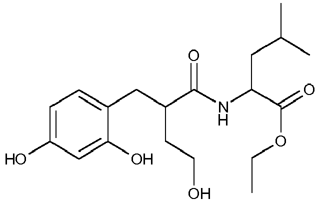 |
| 7 | 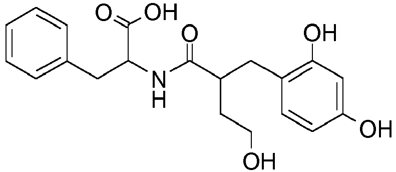 |
| 8 | 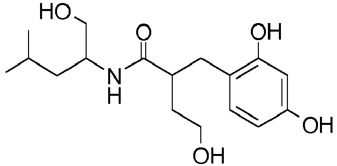 |
| 9 | 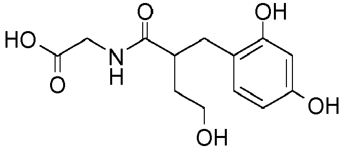 |
| 10 | 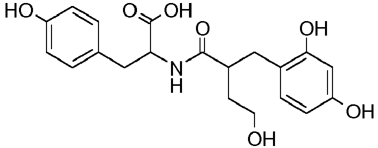 |
| 11 | 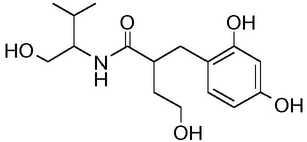 |
| 12 | 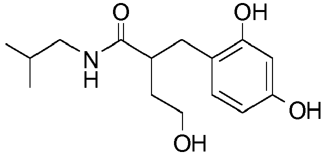 |
| 13 | 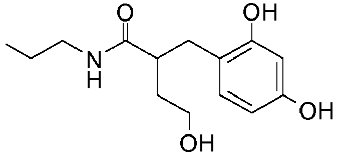 |
| 14 | 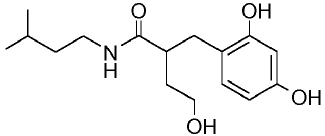 |
| 15 | 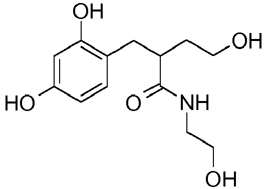 |
| 16 | 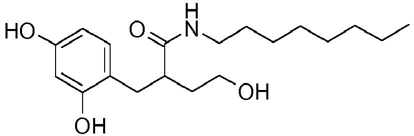 |
| 17 | 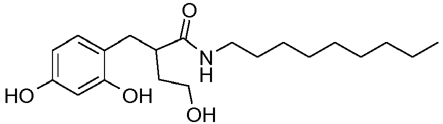 |
| 18 | 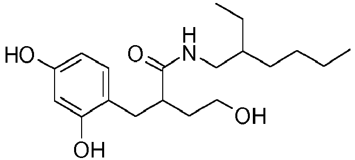 |
| 19 | 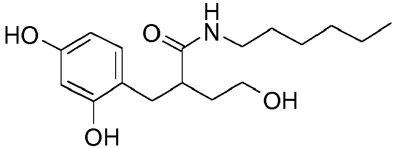 |
| 20 | 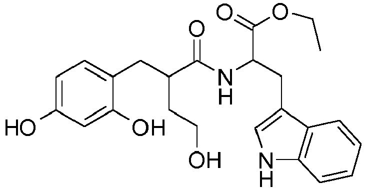 |
| 21 | 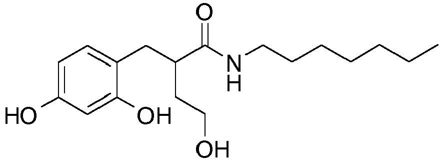 |
| 22 | 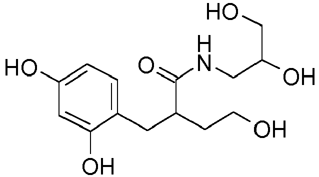 |
| 23 | 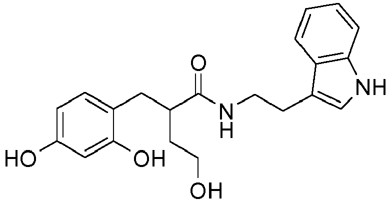 |
| 24 | 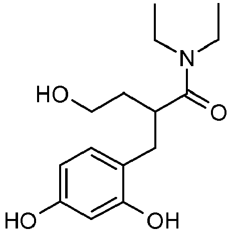 |
| 25 | 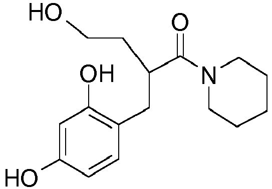 |
| 26 | 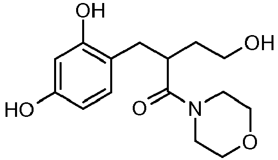 |
| 27 | 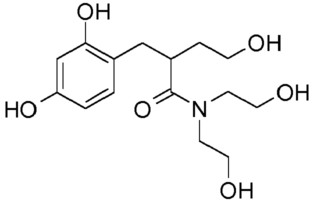 |
| 28 | 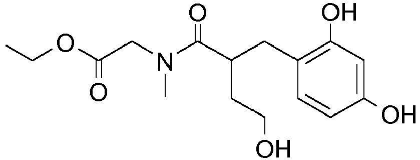 |
| 29 | 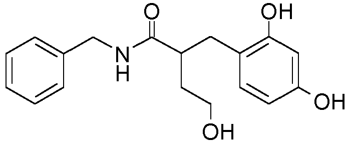 |
| 30 | 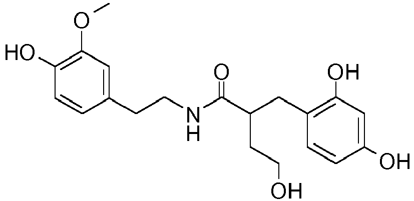 |
| 31 | 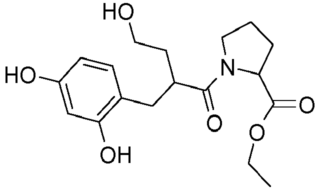 |
| 32 | 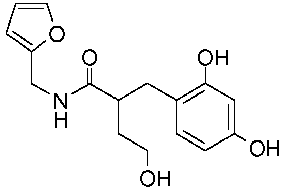 |
| 33 | 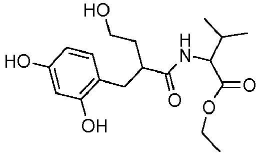 |
| 34 | 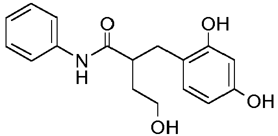 |
| 35 | 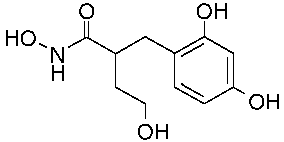 |
| 36 | 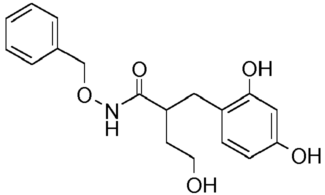 |
| 37 | 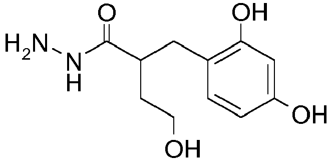 |
| 38 | 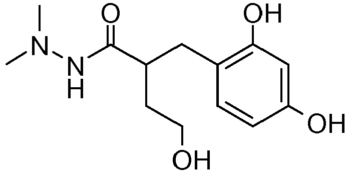 |
| 39 | 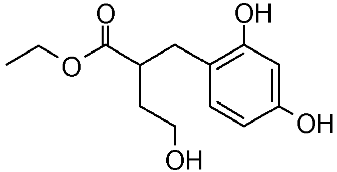 |
| 40 | 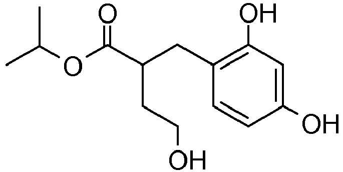 |
| 41 | 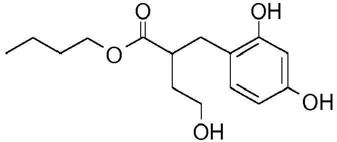 |
| 42 | 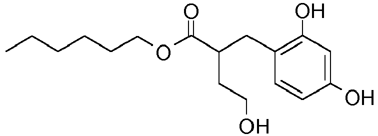 |
| 43 | 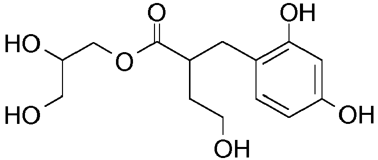 |
| 45 | 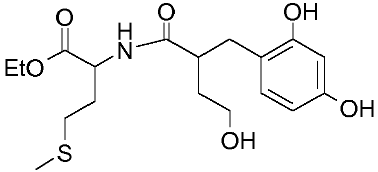 |
| 46 | 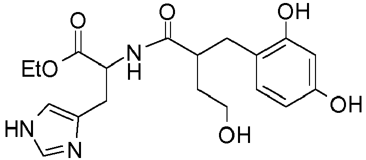 |
| 47 | 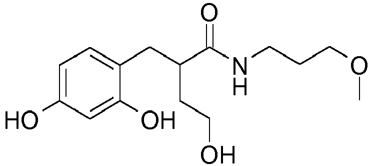 |
| 48 | 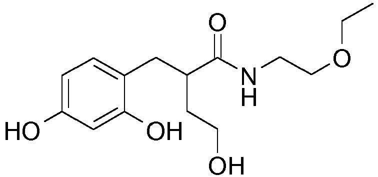 |
| 49 | 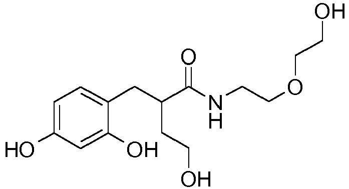 |
| 50 | 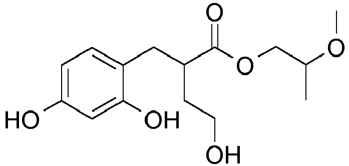 |
| 51 | 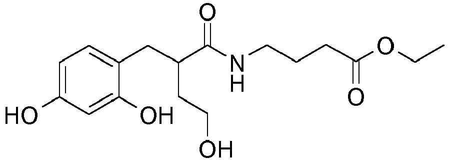 |
| 52 | 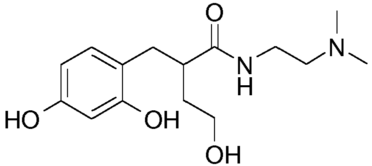 |
| 53 | 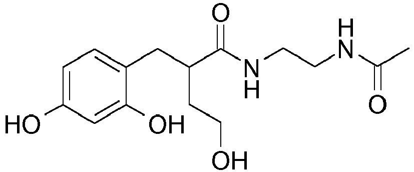 |
| 54 | 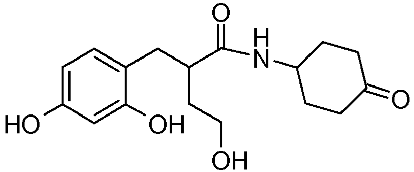 |
| 55 | 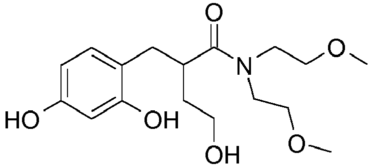 |
| 56 | 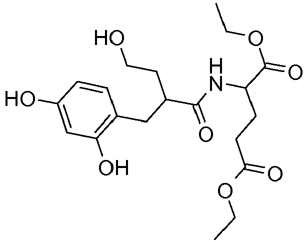 |
| 57 | 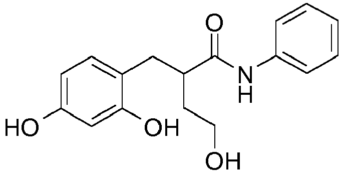 |
| 58 | 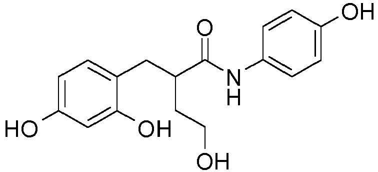 |
| 59 | 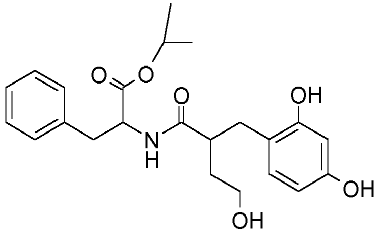 |
ainsi que leurs sels, leurs solvates, leurs isomères optiques et leurs racémiques, seuls ou en mélange.

5. Composition comprenant, dans un milieu physiologiquement acceptable, un composé de formule (I) selon l'une quelconque des revendications précédentes.

6. Composition selon la revendication précédente , **caractérisée en ce que** le composé (I) est présent en une teneur comprise entre 0,01 et 10% en poids, de par rapport au poids total de la composition.

7. Composition selon la revendication précédente 5 ou 6, **caractérisée en ce que** le composé (I) est présent en une teneur comprise entre 0,1% et 5% en poids par rapport au poids total de la composition.

8. Composition selon l'une des revendications 5 à 7, **caractérisée en ce qu'**elle comprend au moins un adjuvant choisi dans le groupe formé par : l'eau, les solvants organiques , les huiles hydrocarbonées, les huiles siliconées, les cires, les pigments, les charges, les colorants, les tensioactifs, les émulsionnants, les actifs cosmétiques, les filtres UV, les polymères, les épaississants, les conservateurs, les parfums, les bactéricides, les céramides, les absorbeurs d'odeur, les antioxydants.

9. Composition selon l'une quelconque des revendications 5 à 8, **caractérisée en ce qu'**elle comprend au moins un actif choisi parmi: les agents desquamants; les agents apaisants, les agents photoprotecteurs organiques ou inorganiques, les agents hydratants; les agents dépigmentants; les agents anti-glycation; les inhibiteurs de NO-synthase; les agents stimulant la synthèse de macromolécules dermiques ou épidermiques et/ou empêchant leur dégradation; les agents stimulant la prolifération des fibroblastes et/ou des kératinocytes ou stimulant la différenciation des kératinocytes; les agents myorelaxants et/ou les agents dermo-décontractants; les agents tenseurs; les agents anti-pollution et/ou anti-radicalaires; les agents agissant sur la microcirculation; les agents agissant sur le métabolisme énergétique des cellules; et leurs mélanges.

10. Procédé cosmétique non thérapeutique de dépigmentation, d'éclaircissement et/ou de blanchiment des matières kératiniques, comprenant l'application d'une composition selon l'une quelconque des revendications 5 à 9.

11. Procédé selon la revendication précédente, pour dépigmenter, éclaircir et/ou blanchir la peau.

12. Utilisation cosmétique non thérapeutique d'un composé de formule (I) tel que défini selon l'une quelconque des revendications 1 à 4, comme agent blanchissant, éclaircissant et/ou dépigmentant des matières kératiniques.

13. Procédé de préparation des composés de formule (I) selon l'une des revendications 1 à 4 comprenant les étapes suivantes : 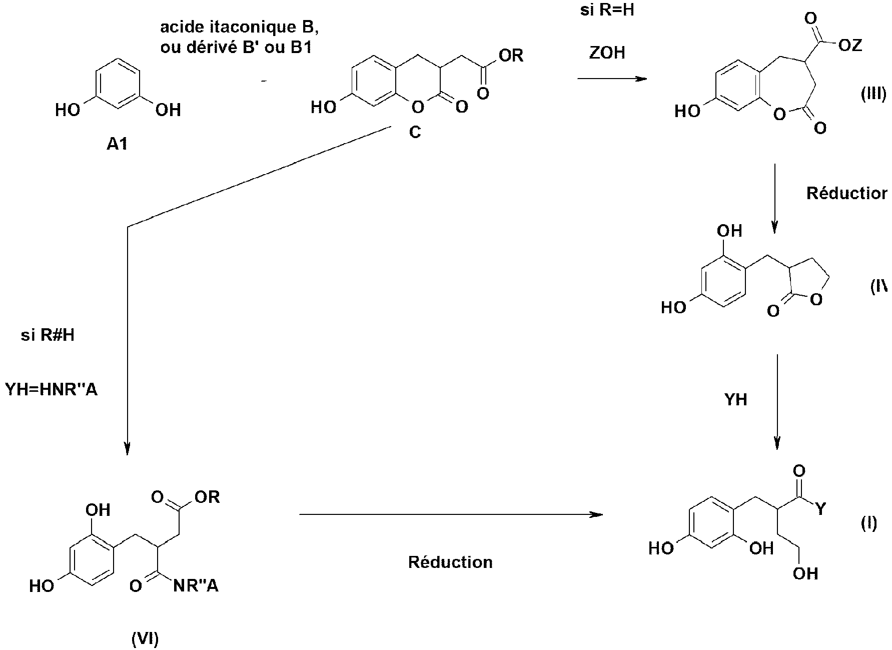 comprenant les étapes suivantes :
a) Réaction du résorcinol (A1) avec l'acide itaconique (B) ou avec son anhydride (B') ou avec un de ses esters de formule (B1) 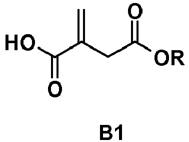 dans laquelle R désigne H ou un groupe alkyle linéaire en C1-C6 ou ramifié en C3-C6 pour former un composé C puis ce composé est activé de pour former un hétérocycle de formule (III) lequel est ensuite soumis à une étape de réduction pour conduire à l'intermédiaire (IV) qui permet d'accéder aux composés (I),
Z désigne un groupe (C1-C6) alkyle linéaire ou un groupe (C3-C6) alkyle ramifié,
Y désigne un radical choisi parmi OR' et NAR"

14. Composés intermédiaires de formules (III) et (IV) 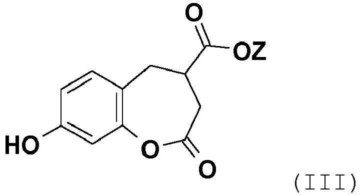 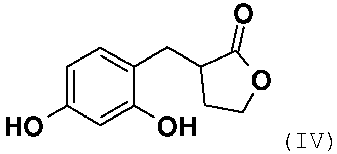 dans laquelle Z désigne H ou un groupe (C₁-C₆) alkyle linéaire ou un groupe (C₃-C₆)alkyle ramifié.
